# EUROPEAN PATENT SPECIFICATION

(11) **EP 2 532 652 B1**
(45) Date of publication and mention of the grant of the patent: **12.11.2014**
(21) Application number: 11737192.2
(22) Date of filing: 31.01.2011
(51) Int. Cl.: C07D 221/12, A61K 31/473, A61P 31/14

(54) **THERAPEUTIC AGENT FOR HEPATITIS C**
THERAPEUTISCHER WIRKSTOFF GEGEN HEPATITIS C
AGENT THERAPEUTIQUE DE L'HEPATITE C

(30) Priority: 01.02.2010 JP 2010020502
(43) Date of publication of application: 12.12.2012
(73) Proprietor: Kagoshima University, Kagoshima-shi, Kagoshima 890-8580 (JP); Oncolys BioPharma, Inc., Tokyo 105-0001 (JP)
(72) Inventor: BABA Masanori, Kagoshima-shi Kagoshima 890-8580 (JP); HASHIMOTO Yuichi, Tokyo 161-0032 (JP); AOYAMA Hiroshi, Kanagawa 213-0013 (JP); SUGITA Kazuyuki, Tokyo 158-0097 (JP); NAKAMURA Masahiko, Saitama 338-0837 (JP); AOYAMA Atsushi, Kawasaki-shi Kanagawa 215-0011 (JP); URATA Yasuo, Tokyo 105-0001 (JP)
(74) Representative: Raynor, Stuart Andrew
(86) International application number: PCT/JP2011/051859
(87) International publication number: WO 2011/093483

(56) References cited:
- WO-A1-94/18197
- CN-A- 101 084 906
- JP-A- 2010 500 328
- AOYAMA, A.: 'LXR Antagonists with a 5-Substituted Phenanthridin-6-one Skeleton: Synthesis and LXR Transrepression Activities of Conformationally Restricted Carba-T0901317 Analogs' HETEROCYCLES vol. 76, no. 1, 2008, pages 137 - 142
- COOKSON, R. F.: 'Synthesis of Some Phenanthridone Derivatives' JOURNAL OF HETEROCYCLIC CHEMISTRY vol. 9, no. 3, 1972, pages 475 - 480
- IWASAKI, H.: 'Samarium(II)-Mediated Spirocyclization by Intramolecular Aryl Radical Addition onto an Aromatic Ring' JOURNAL OF ORGANIC CHEMISTRY vol. 73, no. 18, 2008, pages 7145 - 7152
- MAKOTO NAKAMUTA: 'C-gata Kan'en Kan ni Okeru LXRa ni yoru Men'eki Kaihi Kiko no Kento' JAPANESE JOURNAL OF GASTROENTEROLOGY 2008, page 105
- NAKAMURA, M. ET AL.: 'Structural development studies of anti-hepatitis C virus agents with a phenanthridinone skeleton' BIOORGANIC & MEDICINAL CHEMISTRY vol. 18, no. 7, 2010, pages 2402 - 2411
- CEDRON, J. C.: 'Synthesis and antiplasmodial activity of lycorine derivatives' BIOORGANIC & MEDICINAL CHEMISTRY vol. 18, no. 13, 2010, pages 4694 - 4701
- FURUTA, T.: 'Synthesis of Axially Chiral Amino Acid and Amino Alcohols via Additive-Ligand- Free Pd-Catalyzed Domino Coupling Reaction and Subsequent Transformations of the Product Amidoaza[5]helicene' JOURNAL OF ORGANIC CHEMISTRY vol. 75, no. 20, 2010, pages 7010 - 7013

## Description

### Technical Field

The present invention relates to a therapeutic agent for hepatitis C and a novel compound having anti-HCV activity.

### Background Art

Human hepatitis C virus (HCV) is an RNA virus classified as the hepacivirus genus of the *Flaviviridae* family, and such virus was found in U.S.A. in 1989. In Japan, the number of HCV carriers is deduced to be approximately 1,600,000, which account for 1.3% of the total population. About half of patients infected with HCV develop acute hepatitis and about 70% of the acute hepatitis becomes chronic. When a disease state advances to chronic hepatitis, about 20% of such patients develop cirrhosis and eventually develop hepatic cancer. In Japan, 30,000 patients die of cirrhosis and 3,000 patients die of hepatic cancer every year. In addition, the number of HCV carriers is deduced to be about 100,000,000 all over the world (Non-Patent Documents 1 and 2).

Thus, the number of patients infected with HCV is large, and it is highly likely that such patients develop hepatic cancer, liver failure, or other disease without treatment and eventually die. Accordingly, research has been actively conducted regarding prevention, diagnosis, and treatment.

Regarding hepatitis C diagnosis, immunological analysis of HCV proteins and structural analysis of the HCV genome have been actively conducted, and HCV assay techniques involving the use of HCV-specific antibodies and quantitative examination techniques for the HCV genome had been developed.

At present, however, there are no effective vaccines for treatment of HCV, and treatment of HCV is limited to treatment with the use of interferon in combination with ribavirin (Non-Patent Document 3). However, interferon is less effective on HCV-1b, which is frequently observed in Japan, both agents have strong side effects, and they are expensive. Accordingly, development of a novel anti-HCV agent is desired.

In general, an anti-HCV agent exerts its pharmacological activity by blocking a certain step in an HCV life cycle. An HCV life cycle is as described below. HCV adhered to a host cell surface invades into the host cell via endocytosis. A precursor protein consisting of about 3,000 amino acid residues is produced from HCV genomic RNA that had invaded into the host cell. Such precursor protein is processed with NS3 and NS4 proteases encoded by the HCV genome or a signal peptidase of the host cell, and about 10 types of virus proteins, such as a capsid protein, an envelope protein, NS3 and NS4 proteases, and NS5B RNA polymerase, are produced. Thereafter, genomic RNA replicated by NS5B RNA polymerase aggregates with the capsid protein and the envelope protein mediated by α-glucosidase to form a virus particle. The resulting HCV virus particles are released from the host cell (Non-Patent Document 4).

Anti-HCV drugs, which were developed in the past and have been clinically tested at present, are classified as an RNA polymerase inhibitor-type, a protease inhibitor-type, an α-glucosidase inhibitor-type or other types, based on the HCV life cycle. For example, drugs of RNA polymerase inhibitor-type such as MK-7009 (nucleoside, Merck) and R7128 (nucleoside, Pharmasset/Roche) are at phase I of clinical trials, and VCH-759 (non-nucleoside, Virochem), R1626 (nucleoside, Roche), and valopicitabine (nucleoside, Idenix), are advanced to phase II. A drug of a protease inhibitor-type, ITMN-191 (R-7227, InterMune/Roche) is advanced to phase I, TMC435350 (Medivir/Tibotec) advanced to phase II, and Boceprevir (SCH 503034, Schering) and Telaprevir (Vertex) are advanced to phase III. In addition, a drug of a cyclophilin inhibitor-type, DEBIO-025 (DEBIO), and of a glucosidase I inhibitor-type, Celgosivir (MIGEBIX), are advanced to phase II (Non-Patent Documents 5 and 6).

As described above, several types of anti-HCV drugs have been developed, and clinical trials thereof are in progress. However, appearance of viruses that are resistant to such drugs has already been reported. Accordingly, development of a novel anti-HCV drug exerting anti-HCV activity with a different mechanism from those of known anti-HCV drugs is in an urgent need.

When anti-HCV drugs exerting anti-HCV activity with a same or similar mechanism to those of known anti-HCV drugs are to be developed, structures of such known anti-HCV drugs as lead compounds are modified as appropriate, so that many compounds having the similar structures can be prepared. In order to develop a novel anti-HCV drug exerting anti-HCV activity against drug-resistant viruses, however, it is necessary to screen compounds for discovery of a new lead compound while actually examining their pharmacological activities in an assay system with the use of HCV However, HCV cannot be cultured in test tubes except for certain virus strains. This makes an effective screening of lead compounds difficult and consequently impedes anti-HCV drug discovery and research via high-throughput screening.

As a means for overcoming such problem, a system for HCV genome replication in cultured cells that would not involve HCV production (the HCV replicon cell system) was developed. The HCV replicon cell which has been originally developed is a subgenomic replicon comprising NS3, NS4A, NS4B, NS5A, and NS5B regions and 5'- and 3'-non-translational regions necessary for HCV genome replication. At present, full-length replicons of the HCV genome have been prepared. Since the HCV genomes are autonomously replicated in the HCV replicon cell culture system, measurement of HCV RNA in the cells or immunochemical quantification of the level of HCV antigens expressed in the cells enable quantitative evaluation of activities of test compounds regarding HCV propagation (Non-Patent Document 7).

With the use of such HCV replicon cells, anti-HCV drug discovery and research have been carried out. For example, a ribavirin derivative (VX497 (IC₅₀= 0.5 µM)) and an NS3 protease inhibitor-type compound A (IC₅₀ = 1.4 µM) are reported to have activity of inhibiting the replication of the HCV genome (Non-Patent Document 8).

### Prior Art Documents

### Non-Patent Documents

Non-Patent Document 1: Liang, T.J., Rehermann, B., Seeff, L.B., Hoofnagle, J.H., Pathogenesis, natural history, treatment and prevention of hepatitis C., Ann. Intern. Med., 2000, vol. 132, pp. 296-305
Non-Patent Document 2: Kim, S.R., Kudo, M., Hino, O., Han, K.H., Chung, YH., Lee, H.S., Organizing Committee of Japan-Korea Liver Symposium, Epidemiology of hepatocellular carcinoma in Japan and Korea, A review, Oncology, 2008, vol. 75(S 1), pp. 13-16
Non-Patent Document 3: Hayashi, N., Takehara, T., Antiviral therapy for chronic hepatitis C: past, present, and future., J. Gastroenterol., 2006, vol. 41, pp. 17-27
Non-Patent Document 4: Manns, M.P., Foster, G.R., Rockstroh, J.K., Zeuzem, S., Zoulim, F., Houghton, M., The way forward in HCV treatment - finding the right path, Nat. Rev. Drug. Discov., 2007, vol. 6, pp. 991-1000
Non-Patent Document 5: Kronenberger, B., Welsch, C., Forestier, N., Zeuzem, S., Novel hepatitis C drugs in current trials, Clin Liver Dis., 2008, vol. 12, pp. 529-555
Non-Patent Document 6: HCV experimental treatment., [online], HIVandHepatitis.com, Internet <http://www.hivandhepatitis.com>
Non-Patent Document 7: Ishii, N., Watashi, K., Hishiki, T., Goto, K., Inoue, D., Hijikata, M., Wakita, T., Kato, N., Shimotohno, K., Diverse effects of cyclosporine on hepatitis C virus strain replication., J. Virol., 2006, vol. 80, pp. 4510-4520
Non-Patent Document 8: Nobuyuki Kato, Virus, vol. 52, No. 1, pp. 157-162, 2002

### Summary of the Invention

An object of the present invention is to provide a therapeutic agent for hepatitis C comprising, as an active ingredient, a compound having anti-HCV activity useful in treatment of hepatitis C. In order to overcome the drawbacks of prior art techniques; i.e., insufficient anti-HCV activity and strong side effects, specifically, the present invention is intended to develop a compound exhibiting high degree of anti-HCV activity and attenuated side effects, thereby providing pharmaceutical applications of such compound in the treatment of hepatitis C.

The present inventors examined various means for attaining the above objects. As a result, they have discovered that a group of compounds having a phenanthridin-6-one skeleton have high degree of anti-HCV activity. This has led to the completion of the present invention.

Specifically, the present invention is summarized as follows.
(1) A therapeutic agent for hepatitis C comprising, as an active ingredient, a compound represented by formula (I) or a pharmaceutically acceptable salt thereof: 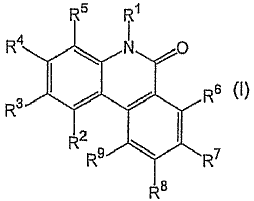 wherein,
   R¹ is selected from among C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl groups (each of these groups being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-Cg alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)); and
   R² to R⁹ are each independently selected from Substituent group A consisting of hydrogen, halogen, hydroxyl, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl) and Q-(heteroarylalkyl) (wherein Q is O or S), C₁-C₈ alkyl, C₂-C₈ alkenyl, C₂-C₈ alkenyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)),
   provided that any two of R² to R⁵ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)), or
   any two of R⁶ to R⁸ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S),
   with the exception of 5-butyl-1-methylphenanthridin-6(5H)-one, 5-butyl-3-methylphenanthridin-6(5H)-one, 5-butyl-4-methylphenanthridin-6(5H)-one, 5-butyl-7-methylphenanthridin-6(5H)-one, 5-butyl-8-methylphenanthridin-6(5H)-one, 5-butyl-9-methylphenanthridin-6(5H)-one, 5-butyl-10-methylphenanthridin-6(5H)-one, 5-butylbenzo[c]phenanthridin-6(5H)-one, and 5-butylbenzo[k]phenanthridin-6(5H)-one.
(2) A compound represented by formula (IA) or a salt thereof: 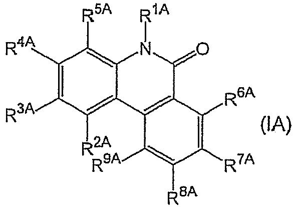 wherein,
   R^{1A} is either butyl or cyclohexylmethyl (each of these groups being independently unsubstituted or substituted with one or more groups selected from among halogen, OH, O-(C₂-C₈ alkynyl), O-(C₃-C₆ cycloalkyl), O-(C₃-C₆ cycloalkenyl), or O-(C₄-C₆ cycloalkynyl)); and
   R^{2A} to R^{9A} are each independently selected from Substituent group B consisting of hydrogen, halogen, hydroxyl, C(O)Z (wherein Z is hydrogen, hydroxyl, C₁-C₈ alkyl, or NH₂), Q-(C₁-C₈ alkyl), Q-(C₂-C₈ alkenyl), Q-(C₂-C₈ alkynyl) and Q-(C₃-C₆ cycloalkyl) (wherein Q is O or S), C₁-C₈ alkyl, C₂-C₈ alkenyl, C₂-C₈ allcynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, OH, and O-(arylalkyl)),
   provided that any two of R^{2A} to R^{5A} of R^{2A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), or
   any two of R^{6A} to R^{9A} of R^{2A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), C₁-C₈ alkyl, C₂-C₈ alkenyl, C₂-C₈ alkynyl, C₃-C₆ cycloalkyl, C₃-C₆ cycloalkenyl, or C₄-C₆ cycloalkynyl,
   with the exception of 5-butyl-1-methylphenanthridin-6(5H)-one, 5-butyl-3-methylphenanthridin-6(5H)-one, 5-butyl-4-methylphenanthridin-6(5H)-one, 5-butyl-7-methylphenanthridin-6(5H)-one, 5-butyl-8-methylphenanthridin-6(5H)-one, 5-butyl-9-methylphenanthridin-6(5H)-one, 5-butyl-10-methylphenanthridin-6(5H)-one, 5-butylbenzo[c]phenanthridin-6(5H)-one, 5-butylbenzo[k]phenanthridin-6(5H)-one, 5-butylphenanthridin-6(5H)-one, and 5-(4-bromobutyl)phenanthridin-6(5H)-one.
(3) A method for prevention or treatment of hepatitis C comprising administering an effective amount of the compound represented by formula (I) according to (1) to a subject who is in need of prevention or treatment of hepatitis C.
(4) The compound represented by formula (I) according to (1) or a salt thereof for use in prevention or treatment of hepatitis C.
(5) Use of the compound represented by formula (I) according to (1) or a salt thereof in the manufacture of a medicament for use in prevention or treatment of hepatitis C.
(6) A pharmaceutical composition comprising the compound represented by formula (I) according to (1) or a salt thereof and one ore more pharmaceutically acceptable carriers.

### Effects of the Invention

The present invention can provide a therapeutic agent for hepatitis C comprising, as an active ingredient, a compound having anti-HCV activity useful in treatment of hepatitis C.

This description includes part or all of the content as disclosed in the description and/or drawings of Japanese Patent Application No. 2010-020502, which is a priority document of the present application.

### Brief Description of the Drawings

Fig. 1 shows a structure ofNNC #2 (full-length replicon).
Fig. 2 shows a structure of #50-1 (subgenomic replion).

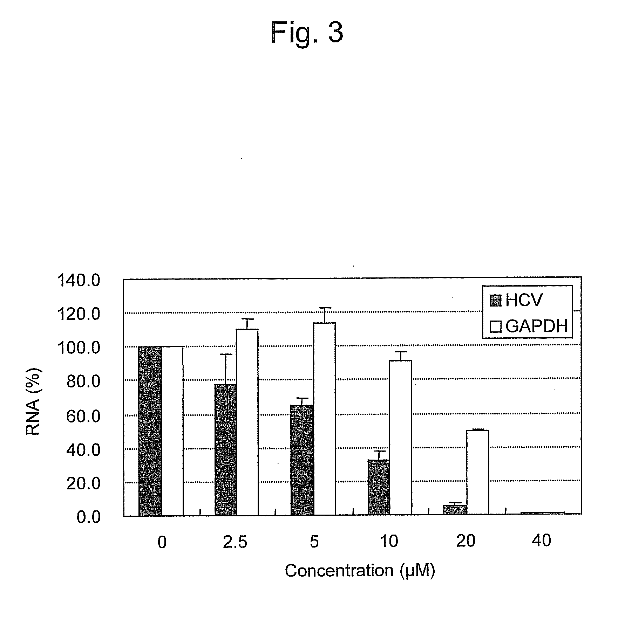

Fig. 3 shows anti-HCV activity of the compound represented by formula (I) (5-benzyl-2-(2'-hydroxy-1',1,1',3',3',3'-hexafluoropropan-2'-yl)-phenanthridin-6(5H)-one) observed in assays using full-length replion cells. A horizontal axis indicates concentration of the test compound (µM) and a vertical axis indicates an amount of viral RNA measured via real-time RT-PCR (the amount is indicated in percentage (%) relative to the amount of RNA of a control group to which the test compound was not administered). In Fig. 3, "HCV" indicates the measured amount of the HCV genomic RNA and "GAPDH" indicates the measured amount of GAPDH mRNA (the same applies to the figures described below).
Fig. 4 shows anti-HCV activity of the compound represented by formula (I) (5-butyl-2-(2'-hydroxy-1 1', 1',3',3',3'-hexafluoropropan-2'-yl)-phenanthridin-6(5H)-one) observed in assays using full-length replion cells.
Fig. 5 shows anti-HCV activity of the compound represented by formula (I) (5-hexyl-2-(2'-hydroxy-1',1',1',3',3',3'-hexafluoropropan-2'-yl)-phenanthridin-6(5H)-one) observed in assays using full-length replion cells.
Fig. 6 shows anti-HCV activity of the compound represented by formula (I) (5-cyclohexylmethyl-2-(2'-hydroxy-1',1',1',3',3',3'-hexafluoropropan-2'-yl)-phenanthridin-6(5H)-one) observed in assays using full-length replion cells.
Fig. 7 shows anti-HCV activity of the compound represented by formula (I) (5-butyl-2-(2'-hydroxy-1',1',1',3',3',3'-hexafluoropropan-2'-yl)-phenanthridin-6(5H)-one) observed in assays using subgenomic replion cells.
Fig. 8A shows anti-HCV activity of the compound represented by formula (I) (5-butyl-2-isopropylphenanthridin-6(5H)-one) observed in assays using full-length replion cells.
Fig. 8B shows anti-HCV activity of the compound represented by formula (I) (5-butyl-2-isopropylphenanthridin-6(5H)-one) observed in assays using subgenomic replion cells.
Fig. 9 shows anti-HCV activity of the compound represented by formula (I) (5-butylbenzo[j]phenanthridin-6(5H)-one) observed in assays using subgenomic replion cells.
Fig. 10 shows anti-HCV activity of the compound represented by formula (I) (5-butyl-2-tert-butylphenanthridin-6(5H)-one) observed in assays using subgenomic replion cells.
Fig. 11 shows anti-HCV activity of the compound represented by formula (I) (6-butyl-8,9,10,11-tetrahydro-8,8,11,11-tetramethylbenzo[2,3-b]phenanthridin-5(6H)-one) observed in assays using subgenomic replion cells.
Fig. 12 shows anti-HCV activity of the compounds represented by formula (I) (5-butyl-1-isopropylphenanthridin-6(5H)-one and 5-butyl-3-isopropylphenanthridin-6(5H)-one) observed in assays using subgenomic replion cells.
Fig. 13 shows anti-HCV activity of the compound represented by formula (I) (2-(2'-benzyloxy-1',1',1',3',3',3'-hexafluoropropan-2'-yl)-5-butyl-3-methoxyphenanthridin-6(5H)-one) observed in assays using subgenomic replion cells having a luciferase gene.
Fig. 14 shows anti-HCV activity of the compound represented by formula (I) (4-butyl-11-(1',1',1',3',3',3'-hexafluoro-2'-hydroxypropan-2'-yl)-7-methoxy-[1,3]dioxolo-[4,5-c]phenanthridin-5(4H)-one) observed in assays using subgenomic replion cells having a luciferase gene.
Fig. 15 shows anti-HCV activity of the compound represented by formula (I) (11-(2'-benzyloxy-1',1',1',3',3',3'-hexafluoropropan-2'-yl)-4-butyl-7-methoxy-[1,3]dioxolo-[4,5-c]phenanthridin-5(4H)-one) observed in assays using subgenomic replion cells having a luciferase gene.

### Embodiments of the Invention

Hereafter, preferable embodiments of the present invention are described in detail.

### 1. Therapeutic agent for hepatitis C comprising, as an active ingredient, a compound having the phenanthridine ring skeleton

The present invention relates to a therapeutic agent for hepatitis C comprising, as an active ingredient, a compound represented by formula (I) or a pharmaceutically acceptable salt thereof: 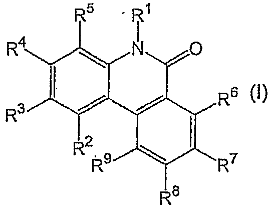 provided that 5-butyl-1-methylphenanthridin-6(5H)-one, 5-butyl-3-methylphenanthridin-6(5H)-one, 5-butyl-4-methylphenanthridin-6(5H)-one, 5-butyl-7-methylphenanthridin-6(5H)-one, 5-butyl-8-methylphenanthridin-6(5H)-one, 5-butyl-9-methylphenanthridin-6(5H)-one, 5-butyl- 1 0-methylphenanthridin-6(5H)-one, 5-butylbenzo[c]phenanthridin-6(5H)-one, and 5-butylbenzo[k]phenanthridin-6(5H)-one are excluded.

The term "alkyl" used herein refers to a linear or branched aliphatic hydrocarbon group having a specific number of carbon atoms. For example, the term "C₁-C₈ alkyl" refers to a linear or branched hydrocarbon chain having at least 1 and up to 8 carbon atoms. Examples of alkyl that can be preferably used include, but are not limited to, methyl, ethyl, n-propyl, isopropyl, n-butyl, sec-butyl, isobutyl, tert-butyl, n-pentyl, n-hexyl, n-heptyl, and n-octyl.

The term "alkenyl" used herein refers to a group resulting from substitution of one or more C-C single bonds of the alkyl with double bonds. Examples of preferable alkenyl include, but are not limited to, vinyl, 1-propenyl, allyl, 1-methylethenyl(isopropenyl), 1-butenyl, 2-butenyl, 3-butenyl, 1-methyl-2-propenyl, 2-methyl-2-propenyl, 1-methyl-1-propenyl, 2-methyl-1-propenyl, 1-pentenyl, 1-hexenyl, n-heptenyl, and 1-octenyl.

The term "alkynyl" used herein refers to a group resulting from substitution of one or more C-C single bonds of the alkyl with triple bonds. Examples of preferable alkynyl include, but are not limited to, ethynyl, 1-propinyl, 2-propinyl, 1-butynyl, 2-butynyl, 3-butynyl, 1-methyl-2-propinyl, 1-pentynyl, 1-hexynyl, 1-heptynyl, and 1-octynyl.

The term "cycloalkyl" used herein refers to alicyclic alkyl having a specific number of carbon atoms. For example, the term "C₃-C₆ cycloalkyl" refers to a cyclic hydrocarbon group having at least 3 and up to 6 carbon atoms. Examples of preferable cycloalkyl include, but are not limited to, cyclopropyl, cyclobutyl, cyclopentyl, and cyclohexyl.

The term "cycloalkenyl" used herein refers to a group resulting from substitution of one or more C-C single bonds of the cycloalkyl with double bonds. Examples of preferable cycloalkenyl include, but are not limited to, cyclopropenyl, cyclobutenyl, cyclopentenyl, and cyclohexenyl.

The term "cycloalkynyl" used herein refers to a group resulting from substitution of one or more C-C single bonds of the cycloalkyl with triple bonds. Examples of preferable cycloalkynyl include, but are not limited to, cyclobutynyl, cyclopentynyl, and cyclohexynyl.

The term "heterocyclyl" used herein refers to a group resulting from substitution of one or more carbon atoms of the cycloalkyl, cycloalkenyl, or cycloalkynyl with a hetero atom or hetero atoms selected from among nitrogen (N), sulfur (S), and oxygen (O). In this case, substitution with N or S includes substitution with N- or S-oxide or dioxide. Examples of preferable heterocyclyl include, but are not limited to, pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, and piperazinyl.

The term "aryl" used herein refers to an aromatic ring group having 6 to 15 carbon atoms. Examples of preferable aryl include, but are not limited to, phenyl, naphthyl, and anthryl (anthracenyl).

The term "arylalkyl" used herein refers to a group resulting from substitution of a hydrogen atom of the alkyl with the aryl. Examples of preferable arylalkyl include, but are not limited to, benzyl, 1-phenethyl, and 2-phenethyl.

The term "arylalkenyl" used herein refers to a group resulting from substitution of a hydrogen atom of the alkenyl with the aryl. An example of preferable arylalkenyl is, but is not limited to, styryl.

The term "heteroaryl" used herein refers to a group resulting from substitution of one or more carbon atoms of the aryl with a hetero atom or hetero atoms selected from among nitrogen (N), sulfur (S), and oxygen (O). In this case, substitution with N or S includes substitution with N- or S-oxide or dioxide. Examples of preferable heteroaryl include, but are not limited to, furanyl, thienyl, pyrrolyl, imidazolyl, pyrazolyl, triazolyl, tetrazolyl, thiazolyl, oxazolyl, isooxazolyl, oxadiazolyl, thiadiazolyl, isothiazolyl, pyridyl, pyridazinyl, pyrazinyl, pyrimidinyl, quinolinyl, isoquinolinyl, and indolyl.

The term "heteroarylalkyl" used herein refers to a group resulting from substitution of a hydrogen atom of the alkyl with the heteroaryl.

The groups described above can be each independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloallcyl, C₃-C₆ cycloalkenyl, C₄-C₆ cycloalkynyl, heterocyclyl, aryl, arylalkyl, arylalkenyl, heteroaryl, heteroarylallcyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S).

The term "halogen" or "halo" used herein refers to fluorine, chlorine, bromine, or iodine.

The term "salt" used herein preferably refers to a pharmaceutically acceptable salt. In this case, examples of preferable counter ions of a compound represented by formula (I) include, but are not limited to, cations such as sodium ions, potassium ions, calcium ions or magnesium ions, or anions such as chloride ions, bromide ions, formate ions, acetate ions, maleate ions, fumarate ions, benzoate ions, ascorbate ions, pamoate ions, succinate ions, bis-methylenesalicylate ions, methanesulfonate ions, ethane disulfonate ions, propionate ions, tartrate ions, salicylate ions, citrate ions, gluconate ions, aspartate ions, stearate ions, palmitate ions, itaconate ions, glycolate ions, p-aminobenzoate ions, glutamate ions, benzenesulfonate ions, cyclohexylsulfamate ions, methanesulfonate ions, ethanesulfonate ions, isethionate ions, benzenesulfonate ions, p-toluenesulfonate ions, naphthalenesulfonate ions, phosphate ions, nitrate ions, sulfate ions, carbonate ions, bicarbonate ions, and perchlorate ions.

The present inventors screened compounds for discovery of one having anti-HCV activity via assay systems using replicon cells. As a result, the present inventors have discovered that a compound having a phenanthridine ring skeleton represented by formula (I) has a high degree of anti-HCV activity.

In the compound represented by formula (I), it is preferable that R¹ is selected from among C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ allcenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)).

In the compound represented by formula (I), it is preferable that R² to R⁹ are each independently selected from Substituent group A consisting of hydrogen, halogen, hydroxyl, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl) and Q-(heteroarylalkyl) (wherein Q is O or S), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)).

In such a case, any two of R² to R⁵ of R² to R⁹ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)).

Alternatively, any two of R⁶ to R⁸ of R² to R⁹ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)).

In the compound represented by formula (I), it is preferable that R² to R⁹ satisfy any of the conditions [1] to [5] below:
[1] R⁵ to R⁹ are hydrogen atoms, any one of R² to R⁴ is a group selected from Substituent group A, and the other remaining groups are hydrogen atoms;
[2] R⁶ to R⁹ are hydrogen atoms, R² and R³ or R³ and R⁴ of R² to R⁵ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)), and the other remaining groups are hydrogen atoms;
[3] R² to R⁵ are hydrogen atoms, R⁶ and R⁷ or R⁷ and R⁸ of R⁶ to R⁹ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)), and the other remaining groups are hydrogen atoms;
[4] R³ is 2'-hydroxy-1',1',1',3',3',3'-hexafluoropropyl or 2'-benzyloxy-1',1',1',3',3',3'-hexafluoropropyl, up to 3 members of R² and R⁴ to R⁹ are each independently a group selected from Substituent group A, and the other remaining groups are hydrogen atoms; and
[5] R³ is 2'-hydroxy-1',1',1',3',3',3'-hexafluoropropyl or 2'-benzyloxy-1',1',1',3',3',3'- hexafluoropropyl, R⁴ and R⁵ or R⁷ and R⁸ of R² and R⁴ to R⁹ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)), any one of the other groups is selected from Substituent group A, and the other remaining groups are hydrogen atoms.

In the compound represented by formula (I), it is preferable that R¹ is a group selected from the group consisting of butyl, benzyl, hexyl, and cyclohexylmethyl. In this case, it is preferable that R² to R⁹ satisfy condition [1] and a group selected from Substituent group A is hydrogen, fluorine, trifluoromethyl, 1',1',1',3',3',3'-hexafluoropropyl, isopropyl, 2'-hydroxy-1',1',1',3',3',3'-hexafluoropropyl, or tert-butyl, with hydrogen being more preferable. Also, it is particularly preferable that R³ of R² to R⁴ is a group selected from Substituent group A. Alternatively, it is preferable that R² to R⁹ satisfy condition [2] or [3], and cycloalkyl, heterocyclyl, or aryl that is fused to the phenanthridine ring is independently unsubstituted or substituted with one or more C₁-C₈ alkyl groups. Alternatively, it is preferable that R² to R⁹ satisfy condition [4] and a group selected from Substituent group A is hydroxyl or methoxyl. Also, it is preferable that R² to R⁹ satisfy condition [5] and cycloalkyl, heterocyclyl, or aryl that is fused to the phenanthridine ring is unsubstituted 1,3-dioxole.

It is particularly preferable that the compound represented by formula (I) is selected from the group consisting of the compounds below:
5-butyl-2-(2'-hydroxy-1,1',1',3',3',3'-hexafluoropropan-2'-yl)-phenanthridin-6(5H)-one;
5-benzyl-2-(2'-hydroxy-1', 1', 1',3',3',3'-hexafluoropropan-2'-yl)-phenanthridin-6(5H)-one;
5-hexyl-2-(2'-hydroxy-1', 1', 1',3',3',3'-hexafluoropropan-2'-yl)-phenanthridin-6(5H)-one;
5-cyclohexylmethyl-2-(2'-hydroxy-1',1',1',3',3',3'-hexafluoropropan-2'-yl)-phenanthridin-6 (5H)-one;
5-butyl-1-isopropylphenanthridin-6(5H)-one;
5-butyl-2-isopropylphenanthridin-6(5H)-one;
5-butyl-3-isopropylphenanthridin-6(5H)-one;
5-butyl-2-tert-butylphenanthridin-6(5H)-one;
6-butylbenzo[a]phenanthridin-5(6H)-one;
6-butylbenzo[b]phenanthridin-5(6H)-one;
6-butylbenzo[i]phenantlu-idin-5(6H)-one;
5-butylbenzo[j]phenanthridin-6(5H)-one;
6-butyl-8,9,10,11-tetrahydro-8,8,11,11-tetramethylbenzo[2,3-b]phenanthridin-5(6H)-one;
2-((2'-benzyloxy-1',1',1',3',3',3'-hexafluoropropan-2'-yl)-5-butyl-3-methoxyphenanthridin-6(5H)-one;
4-butyl-11-(1',1',1',3',3',3'-hexafluoro-2'-hydroxypropan-2'-yl)-7-methoxy-[1,3]dioxolo[4,5-c]phenanthridin-5(4H)-one; and
11-((2'-benzyloxy-1',1',1',3',3',3'-hexafluoropropan-2'-yl)-4-butyl-7-methoxy-[1,3]dioxolo[4,5-c]phenanthridin-5(4H)-one.

Some compounds represented by formula (I) are known. For example, the literature of the present inventors (Aoyama, A. et al., Heterocycles, vol. 76, pp. 137-142, 2008) discloses that a compound represented by formula (I) (wherein R¹ represents methyl, 1',1',1'-trifluoroethyl,or benzyl; R³ is 2'-hydroxy-1',1',1',3',3',3'-hexafluoropropan-2-yl; and R² and R⁴ to R⁹ are hydrogen atoms) has liver X receptor (LXR) antagonistic activity. However, the fact that a compound represented by formula (I) has a high degree of anti-HCV activity was not demonstrated in the past, and it has been first discovered by the present inventors.

Also, the present invention relates to a compound represented by formula (IA) or a salt thereof: 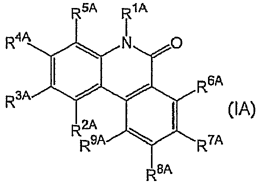 wherein,
R^{1A} is either butyl or cyclohexylmethyl (each of these groups being independently unsubstituted or substituted with one or more groups selected from among halogen, OH, O-(C₂-C₈ alkynyl), O-(C₃-C₆ cycloalkyl), O-(C₃-C₆ cycloalkenyl), or O-(C₄-C₆ cycloalkynyl)); and
R^{2A} to R^{9A} are each independently selected from Substituent group B consisting of hydrogen, halogen, hydroxyl, C(O)Z (wherein Z is hydrogen, hydroxyl, C₁-C₈ alkyl, or NH₂), Q-(C₁-C₈ alkyl), Q-(C₂-C₈ alkenyl), Q-(C₂-C₈ alkynyl) and Q-(C₃-C₆ cycloalkyl) (wherein Q is O or S), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, OH, or O-(arylalkyl)),
provided that any two of R^{2A} to R^{5A} of R^{2A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), or
any two of R^{6A} to R^{9A} of R^{2A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), C₁-C₈ alkyl, C₂-C₈ alkenyl, C₂-C₈ alkynyl, C₃-C₆ cycloalkyl, C₃-C₆ cycloalkenyl, or C₄-C₆ cycloalkynyl,
with the exception of 5-butyl-1-methylphenanthridin-6(5H)-one, 5-butyl-3-methylphenanthridin-6(5H)-one, 5-butyl-4-methylphenanthridin-6(5H)-one, 5-butyl-7-methylphenanthridin-6(5H)-one, 5-butyl-8-methylphenanthridin-6(5H)-one, 5-butyl-9-methylphenanthridin-6(5H)-one, 5-butyl-10-methylphenanthridin-6(5H)-one, 5-butylbenzo [c]phenanthridin-6(5H)-one, 5-butylbenzopc]phenanthridin-6(5H)-one, 5-butylphenanthridin-6(5H)-one, and 5-(4-bromobutyl)phenanthridin-6(5H)-one.

A compound represented by formula (IA) is within the scope of the compound represented by formula (I), which is a novel compound discovered by the present inventors.

In the compound represented by formula (IA), it is preferable that R^{2A} to R^{9A} satisfy any of the conditions [1A] to [5A] below:
[1A] R^{5A} to R^{9A} are hydrogen atoms, any one of R^{2A} to R^{4A} is a group selected from Substituent group B, and the other remaining groups are hydrogen atoms;
[2A] R^{6A} to R^{9A} are hydrogen atoms, R^{2A} and R^{3A} or R^{3A} and R^{4A} of R^{2A} to R^{5A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), and the other remaining groups are hydrogen atoms;
[3A] R^{2A} to R^{5A} are hydrogen atoms, R^{6A} and R^{7A} or R^{7A} and R^{8A} of R^{6A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), and the other remaining groups are hydrogen atoms;
[4A] R^{3A} is 2'-hydroxy-1',1',1',3',3',3'-hexafluoropropyl or 2'-benzyloxy-1',1',1',3',3',3'-hexafluoropropyl, up to 3 members of R^{2A} and R^{4A} to R^{9A} are each independently a group selected from Substituent group B, and the other remaining groups are hydrogen atoms; and
[5A] R^{3A} is 2'-hydroxy-1',1',1',3',3',3'-hexafluoropropyl or 2'-benzyloxy-1',1',1',3',3',3'-hexafluoropropyl, R^{4A} and R^{5A} or R^{7A} and R^{8A} of R^{2A} and R^{4A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), any one of the other groups is selected from Substituent group B, and the other remaining groups are hydrogen atoms.

In the compound represented by formula (IA), R^{1A} is preferably butyl. In this case, it is preferable that R^{2A} to R^{9A} satisfy condition [1A] and a group selected from Substituent group B is fluorine, trifluoromethyl, 1',1',1',3',3',3'-hexafluoropropyl, isopropyl, 2'-hydroxy-1',1',1',3',3',3'-hexafluoropropyl, or tert-butyl. It is particularly preferable that R^{3A} of R^{2A} to R^{4A} is a group selected from Substituent group B. Alternatively, it is preferable that R^{2A} to R^{9A} satisfy any of the conditions [2A] to [5A].

It is particularly preferable that a compound represented by formula (IA) is selected from the group consisting of the compounds below:
5-butyl-2-(2'-hydroxy-1',1',1',3',3',3'-hexafluoropropan-2'-yl)-phenanthridin-6(5H)-one;
5-cyclohexylmethyl-2-(2'-hydroxy-1',1',1',3',3',3'-hexafluoropropan-2'-yl)-phenanthridin-6(5H)-one;
5-butyl-1-isopropylphenanthridin-6(5H)-one;
5-butyl-2-isopropylphenanthridin-6(5H)-one;
5-butyl-3-isopropylphenanthridin-6(5H)-one;
5-butyl-2-tert-butylphenanthridin-6(5H)-one;
6-butylbenzo[a]phenanthridin-5(6H)-one;
6-butylbenzo[b]phenanthridin-5(6H)-one;
6-butylbenzo [i]phenanthridin-5 (6H)-one;
5-butylbenzo[j]phenanthridin-6(5H)-one;
6-butyl-8,9,10,11-tetrahydro-8,8,11,11-tetramethylbenzo[2,3-b]phenanthridin-5(6H)-one;
2-((2'-benzyloxy-1',1',1',3',3',3'-hexafluoropropan-2'-yl)-5-butyl-3-methoxyphenanthridin-6(5H)-one;
4-butyl-11-(1',1',1',3',3',3'-hexafluoro-2'-hydroxypropan-2'-yl)-7-methoxy-[1,3]dioxolo[4,5-c]phenanthridin-5(4H)-one; and
11-((2'-benzyloxy-1',1',1',3',3',3'-hexafluoropropan-2'-yl)-4-butyl-7-methoxy-[1,3]dioxolo[4,5-c]phenanthridin-5(4H)-one.

The compound represented by formula (I) or (IA) has a high degree of anti-HCV activity. Drugs having anti-HCV activity are known to exert satisfactory therapeutic effects in treatment of hepatitis C, as established by the typical therapeutic agent for hepatitis C, interferon and ribavirin. Thus, the compound represented by formula (I) or (IA) or a pharmaceutically acceptable salt thereof is useful in treatment of hepatitis C.

At present, there are no effective HCV vaccines, and anti-HCV drugs are utilized in prevention of hepatitis C, in addition to treatment of hepatitis C, in general. Therefore, the compound represented by formula (I) or (IA) or a pharmaceutically acceptable salt thereof is useful in prevention of hepatitis C, in addition to treatment of hepatitis C.

Because of the feature of the compound represented by formula (I) or (IA); that is, it has a high degree of anti-HCV activity, a therapeutic agent for hepatitis C comprising, as an active ingredient, the compound represented by formula (I) or (IA) or pharmaceutically acceptable salt thereof can be provided.

The therapeutic agent for hepatitis C of the present invention is safe and low in toxicity. Accordingly, administration thereof to a subject or patient who is in need of treatment or prevention of hepatitis C, such as a human or non-human mammalian (e.g., a mouse, rat, guinea pig, rabbit, chicken, sheep, pig, cow, cat, dog, monkey, baboon, or chimpanzee) leads to treatment, alleviation, and/or prevention of diseases or symptoms caused by hepatitis C.

When the therapeutic agent for hepatitis C of the present invention is administered to a subject who is in need of treatment or prevention of hepatitis C, a human subject (patient), in particular, a primary doctor should make definitive decisions regarding an accurate dose and the frequency of administration based on many factors, such as age and sexuality of the subject or patient, precise symptoms to be treated, the severity thereof, and the route of administration.

When the therapeutic agent for hepatitis C of the present invention is used in treatment of hepatitis C of a human, in general, a recommended dose of the compound represented by formula (I) or (IA) or a salt thereof is preferably from 0.01 to 100 mg, and more preferably from 0.1 to 10 mg per kg of body weight per day. In the case of an adult subject, a dose is generally from 0.6 to 6,000 mg, and preferably from 6 to 600 mg per day. The aforementioned amount of the therapeutic agent for hepatitis C of the present invention may be administered in a single instance. Alternatively, administration may be made, for example, in a single instance to several separate instances per day.

The therapeutic agent for hepatitis C of the present invention can be formulated into a variety of dosage forms known in the art, in accordance with relevant methods of administration. For example, the compound represented by formula (I) or (IA) or a salt thereof may be mixed with additives, such as one or more types of pharmaceutically acceptable carriers, excipients, binders, vehicles, solubilizers, preservatives, stabilizers, bulking agents, lubricants, surfactants, oily fluid, buffer, soothing agents, antioxidants, sweetening agents, or flavoring agents to prepare the pharmaceutical formulation in a unit dosage or multiple dosage form.

Such pharmaceutical formulations can be subjected to various types of administration techniques that are commonly employed in the art. For example, the pharmaceutical formulations can be in the form of tablets with sugar coatings or soluble coatings, capsules, elixirs, microcapsules, tablets, syrup agents, suspensions, or the like according to need, and such formulations can be subjected to oral administration. Examples of additives that can be mixed with tablets, capsules, and the like include, but are not limited to, binders, such as gelatin, corn starch, gum tragacanth or gum arabic, excipients, such as crystalline cellulose, bulking agents, such as corn starch, gelatin or alginic acid, lubricants, such as magnesium stearate, sweetening agents, such as sucrose, lactose, and saccharin, and flavoring agents, such as peppermint, *Gaultheria adenothrix* oil, and cherry. When pharmaceutical formulations are in the form of capsules, liquid carriers such as oil and fat may be incorporated therein.

The therapeutic agent for hepatitis C of the present invention can be formulated in the form of injection formulations thereof in a sterile solution or suspension in water or other types of pharmaceutically acceptable liquids, and the resulting formulations can be subjected to parenteral administration via, for example, intravascular administration into the vein, subcutaneous injection, or continuous infusion. Examples of additives that can be mixed with such injection formulations include, but are not limited to, vehicles, such as isotonic solutions containing physiological saline, glucose, or other types of adjuvants (e.g., D-sorbitol, D-mannitol, or sodium chloride), solubilizers, such as alcohols (e.g., ethanol and benzyl alcohol), esters (e.g., benzyl benzoate), polyalcohols (e.g., propylene glycol and polyethylene glycol), nonionic surfactants, such as polysorbate 80 and polyoxyethylene hydrogenated castor oil, oily fluids, such as sesame oil and soybean oil, buffers, such as phosphate buffer and sodium acetate buffer, soothing agents, such as benzalkonium chloride and procaine hydrochloride, stabilizers, such as human serum albumin and polyethylene glycol, preservatives, and antioxidants. In general, the resulting injection formulations are filled in appropriate ampules and then stored under suitable conditions before use.

The therapeutic agent for hepatitis C of the present invention can be formulated in the form of a depot preparation. For example, such long-acting pharmaceutical formulations can be implanted subcutaneously or intramuscularly, or such formulations can be administered via intramuscular injection. Thus, the therapeutic agent for hepatitis C of the present invention can be formulated in the form of a depot formulation together with appropriate polymeric or hydrophobic substances or ion-exchange resin. Alternatively, the therapeutic agent can be derivatized to a less-soluble derivative, such as a hardly-soluble salt.

In addition, the therapeutic agent for hepatitis C of the present invention can be subjected to any administration techniques, such as direct administration (e.g., topical administration).

The therapeutic agent for hepatitis C of the present invention can be used in combination with other therapeutic agents that are useful as a medicament. In such a case, other therapeutic agents may be added to the therapeutic agent for hepatitis C of the present invention to prepare a single pharmaceutical formulation, and the resulting single pharmaceutical formulation may be used. Alternatively, a plurality of pharmaceutical formulations independently comprising the therapeutic agent for hepatitis C of the present invention separately from other therapeutic agents may be used. Such combinations of the formulations can be administered continuously or simultaneously.

The therapeutic agent for hepatitis C of the present invention may comprise the compound represented by formula (I) or (IA) in the form of a precursor or prodrug compound that is converted into the compound represented by formula (I) or (IA) by metabolic reactions *in vivo.* This form is within the scope of the present invention.

The present invention can provide a method for prevention or treatment of hepatitis C comprising administering an effective amount of the compound represented by formula (I) or (IA) to a subject who is in need of prevention or treatment of hepatitis C. Examples of subjects of the method for prevention or treatment of the present invention include humans or nonhuman mammalians described above that are in need of prevention or treatment of hepatitis C.

Further, the present invention can provide the compound represented by formula (I) or (IA) for use in prevention or treatment of hepatitis C or a salt thereof.

The compound represented by formula (I) or (IA) of the present invention or a salt thereof can be used for pharmaceutical applications described above, so that hepatitis C can be prevented or treated.

### 2. Method for producing a compound having a phenanthridine ring skeleton

The compound represented by formula (I) or a salt thereof can be prepared by the method shown in scheme 1.

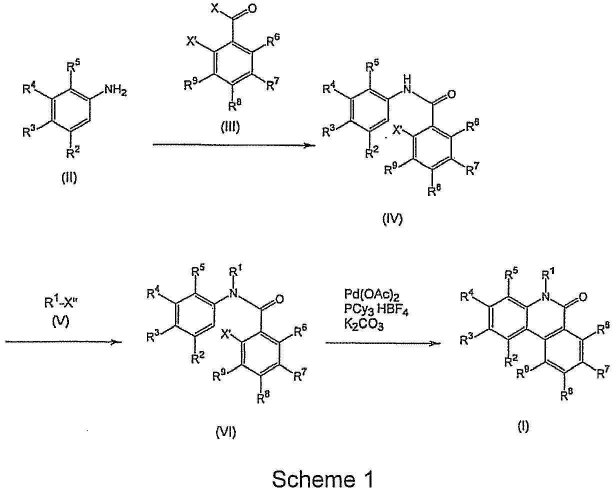

In scheme 1, R¹ to R⁹ are as defined above and X, X', and X" are each independently selected from among halogen atoms.

In scheme 1, the aniline derivative represented by formula (II) or a protected derivative thereof is allowed to be bound to a benzoyl halide represented by formula (III) or a protected derivative thereof in an aprotic solvent, such as dichloromethane or N,N-dimethylformamide, in the presence of a base, such as triethylamine or N,N-diisopropylethylamine, and a benzanilide represented by formula (IV) or a protected derivative thereof is consequently generated (step (1A)). Subsequently, the benzanilide represented by formula (IV) or a protected derivative thereof is allowed to react with a halide represented by formula (V) or a protected derivative thereof in an aprotic polar solvent, such as N,N-dimethylformamide, in the presence of a hydride reagent such as sodium hydride, and a N-substituted benzanilide represented by formula (VI) or a protected derivative thereof is consequently generated (step (1B)). Thereafter, the N-substituted benzanilide represented by formula (VI) or a protected derivative thereof is subjected to an intramolecular cyclization reaction in an aprotic polar solvent, such as N,N-dimethylacetamide, in the presence of palladium acetate, tricyclohexylphosphine-tetrafluoroborate, and potassium carbonate, and a compound represented by formula (I) or a protected derivative thereof is consequently generated (step (1C)). When a protected derivative of the compound represented by formula (I) is generated in the step above, the protected derivative of the compound represented by formula (I) is appropriately deprotected, and a compound represented by formula (I) or a salt thereof is consequently generated (step (1D)).

When the compound represented by formula (I) in which R¹ is n-butyl, R³ is hydroxyl, and the other remaining groups are hydrogen atoms or a salt thereof is prepared, it is preferable that the method shown in scheme 2 is employed.

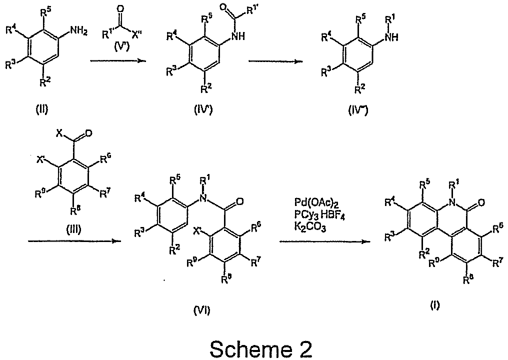

In scheme 2, R¹ to R⁹ are as defined above, R^{1'} is selected from among C₁-C₇ alkyl, C₂-C₇ alkenyl, C₂-C₇ alkynyl, arylalkyl, arylalkenyl, and heteroarylalkyl (each of these groups being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-C₁-C₈ alkyl, Q-C₂-C₈ alkenyl, Q-C₂-C₈ alkynyl, Q-C₃-C₆ cycloalkyl, Q-C₃-C₆ cycloalkenyl, Q-C₄-C₆ cycloalkynyl, Q-heterocyclyl, Q-aryl, Q-arylalkyl, Q-heteroaryl, or Q-heteroarylalkyl (wherein Q is O or S)), and X, X', and X" are each independently selected from among halogen atoms.

In scheme 2, the aniline derivative represented by formula (II) or a protected derivative thereof is allowed to react with a carboxylic acid halide represented by formula (V') or a protected derivative thereof in an aprotic solvent, such as dichloromethane or N,N-dimethylformamide, in the presence of a base, such as triethylamine or N,N-diisopropylethylamine, and a carboxylic acid anilide represented by formula (IV') or a protected derivative thereof is consequently generated (step (2A)). Subsequently, the carboxylic acid anilide represented by formula (IV') or a protected derivative thereof is subjected to reduction in an aprotic polar solvent, such as tetrahydrofuran, in the presence of a reducing reagent such as aluminum lithium hydride, and a N-substituted aniline represented by formula (IV") or a protected derivative thereof is consequently generated (step (2B)). Subsequently, the N-substituted aniline represented by formula (IV") or a protected derivative thereof is allowed to be bound to a benzoyl halide represented by formula (III) or a protected derivative thereof in an aprotic solvent, such as dichloromethane or N,N-dimethylformamide, in the presence of a base, such as triethylamine or N,N-diisopropylethylamine, and a N-substituted benzanilide represented by formula (VI) or a protected derivative thereof is consequently generated (step (2C)). Thereafter, the N-substituted benzanilide represented by formula (VI) or a protected derivative thereof is subjected to an intramolecular cyclization reaction in an aprotic polar solvent, such as N,N-dimethylacetamide, in the presence of palladium acetate, tricyclohexylphosphine-tetrafluoroborate, and potassium carbonate, and a compound represented by formula (I) or a protected derivative thereof is consequently generated (step (2D)). When a protected derivative of the compound represented by formula (I) is generated in the step above, the protected derivative of the compound represented by formula (I) is appropriately deprotected, and a compound represented by formula (I) or a salt thereof is consequently generated (step (2E)).

When the compound represented by formula (I) in which R¹ is n-butyl, R⁶ to R⁹ are hydrogen atoms, R² and R³ or R³ and R⁴ of R² to R⁵ together with carbon atoms on the phenanthridine ring to which they are bound may form aryl that is fused to a phenanthridine ring and the other remaining groups are hydrogen atoms or a salt thereof is prepared, it is preferable that the method shown in scheme 3 is employed.

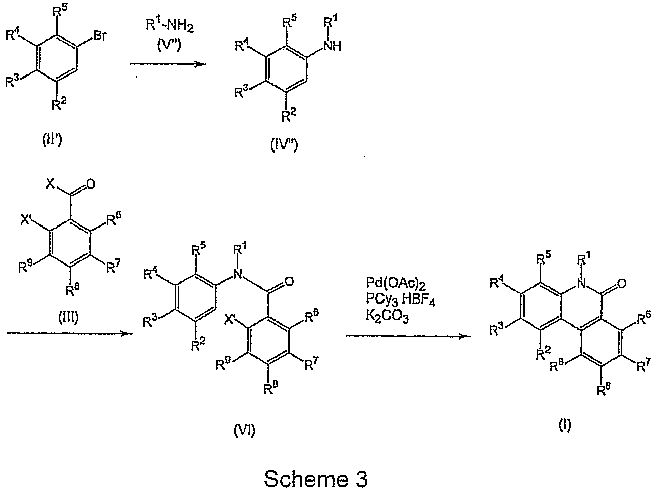

In scheme 3, R¹ to R⁹ are as defined above and X, X', and X" are each independently selected from among halogen atoms.

In scheme 3, the bromobenzene represented by formula (II') or a protected derivative thereof is allowed to react with a primary amine represented by formula (V") or a protected derivative thereof in an aprotic solvent, such as toluene, in the presence of tris(dibenzylideneacetone)dipalladium (Pd₂(dba)₃), sodium tert-butoxide (tert-BuONa), and 2,2'-bis(diphenylphosphino)-1,1'-binaphthyl (BINAP), and a N-substituted aniline represented by formula (IV") or a protected derivative thereof is consequently generated (step (3A)). Subsequently, the N-substituted aniline represented by formula (IV") or a protected derivative thereof is allowed to be bound to a benzoyl halide represented by formula (III) or a protected derivative thereof in an aprotic solvent, such as dichloromethane or N,N-dimethylformamide, in the presence of a base, such as triethylamine or N,N-diisopropylethylamine, and a N-substituted benzanilide represented by formula (VI) or a protected derivative thereof is consequently generated (step (3B)). Thereafter, the N-substituted benzanilide represented by formula (VI) or a protected derivative thereof is subjected to an intramolecular cyclization reaction in an aprotic polar solvent, such as N,N-dimethylacetamide, in the presence of palladium acetate, tricyclohexylphosphine-tetrafluoroborate, and potassium carbonate, and a compound represented by formula (I) or a protected derivative thereof is consequently generated (step (3C)). When a protected derivative of the compound represented by formula (I) is generated in the step above, the protected derivative of the compound represented by formula (I) is appropriately deprotected, and the compound represented by formula (I) or a salt thereof is consequently generated (step (3D)).

When the compound represented by formula (I) in which R¹ is n-butyl and R³ is 2'-hydroxy-1',1',1',3',3',3'-hexafluoropropyl or 2'-benzyloxy-1',1',1',3',3',3'-hexafluoropropyl or a salt thereof is prepared, it is preferable that the method shown in scheme 4 is employed.

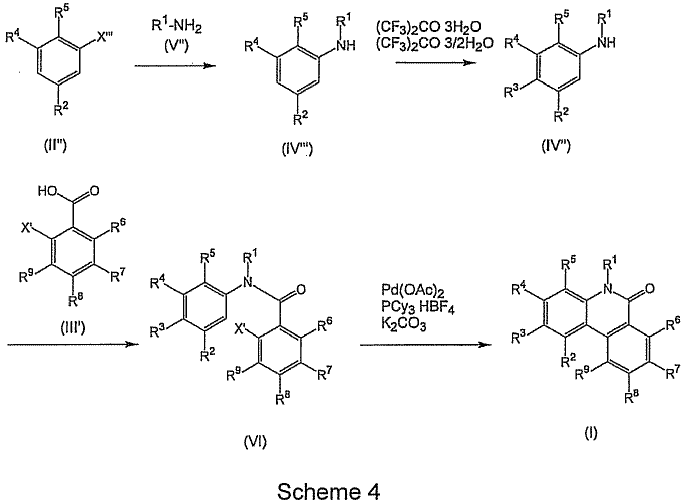

In scheme 4, R¹ to R⁹ are as defined above and X' and X'" are each independently selected from among halogen atoms.

In scheme 4, the halobenzene represented by formula (II") or a protected derivative thereof is allowed to react with a primary amine represented by formula (V") or a protected derivative thereof in an aprotic solvent, such as anhydrous dimethyl sulfoxide, in the presence of copper iodide, L-proline, and potassium carbonate, and a N-substituted aniline represented by formula (IV"') or a protected derivative thereof is consequently generated (step (4A)). Subsequently, the N-substituted aniline represented by formula (IV"') or a protected derivative thereof is allowed to react with hexafluoroacetone sesquihydrate, hexafluoroacetone trihydrate, and p-toluenesulfonate monohydrate, in an aprotic solvent, such as anhydrous toluene, in the presence of a molecular sieve, such as Molecular Sieve 4A, and a N-substituted aniline represented by formula (IV") or a protected derivative thereof is consequently generated (step (4B)). Subsequently, the N-substituted aniline represented by formula (IV") or a protected derivative thereof is allowed to be bound to a halobenzoic acid or a protected derivative thereof represented by formula (III') in an aprotic solvent, such as dichloromethane or N,N-dimethylformamide, in the presence of a base, such as triethylamine and N,N-diisopropylethylamine, and a N-substituted benzanilide represented by formula (VI) or a protected derivative thereof is consequently generated (step (4C)). Thereafter, the N-substituted benzanilide represented by formula (VI) or a protected derivative thereof is subjected to an intramolecular cyclization reaction in an aprotic polar solvent, such as N,N-dimethylacetamide, in the presence of palladium acetate, tricyclohexylphosphine-tetrafluoroborate, and potassium carbonate, and a compound represented by formula (I) or a protected derivative thereof is consequently generated (step (4D)). When a protected derivative of the compound represented by formula (I) is generated in the step above, the protected derivative of the compound represented by formula (I) is appropriately deprotected, and a compound represented by formula (I) or a salt thereof is consequently generated (step (4E)).

When the compound represented by formula (I) in which R¹ is n-butyl and R³ is 2'-hydroxy-1',1',1',3',3',3'-hexafluoropropyl or 2'-benzyloxy-1',1',1',3',3',3'-hexafluoropropyl or a salt thereof is prepared, alternatively, it is preferable that the method shown in scheme 5 is employed.

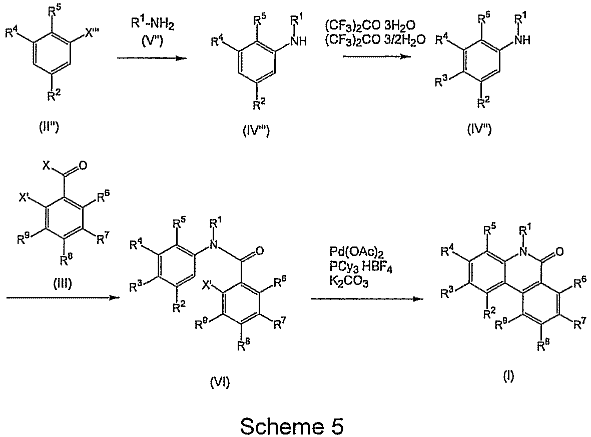

In scheme 5, R¹ to R⁹ are as defined above and X, X', and X"' are each independently selected from among halogen atoms.

In scheme 5, the halobenzene represented by formula (II") or a protected derivative thereof is allowed to react with a primary amine represented by formula (V") or a protected derivative thereof in an aprotic solvent, such as anhydrous dimethyl sulfoxide, in the presence of copper iodide, L-proline, and potassium carbonate, and a N-substituted aniline represented by formula (IV"') or a protected derivative thereof is consequently generated (step (5A)). Subsequently, the N-substituted aniline represented by formula (IV"') or a protected derivative thereof is allowed to react with hexafluoroacetone sesquihydrate, hexafluoroacetone trihydrate, and p-toluenesulfonate monohydrate in an aprotic solvent, such as anhydrous toluene, in the presence of a molecular sieve, such as Molecular Sieve 4A, and a N-substituted aniline represented by formula (IV") or a protected derivative thereof is consequently generated (step (5B)). Subsequently, the N-substituted aniline represented by formula (IV") or a protected derivative thereof is allowed to be bound to a benzoyl halide represented by formula (III) or a protected derivative thereof in an aprotic solvent, such as dichloromethane or N,N-dimethylformamide, in the presence of a base, such as triethylamine or N,N-diisopropylethylamine, and a N-substituted benzanilide represented by formula (VI) or a protected derivative thereof is consequently generated (step (5C)). Thereafter, the N-substituted benzanilide represented by formula (VI) or a protected derivative thereof is subjected to an intramolecular cyclization reaction in an aprotic polar solvent, such as N,N-dimethylacetamide, in the presence of palladium acetate, tricyclohexylphosphine-tetrafluoroborate, and potassium carbonate, and a compound represented by formula (I) or a protected derivative thereof is consequently generated (step (5D)). When a protected derivative of the compound represented by formula (I) is generated in the step above, the protected derivative of the compound represented by formula (I) is appropriately deprotected, and a compound represented by formula (I) or a salt thereof is consequently generated (step (5E)).

In schemes 1 to 5, the compounds represented by formulae (II) to (VI), (II'), (II"), (IV'), (IV"), (IV"'), (V'), and (V") are known compounds, or such compounds can be prepared in accordance with methods described in well-known literature.

In schemes 1 to 5, it is necessary or desirable to protect one or more sensitive groups in the molecule at any stage of the process for preparing a compound represented by formula (I) to avoid unpreferable side effects. This is obvious to a person skilled in the art. Schemes 1 to 5, accordingly, may involve a further step of introducing an appropriate protective group into a sensitive group contained in the compound represented by any of formulae (II) to (VI), (II'), (II"), (IV'), (IV"), (IV"'), (V'), and (V") to generate a protected derivative of the compound, or preparing a protected derivative of the compound into which an appropriate group has been introduced in advance.

Any protective group known in the art can be used for preparing a protected derivative of the compound represented by formula (I). A method of preparing a protected derivative via introduction of such protective group or a method of deprotecting such protected group known in the art can be appropriately employed. Examples of preferable protective groups used in preparing a protected derivative of the compound represented by formula (I) include, but are not limited to, benzyl (Bn), tert-butyldimethylsilyl (TBS), and methoxyethoxymethyl groups in the case of a hydroxyl protecting group. 4-Methoxybenzyl or the like is preferable in the case of an amino group protecting group.

According to scheme 1, the compound represented by formula (IA) or a salt thereof can be prepared by a method comprising the steps described below:
step (1A) of allowing an aniline derivative represented by formula (II): 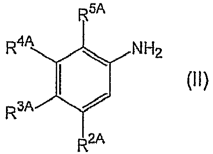 wherein
   R^{2A} to R^{5A} are as defined above,
   to be bound to a benzoyl halide represented by formula (III): 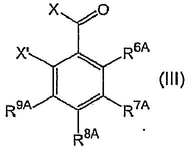 wherein
   R^{6A} to R^{9A} are as defined above; and
   X and X' are each independently selected from among halogen atoms,
   to generate a benzanilide represented by formula (IV): 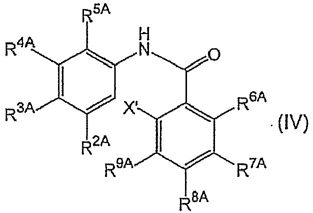 wherein
   R^{2A} to R^{9A} and X' are as defmed above;
step (1B) of allowing the benzanilide represented by formula (IV) to react with a halide represented by formula (V):

   R^{1A}-X" (V)

   wherein
   R^{1A} is as defined above; and
   X" is halogen,
   to generate a N-substituted benzanilide represented by formula (VI): 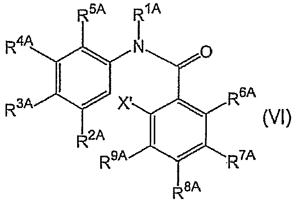 wherein
   R^{1A} to R^{9A} and X' are as defined above; and
step (1C) of subjecting the N-substituted benzanilide represented by formula (VI) to an intramolecular cyclization reaction in the presence of palladium acetate to generate the compound represented by formula (IA).

When a protected derivative of the compound represented by formula (IA) is generated via step (1C), the method may further comprise a step of deprotection described above and step (1D) below:
step (1D) of deprotecting the protected derivative of the compound represented by formula (IA) to generate the compound represented by formula (IA) or a salt thereof.

According to scheme 2, the compound represented by formula (IA) in which R^{1A} is n-butyl, R^{3A} is hydroxyl, and the other remaining groups are hydrogen atoms or a salt thereof is preferably prepared by the method comprising the steps described below:
step (2A) of allowing an aniline derivative represented by formula (II): 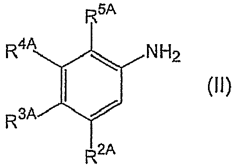 wherein
   R^{2A} to R^{5A} are as defined above,
   to react with a carboxylic acid halide represented by formula (V'):

   R^{1A'}-CO-X" (V')

   wherein
   R^{1A'} is selected from among propyl, phenyl, and cyclohexyl (each of these groups being independently unsubstituted or substituted with one or more groups selected from among halogen, OH, O-C₂-C₈ alkynyl, O-C₃-C₆ cycloalkyl, O-C₃-C₆ cycloalkenyl, or O-C₄-C₆ cycloalkynyl); and
   X" is halogen,
   to generate a carboxylic acid anilide represented by formula (IV'): 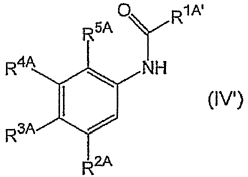 wherein
   R^{1A'} and R^{2A} to R^{5A} are as defined above;
step (2B) of subjecting the carboxylic acid anilide represented by formula (IV') to reduction to generate a N-substituted aniline represented by formula (IV"): 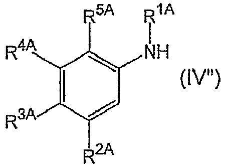 wherein
   R^{1A} to R^{5A} are as defined above; and
step (2C) of allowing the N-substituted aniline represented by formula (IV") to be bound to a benzoyl halide represented by formula (III): 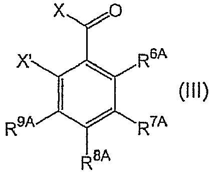 wherein
   R^{6A} to R^{9A} are as defined above; and
   X and X' are each independently selected from among halogen atoms,
   to generate a N-substituted benzanilide represented by formula (VI): 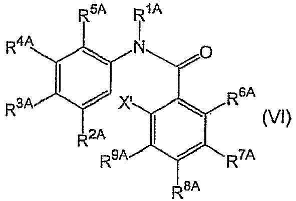 wherein
   R^{1A} to R^{9A} and X' are as defined above; and
step (2D) of subjecting the N-substituted benzanilide represented by formula (VI) to an intramolecular cyclization reaction in the presence of palladium acetate to generate the compound represented by formula (IA).

When a protected derivative of the compound represented by formula (IA) is generated via step (2D), the method may further comprise a step of deprotection described above and step (2E) below:
step (2E) of deprotecting the protected derivative of the compound represented by formula (IA) to generate the compound represented by formula (IA) or a salt thereof.

According to scheme 3, further, the compound represented by formula (IA) in which R^{1A} is n-butyl, R^{6A} to R^{9A} are hydrogen atoms, R^{2A} and R^{3A} or R^{3A} and R^{4A} of R^{2A} to R^{5A} together with carbon atoms on the phenanthridine ring to which they are bound may form aryl that is fused to the phenanthridine ring, and the other remaining groups are hydrogen atoms or a salt thereof is preferably prepared by the method comprising the steps described below:
steps (3A) of allowing a bromobenzene represented by formula (II'): 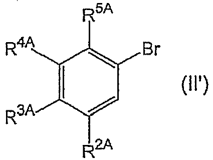 wherein
   R^{2A} to R^{5A} are as defined above,
   to react with a primary amine represented by formula (V"):

   R^{1A}-NH₂ (V")

   wherein
   R^{1A} is as defined above,
   to generate a N-substituted aniline represented by formula (IV"): 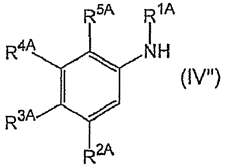 wherein
   R^{1A} to R^{5A} are as defined above;
step (3B) of allowing the N-substituted aniline represented by formula (IV") to be bound to a benzoyl halide represented by formula (III): 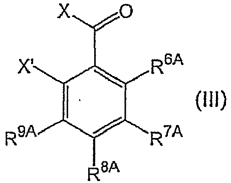 wherein
   R^{6A} to R^{9A} are as defined above; and
   X and X' are each independently selected from among halogen atoms,
   to generate a N-substituted benzanilide represented by formula (VI): 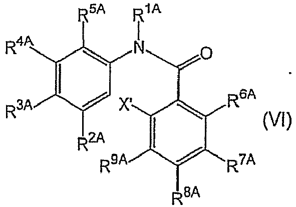 wherein
   R^{1A} to R^{9A} and X' are as defined above; and
step (3C) of subjecting the N-substituted benzanilide represented by formula (VI) to an intramolecular cyclization reaction in the presence of palladium acetate to generate the compound represented by formula (IA).

When a protected derivative of the compound represented by formula (IA) is generated via step (3C), the method may further comprise a step of deprotection described above and step (3D) below:
step (3D) of deprotecting the protected derivative of the compound represented by formula (IA) to generate the compound represented by formula (IA) or a salt thereof.

According to scheme 4, the compound represented by formula (IA) in which R^{1A} is n-butyl and R^{3A} is 2'-hydroxy-1',1',1',3',3',3'-hexafluoropropyl or 2'-benzyloxy-1',1',1',3',3',3'-hexafluoropropyl or a salt thereof is preferably prepared by the method comprising the steps described below:
step (4B) of allowing a N-substituted aniline represented by formula (IV"'): 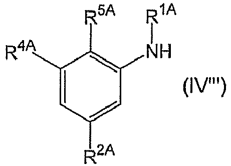 wherein
   R^{1A}, R^{2A}, R^{4A}, and R^{5A} are as defined above,
   to react with hexafluoroacetone sesquihydrate, hexafluoroacetone trihydrate, and p-toluenesulfonate monohydrate to generate a N-substituted aniline represented by formula (IV"): 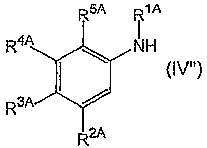 wherein
   R^{1A} to R^{5A} are as defined above;
step (4C) of allowing the N-substituted aniline represented by formula (IV") to be bound to a halobenzoic acid represented by formula (III'): 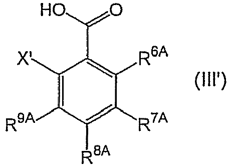 wherein
   R^{6A} to R^{9A} are as defined above; and
   X' is a halogen atom,
   to generate a N-substituted benzanilide represented by formula (VI): 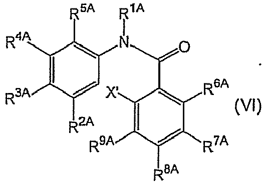 wherein
   R^{1A} to R^{9A} and X' are as defined above; and
step (4D) of subjecting the N-substituted benzanilide represented by formula (VI) to an intramolecular cyclization reaction in the presence of palladium acetate to generate the compound represented by formula (IA).

When a protected derivative of the compound represented by formula (IA) is generated via step (4D), the method may further comprise a step of deprotection described above and step (4E) below:
step (4E) of deprotecting the protected derivative of the compound represented by formula (IA) to generate the compound represented by formula (IA) or a salt thereof.

According to scheme 5, further, the compound represented by formula (IA) in which R^{1A} is n-butyl and R^{3A} is 2'-hydroxy-1',1',1',3',3',3'-hexafluoropropyl or 2'-benzyloxy-1',1',1',3',3',3'-hexafluoropropyl or a salt thereof is preferably prepared by the method comprising the steps described below:
step (5B) of allowing a N-substituted aniline represented by formula (IV"'): 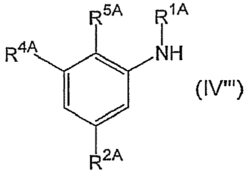 wherein
   R^{1A}, R^{2A}, R^{4A}, and R^{5A} are as defined above,
   to react with hexafluoroacetone sesquihydrate, hexafluoroacetone trihydrate, and p-toluenesulfonate monohydrate to generate a N-substituted aniline represented by formula (IV"): 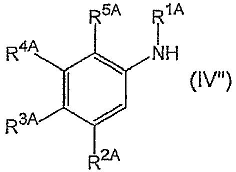 wherein
   R^{1A} to R^{5A} are as defined above;
step (5C) of allowing the N-substituted aniline represented by fonnula (IV") to be bound to a benzoyl halide represented by formula (III): 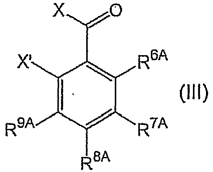 wherein
   R^{6A} to R^{9A} are as defined above; and
   X and X' are each independently selected from among halogen atoms,
   to generate a N-substituted benzanilide represented by formula (VI): 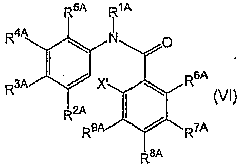 wherein
   R^{1A} to R^{9A} and X' are as defined above; and
step (5D) of subjecting the N-substituted benzanilide represented by formula (VI) to an intramolecular cyclization reaction in the presence of palladium acetate to generate the compound represented by formula (IA).

When a protected derivative of the compound represented by formula (IA) is generated via step (5D), the method may further comprise a step of deprotection described above and step (5E) below:
step (5E) of deprotecting the protected derivative of the compound represented by formula (IA) to generate the compound represented by formula (IA) or a salt thereof.

As described above, the compound represented by formula (I) or (IA) of the present invention has a high degree of anti-HCV activity. Accordingly, it can be used as an active ingredient of a therapeutic agent for hepatitis C. According to the production method of the present invention, also, a compound represented by formula (I) or (IA) can be produced with high yield and high purity.

### Examples

Hereafter; the present invention is described in detail with reference to Synthesis Examples A and B, although the technical scope of the present invention is not limited thereto.

### [Synthesis, isolation, and purification of compound]

### (Synthesis Example A-1)

### Synthesis, of 2-(1',1',1',3',3',3'-hexafluoro-2'-hydroxypropan-2'-yl)-5-benzylphenanthridin-6(5H)-one (AA-04)

Synthesis, isolation, and purification were carried out in accordance with the method described in a known literature (Aoyama et al, Heterocycles, vol. 76(1), pp. 137-142, 2008).

### (Synthesis Example A-2)

### Synthesis of 2-(1',1',1',3',3',3'-hexafluoro-2'-hydroxypropan-2'-yl)-5-butylphenanthridin-6(5H)-one (AA-24)

Synthesis, isolation, and purification were carried out in accordance with the method described in a known literature (Aoyama et al, Heterocycles, vol. 76(1), pp. 137-142, 2008).

### (Synthesis Example A-3)

### Synthesis of 2-(1',1',1',3',3',3'-hexafluoro-2'-hydroxypropan-2'-yl)-5-hexylphenanthridin-6(5H)-one (AA-25)

Synthesis, isolation, and purification were carried out in accordance with the method described in a known literature (Aoyama et al, Heterocycles, vol. 76(1), pp. 137-142, 2008).

### (Synthesis Example A-4)

### Synthesis of 2-(1',1',1',3',3',3'-hexafluoro-2'-hydroxypropan-2'-yl)-5-cyclohexylmethyl-phenanthridin-6(5H)-one (AA-26)

Synthesis, isolation, and purification were carried out in accordance with the method described in a known literature (Aoyama et al, Heterocycles, vol. 76(1), pp. 137-142, 2008).

### (Synthesis Example B-1)

### Synthesis of 5-butylphenanthridin-6(5H)-one (PN-H)

A process of synthesis is as shown in scheme 1.

### (1) Synthesis of 2-iodobenzanilide (a)

Anhydrous dichloromethane (2 ml), triethylamine (105 µl, 753 µmol), and 2-iodobenzoyl chloride (134 mg, 502 µmol) were added to aniline (55.9 mg, 600 µmol), and the mixture was agitated at room temperature for 14 hours. Water was added to the reaction solution, and an organic phase was extracted with ethyl acetate. The organic phase was washed with water, and aqueous solutions of saturated sodium bicarbonate and of saturated sodium chloride, and the resultant was dried over magnesium sulfate, followed by concentration under a reduced pressure. A crude product was purified via silica gel column chromatography using hexane:ethyl acetate (1:1) as an eluting solvent (yield: 160 mg, 99%).

### (2) Synthesis of N-butyl-2-iodobenzanilide (b)

Anhydrous N,N-dimethylformamide (1 ml), 60% sodium hydride (19.5 mg, 488 µmol), and butyl iodide (93 µl, 809 µmol) were added to 2-iodo-N-phenylbenzamide (a) (129 mg, 400 µmol), and the mixture was agitated at room temperature for 21 hours. Water was added to the reaction solution, and an organic phase was extracted with ethyl acetate. The organic phase was washed with water, and aqueous solutions of saturated sodium bicarbonate and of saturated sodium chloride, and the resultant was dried over magnesium sulfate, followed by concentration under a reduced pressure. A crude product was purified via silica gel column chromatography using hexane:ethyl acetate (6:1) as an eluting solvent (yield: 149 mg, 98%).

### (3) Synthesis of 5-butylphenanthlidin-6(5H)-one (PN-H)

Anhydrous N,N-dimethylacetamide (1.5 ml), potassium carbonate (82.9 mg, 600 µmol), palladium acetate (3.4 mg, 15.1 µmol), and tri(cyclohexyl)phosphine-tetrafluoroborate (11.3 mg, 30.7 µmol) were added to N-butyl-2-iodo-N-phenylbenzamide (b) (114 mg, 300 µmol), and the mixture was agitated at 130°C for 14 hours. A reaction solution was diluted with ethyl acetate and filtered through celite. The organic phase was washed with water and an aqueous solution of saturated sodium chloride, and the resultant was dried over sodium sulfate, followed by concentration under a reduced pressure. A crude product was purified via silica gel column chromatography using hexane:ethyl acetate (4:1) as an eluting solvent (yield: 75.5 mg, 100%).

FAB-MS m/z 252 (M+H)⁺; ¹H NMR (500 MHz, CDCl₃) δ 8.54 (dd, 1H, J = 7.9, 1.3 Hz), 8.29 (dd, 1H, J = 8.6, 1.3 Hz), 8.27 (d, 1H, J = 8.6 Hz), 7.74 (ddd, 1H, J = 8.0, 7.4, 1.2 Hz), 7.57 (t, 1H, J = 7.4, Hz), 7.53 (ddd, 1H, J = 8.5, 7.4,1.2 Hz), 7.40 (d, 1H, J = 8.5 Hz), 7.30 (dd, 1H, J = 8.0, 7.4 Hz), 4.38 (t, 2H, J = 7.9, Hz), 1.78 (td, 2H, J = 7.9, 7.3 Hz), 1.52 (sextet, 2H, J = 7.3 Hz), 1.00 (t, 3H, J = 7.3, Hz).

### (Synthesis Example B-2)

### Synthesis of 5-butyl-1-methylphenanthridin-6(5H)-one (MN290)

A target compound was obtained in the same manner as in Synthesis Example B-1, except for the use of 2-iodo-3-methylaniline instead of aniline and benzoyl chloride instead of 2-iodobenzoyl chloride.

FAB-MS m/z 266 (M+H)⁺; ¹H NMR (500 MHz, CDCl₃): δ 8.64 (d, 1H, J = 8.0 Hz), 8.44 (d, 1H, J = 8.0 Hz), 7.73 (t, 1H, J = 8.0 Hz), 7.59 (t, 1H, J = 8.0 Hz), 7.42 (t, 1H, J = 8.0 Hz), 7.33 (d, 1H, J = 8.0 Hz), 7.17 (d, 1H, J = 8.0 Hz), 4.40 (t, 2H, J = 7.6 Hz), 2.96 (s, 3H), 184-1.77 (m, 2H), 158-1.48 (m, 2H), 1.02 (t, 3H, J = 7.6 Hz).

### (Synthesis Example B-3)

### Synthesis of 5-butyl-2-methylphenanthridin-6(5H)-one (MN267)

A target compound was obtained in the same manner as in Synthesis Example B-1, except for the use of 4-methylaniline instead of aniline and 2-bromobenzoyl chloride instead of 2-iodobenzoyl chloride.

FAB-MS m/z 266 (M+H)⁺; ¹H NMR (500 MHz, CDCl₃): δ 8.55 (dd, 1H, J = 8.0, 1.2 Hz), 8.28 (d, 1H, J = 8.0 Hz), 8.10 (s, 1H), 7.74 (td, 1H, J = 8.0, 1.2 Hz), 7.57 (m, 1H), 7.37-7.30 (d, 1H, J = 7.6 Hz), 4.38 (t, 2H, J = 7.6 Hz), 2.49 (s, 3H), 1.82-1.75 (m, 3H), 1.56-1.48 (m, 2H), 1.01 (t, 3H, J = 7.3 Hz).

### (Synthesis Example B-4)

### Synthesis of 5-butyl-3-methylphenanthridin-6(5H)-one (MN289)

A target compound was obtained in the same manner as in Synthesis Example B-1, except for the use of 2-bromo-5-methylaniline instead of aniline and benzoyl chloride instead of 2-iodobenzoyl chloride.

FAB-MS m/z 266 (M+H)⁺; ¹H NMR (500 MHz, CDCl₃): δ 8.53 (d, 1H, J = 8.0 Hz), 8.24 (d, 1H, J = 8.0 Hz), 8.80 (d, 1H, J = 8.0 Hz), 7.73 (t, 1H, J = 8.0 Hz), 7.55 (t, 1H, J = 8.0 Hz), 7.20 (s, 1H), 7.13 (d, 1H, J = 8.0 Hz), 4.39 (t, 2H, J = 7.5 Hz), 2.52 (s, 3H), 1.83-1.76 (m, 2H), 1.57-1.49 (m, 2H), 1.03 (t, 3H, J = 7.5 Hz).

### (Synthesis Example B-5)

### Synthesis of 5-butyl-4-methylphenanthridin-6(5H)-one (MN265)

A target compound was obtained in the same manner as in Synthesis Example B-1, except for the use of 2-methylaniline instead of aniline and 2-bromobenzoyl chloride instead of 2-iodobenzoyl chloride.

FAB-MS m/z 266 (M+H)⁺; ¹H NMR (500 MHz, CDCl₃): δ 8.50 (dd, 1H, J = 8.0, 1.2 Hz), 8.22 (d, 1H, J = 8.0 Hz), 8.13 (d, 1H, J = 8.0 Hz), 7.20 (td, 1H, J = 7.6, 1.4 Hz), 7.55 (t, 1H, J = 8.0 Hz), 7.30 (d, 1H, J = 7.6 Hz), 7.21 (t, 1H, J = 7.6 Hz), 4.49 (t, 2H, J = 7.6 Hz), 2.66 (s, 3H), 1.66-1.60 (m, 2H), 1.20-1.19 (m, 2H), 0.86 (t, 3H, J = 7.3 Hz).

### (Synthesis Example B-6)

### Synthesis of 5-butyl-7-methylphenanthridin-6(5H)-one (MN277)

A target compound was obtained in the same manner as in Synthesis Example B-1, except for the use of 2-bromoaniline instead of aniline and 2-methylbenzoyl chloride instead of 2-iodobenzoyl chloride.

FAB-MS m/z 266 (M+H)⁺; ¹H NMR (500 MHz, CDCl₃): δ 8.27 (d, 1H, J = 7.5 Hz), 8.17 (d, 1H, J = 7.5 Hz), 7.58 (t, 1H, J = 7.5 Hz), 7.51 (t, 1H, J = 7.5 Hz), 7.35 (t, 1 H, J = 7.5 Hz), 7.26 (t, 1H, J = 7.5 Hz), 4.31 (t, 2H, J = 8.0 Hz), 2.98 (s, 3H), 1.81-1.74 (m, 2H), 1.58-1.49 (m, 2H), 1.02 (t, 3H, J = 7.3 Hz).

### (Synthesis Example B-7)

### Synthesis of 5-butyl-8-methylphenanthridin-6(5H)-one (MN296-B)

A target compound was obtained in the same manner as in Synthesis Example B-1, except for the use of 2-bromoaniline instead of aniline and 3-methylbenzoyl chloride instead of 2-iodobenzoyl chloride.

FAB-MS m/z 266 (M+H)⁺; ¹H NMR (500 MHz, CDCl₃): δ 8.36 (s, 1H), 8.28 (d, 1H, J = 8.0 Hz), 8.18 (d, 1H, J = 8.0 Hz), 7.58 (dd, 1H, J = 8.0, 1.8 Hz), 7.52 (t, 1H, J = 8.0 Hz), 7.41 (d, 1H, J = 8.0 Hz), 7.30 (t, 1H, J = 8.0 Hz), 4.40 (t, 2H, J = 8.0 Hz), 2.52 (s, 3H), 1.83-1.76 (m, 2H), 1.56-1.50 (m, 2H), 1.02 (t, 3H, J = 7.3 Hz).

### (Synthesis Example B-8)

### Synthesis of 5-butyl-9-methylphenanthridin-6(5H)-one (MN278)

A target compound was obtained in the same manner as in Synthesis Example B-1, except for the use of 2-bromoaniline instead of aniline and 4-methylbenzoyl chloride instead of 2-iodobenzoyl chloride.

FAB-MS m/z 266 (M+H)⁺; ¹H NMR (500 MHz, CDCl₃): δ 8.44 (d, 1H, J = 7.5 Hz), 8.30 (d, 1H, J = 7.5 Hz), 8.07 (s, 1H7.5), 7.53 (td, 1H, J = 7.5 Hz), 7.40 (d, 2H, J = 7.5 Hz), 7.30 (dd, 1H, J = 7.5 Hz), 4.39 (t, 2H, J = 8.0 Hz), 2.57 (s, 3H), 1.81.75 (m, 2H), 1.51.49 (m, 2H), 1.02 (t, 3H, J = 7.3 Hz).

### (Synthesis Example B-9)

### Synthesis of 5-butyl-10-methylphenanthridin-6(5H)-one (MN296-A)

A target compound was obtained in the same manner as in Synthesis Example B-1, except for the use of 2-bromoaniline instead of aniline and 3-methylbenzoyl chloride instead of 2-iodobenzoyl chloride.

FAB-MS m/z 266 (M+H)⁺; ¹H NMR (500 MHz, CDCl₃): δ 8.53 (d, 1H, J = 8.0 Hz), 8.70 (dd, 1H, J = 8.0, 1.2 Hz), 7.61 (d, 1H, J = 8.0 Hz), 7.56-7.52 (m, 1H), 7.49 (d, 1H, J = 8.0 Hz), 7.46 (dd, 1H, J = 8.0, 1.2 Hz), 7.32-7.27 (m, 1H), 4.40 (t, 2H, J = 7.3 Hz), 2.97 (s, 3H), 1.85-1.78 (m, 2H), 1.50-1.50 (m, 2H), 1.02 (t, 3H, J = 7.3 Hz).

### (Synthesis Example B-10)

### Synthesis of 5-butyl-2-ethylphenanthridin-6(5H)-one (MN324)

A target compound was obtained in the same manner as in Synthesis Example B-1, except for the use of 4-ethylaniline instead of aniline and 2-bromobenzoyl chloride instead of 2-iodobenzoyl chloride.

FAB-MS m/z 280 (M+H)⁺; ¹H NMR (500 MHz, CDCl₃): δ 8.55 (dd, 1H, J = 8.0, 1.5 Hz), 8.30 (d, 1H, J = 8.0 Hz), 8.12 (d, 1H, J = 1.5 Hz), 7.75 (td, 1H, J = 8.0, 1.5 Hz), 7.58 (t, 1H, J = 8.0 Hz), 7.39 (dd, 1H, J = 8.0, 1.5 Hz), 7.34 (d, 1H, J = 8.0 Hz), 4.39 (t, 2H, J = 8.0 Hz), 2.79 (q, 2H, J = 8.0 Hz), 1.83-1.76 (m, 2H), 1.57-1.49 (m, 2H), 1.34 (t, 3H, J = 7.6 Hz), 1.02 (t, 3H, J = 7.3 Hz).

### (Synthesis Example B-11)

### Synthesis of 5-butyl-2-hexylphenanthridin-6(5H)-one (MN338)

A target compound was obtained in the same manner as in Synthesis Example B-1, except for the use of 4-hexylaniline instead of aniline and 2-bromobenzoyl chloride instead of 2-iodobenzoyl chloride.

FAB-MS m/z 336 (M+H)⁺; ¹H NMR (500 MHz, CDCl₃): δ 8.55 (d, 1H, J = 8.0 Hz), 8.30 (d, 1H, J = 8.0 Hz), 8.09 (s, 1H), 7.75 (t, 1H, J = 8.0 Hz), 7.57 (t, 1H, J = 8.0 Hz), 7.37 (dd, 1H, J = 8.0, 1.5 Hz), 7.33 (dd, 1H, J = 8.0, 1.5 Hz), 4.39 (t, 2H, J = 7.6 Hz), 2.74 (t, 2H, J = 7.9 Hz), 1.83-1.76 (m, 2H), 1.73-1.66 (m, 2H), 1.58-1.49 (m, 2H), 1.41-1.32 (m, 6H), 1.02 (t, 3H, J = 7.3 Hz), 0.90 (t, 3H, J = 7.0 Hz).

### (Synthesis Example B-12)

### Synthesis of 5-butyl-2-dodecylphenanthridin-6(5H)-one (MN339)

A target compound was obtained in the same manner as in Synthesis Example B-1, except for the use of 4-dodecylaniline instead of aniline and 2-bromobenzoyl chloride instead of 2-iodobenzoyl chloride.

FAB-MS m/z 420 (M+H)⁺; ¹H NMR (500 MHz, CDCl₃): δ 8.55 (dd, 1H, J = 8.0, 1.5 Hz), 8.30 (d, 1H, J = 8.0 Hz), 8.09 (d, 1H, J = 1.5 Hz), 7.75 (ddd, 1H, J = 8.0, 6.5, 1.5 Hz), 7.57 (td, 1H, J = 8.0, 1.5 Hz), 7.36 (dd, 1H, J = 8.0, 1.5 Hz), 7.33 (d, 1H, J = 8.0 Hz), 4.38 (t, 2H, J = 7.6 Hz), 2.74 (t, 2H, J = 7.6 Hz), 1.83-1.76 (m, 2H), 1.73-1.66 (m, 2H), 1.57-1.49 (m, 2H), 1.45-1.20 (m, 18H), 1.02 (t, 3H, J = 7.3 Hz), 0.88 (t, 3H, J = 7.0 Hz).

### (Synthesis Example B-13)

### Synthesis of 5-butyl-1-isopropylphenanthridin-6(5H)-one (MN315-A)

A target compound was obtained in the same manner as in Synthesis Example B-1, except for the use of 3-isopropylaniline instead of aniline.

FAB-MS m/z 294 (M+H)⁺; ¹H NMR (500 MHz, CDCl₃): δ 8.58 (dd, 1H, J = 8.0, 1.5 Hz), 8.13 (d, 1H, J = 8.0 Hz), 7.69 (t, 1H, J = 8.0 Hz), 7.58 (t, 1H, J = 8.0 Hz), 7.48 (t, 1H, J = 8.0, 1.5 Hz), 7.35 (d, 1H, J = 8.0 Hz), 7.24 (d, 1H, J = 8.0 Hz), 4.36 (t, 2H, J = 8.0 Hz), 4.00 (sep, 1H, J = 6.7 Hz), 1.83-1.75 (m, 2H), 1.55-1.47 (m, 2H), 1.44 (d, 6H, J = 6.7 Hz), 1.01 (t, 3H, J = 7.3 Hz).

### (Synthesis Example B-14)

### Synthesis of 5-butyl-2-isopropylphenanthridin-6(5H)-one (PN-iPr)

A target compound was obtained in the same manner as in Synthesis Example B-1, except for the use of 4-isopropylanline instead of aniline.

FAB-MS m/z 294 (M+H)⁺; ¹H NMR (500 MHz, CDCl₃): δ 8.54 (dd, 1H, J = 8.0, 1.2 Hz), 8.30 (d, 1H, J = 8.0 Hz), 8.12 (d, 1H, J = 1.9 Hz), 7.74 (ddd, 1H, J = 7.9, 7.4, 1.2 Hz), 7.56 (dd, 1H, J = 8.0, 7.4 Hz), 7.41 (dd, 1H, J = 8.6, 1.9 Hz), 7.34 (d, 1H, J = 8.6 Hz), 4.37 (t, 2H, J = 8.0 Hz), 3.05 (septet, 1H, J = 7.4 Hz), 1.77 (quintet, 2H, J = 8.0 Hz), 1.51 (sextet, 2H, J = 7.4 Hz), 1.34 (d, 6H, J = 7.4 Hz), 1.00 (t, 3H, J = 7.4 Hz).

### (Synthesis Example B-15)

### Synthesis of 5-butyl-3-isopropylphenanthridin-6(5H)-one (MN315-B)

A target compound was obtained in the same manner as in Synthesis Example B-1, except for the use of 3-isopropylaniline instead of aniline.

FAB-MS m/z 294 (M+H)⁺; ¹H NMR (500 MHz, CDCl₃): δ 8.53 (dd, 1H, J = 8.0, 1.5 Hz), 8.40 (d, 1H, J = 8.0 Hz), 8.22 (d, 1H, J = 8.0 Hz), 7.73 (t, 1H, J = 8.0 Hz), 7.55 (td, 1H, J = 8.0, 1.5 Hz), 7.25 (d, 1H, J = 1.5 Hz), 7.20 (dd, 1H, J = 8.0, 1.5 Hz), 4.42 (t, 2H, J = 7.5 Hz), 3.06 (sep, 1H, J = 6.7 Hz), 1.85-1.77 (m, 2H), 1.57-1.51 (m, 2H), 1.35 (d, 6H, J = 7.3 Hz), 1.04 (t, 3H, J = 7.6 Hz).

### (Synthesis Example B-16)

### Synthesis of 5-butyl-2-tert-butylphenanthridin-6(5H)-one (MN325)

A target compound was obtained in the same manner as in Synthesis Example B-1, except for the use of 4-tert-butylaniline instead of aniline and 2-bromobenzoyl chloride instead of 2-iodobenzoyl chloride.

FAB-MS m/z 308 (M+H)⁺; ¹H NMR (500 MHz, CDCl₃): δ 8.56 (dd, 1H, J = 8.0, 1.5 Hz), 8.32 (d, 1H, J = 8.0 Hz), 8.31 (d, 1H, J = 1.5 Hz), 7.78-7.74 (m, 1H), 7.60 (dd, 1H, J = 8.0, 1.5 Hz), 7.57 (d, 1H, J = 8.0 Hz), 7.37 (d, 1H, J = 8.0 Hz), 4.39 (t, 2H, J = 7.6 Hz), 1.83-1.76 (m, 2H), 1.56-1.50 (m, 2H), 1.44 (s, 9H), 1.02 (t, 3H, J = 7.3 Hz).

### (Synthesis Example B-17)

### Synthesis of 5-butyl-2-fluorophenanthridin-6(5H)-one (MN329)

A target compound was obtained in the same manner as in Synthesis Example B-1, except for the use of 4-fluoroaniline instead of aniline and 2-bromobenzoyl chloride instead of 2-iodobenzoyl chloride.

FAB-MS m/z 270 (M+H)⁺; ¹H NMR (500 MHz, CDCl₃): δ 8.56 (dd, 1H, J = 8.0, 1.5 Hz), 8.17 (d, 1H, J = 8.0 Hz), 7.90 (dd, 1H, J = 9.7, 3.0 Hz), 7.77 (t, 1H, J = 8.0 Hz), 7.63 (t, 1H, J = 8.0 Hz), 7.37 (dd, 1H, J = 9.0, 4.5 Hz), 7.29-7.24 (m, 1H), 4.38 (t, 2H, J = 8.0 Hz), 1.82-1.75 (m, 2H), 1.57-1.48 (m, 2H), 1.02 (t, 3H, J = 7.3 Hz).

### (Synthesis Example B-18)

### Synthesis of 5-butyl-2-trifluoromethylphenanthridin-6(5H)-one (MN337)

A target compound was obtained in the same manner as in Synthesis Example B-1, except for the use of 4-trifluoromethylaniline instead of aniline and 2-bromobenzoyl chloride instead of 2-iodobenzoyl chloride.

FAB-MS m/z 320 (M+H)⁺; ¹H NMR (500 MHz, CDCl₃): δ 8.56 (d, 1H, J = 8.0 Hz), 8.53 (s, 1H), 8.30 (d, 1H, J = 8.0 Hz), 7.82 (t, 1H, J = 8.0 Hz), 7.77 (dd, 1H, J = 8.0, 1.8 Hz), 7.65 (t, 1H, J = 8.0 Hz), 7.00 (d, 1H, J = 8.0 Hz), 4.41 (t, 2H, J = 7.9 Hz), 1.83-1.75 (m, 2H), 1.58-1.49 (m, 2H), 1.03 (t, 3H, J = 7.3 Hz).

### (Synthesis Example B-19)

### Synthesis of 5-butyl-benzo[c][1,6]naphthyridine-6(5H)-one (PN-Py)

A target compound was obtained in the same manner as in Synthesis Example B-1, except for the use of 4-aminopyridine instead of aniline.

FAB-MS m/z 253 (M+H)⁺; ¹H NMR (500 MHz, CDCl₃): δ 9.48 (s, 1H), 8.61 (d, 1H, J = 5.5 Hz), 8.52 (d, 1H, J = 7.4 Hz), 8.34 (d, 1H, J = 7.9 Hz), 7.80 (t, 1H, J = 7.4 Hz), 7.63 (dd, 1H, J = 7.9, 7.4 Hz), 7.23 (d, 1H, J = 5.5 Hz), 4.32 (t, 2H, J = 8.0 Hz), 1.75 (quintet, 2H, J = 8.0 Hz), 1.50 (sextet, 2H, J = 7.3 Hz), 1.00 (t, 3H, J = 7.3 Hz).

### (Synthesis Example B-20)

### Synthesis of 6-butyl-8,9,10,11-tetrahydro-8,8,11,11-tetramethylbenzo[2,3-b]phenanthridin-5(6H)-one (MN256)

A target compound was obtained in the same manner as in Synthesis Example B-1, except for the use of 2-amino-5,6,7,8-tetrahydro-5,5,8,8-tetramethymaphthalene instead of aniline.

FAB-MS m/z 362 (M+H)⁺; ¹H NMR (500 MHz, CDCl₃): δ 8.52 (d, 1H, J = 8.0 Hz), 8.26 (d, 1H, J = 8.0 Hz), 8.21 (s, 1H), 7.73 (t, 1H, J = 8.0 Hz), 7.54 (t, 1H, J = 8.0 Hz), 7.31 (s, 1H), 4.39 (t, 2H, J = 7.3 Hz), 1.84-1.75 (m, 6H), 1.59-1.50 (m, 2H), 1.40 (s, 6H), 1.38 (s, 6H), 1.04 (t, 3H, J = 7.3 Hz).

### (Synthesis Example B-21)

### Synthesis of 5-butyl-2-methoxycarbonylphenanthridin-6(5H)-one (MN318)

A target compound was obtained in the same manner as in Synthesis Example B-1, except for the use of methyl 4-aminobenzoate instead of aniline and 2-bromobenzoyl chloride instead of 2-iodobenzoyl chloride.

FAB-MS m/z 310 (M+H)⁺; ¹H NMR (500 MHz, CDCl₃): δ 8.98 (d, 1H, J = 1.8 Hz), 8.54 (dd, 1H, J = 8.0, 1.8 Hz), 8.37 (d, 1H, J = 8.0 Hz), 8.17 (dd, 1H, J = 8.0, 1.8 Hz), 7.80 (ddd, 1H, J = 8.0, 6.6, 1.8 Hz), 7.62 (t, 1H, J = 8.0 Hz), 7.43 (d, 1H, J = 8.0 Hz), 4.40 (t, 2H, J = 7.6 Hz), 3.99 (s, 3H), 1.82-1.75 (m, 2H), 1.58-149 (m, 2H), 1.03 (t, 3H, J = 7.6 Hz).

### (Synthesis Example B-22)

### Synthesis of 5-butyl-2-phenethylphenanthridin-6(5H)-one (MN343)

### (1) Synthesis of 5-butyl-2-hydroxymethylphenanthridin-6(5H)-one (MN319)

Anhydrous tetrahydrofuran (5 ml), lithium borohydride (86.8 mg, 5.62 mmol), and tert-butyldimethylchlorosilane (668 mg, 4.43 mmol) were added to 5-butyl-2-methoxycarbonylphenanthridin-6(5H)-one (MN318) (239 mg, 772 µmol) under ice cooling, and the mixture was agitated at room temperature for 14 hours. The resultant was diluted with ethyl acetate under ice cooling, water was added thereto, and an organic phase was extracted with ethyl acetate. The organic phase was washed with water and an aqueous solution of saturated sodium chloride, and the resultant was dried over magnesium sulfate, followed by concentration under a reduced pressure. A crude product was purified via silica gel column chromatography using hexane:ethyl acetate (2:1) as an eluting solvent (yield: 193 mg, 89%).

FAB-MS m/z 282 (M+H)⁺; ¹H NMR (500 MHz, CDCl₃): δ 8.54 (d, 1H, J = 8.0 Hz), 8.28-8.26 (m, 2H), 7.75 (t, 1H, J = 8.0 Hz), 7.59 (t, 1H, J = 8.0, 1.8 Hz), 7.52 (dd, 1H, J = 8.0, 1.8 Hz), 7.36 (d, 1H, J = 8.0 Hz), 4.83 (d, 2H, J = 6.0 Hz), 4.37 (t, 2H, J = 7.5 Hz), 1.99-1.94 (m, 1H), 1.81-1.74 (m, 2H), 1.55-1.48 (m, 2H), 1.01 (t, 3H, J = 7.5 Hz).

### (2) Synthesis of 5-butyl-2-formylphenanthridin-6(5H)-one (MN330)

Anhydrous dichloromethane (20 ml) and manganese dioxide (1.0 g, 11.5 mmol) were added to 5-butyl-2-hydroxymethylphenanthridin-6(5H)-one (MN319) (100 mg, 355 µmol), and the mixture was agitated at room temperature for 14 hours. A reaction solution was filtered through celite, washed with dichloromethane, and then concentrated under a reduced pressure to obtain a target compound (yield: 96.5 mg, 97%).

FAB-MS m/z 280 (M+H)⁺; ¹H NMR (500 MHz, CDCl₃): δ 10.11 (s, 1H), 8.81 (d, 1H, J = 1.8 Hz), 8.55 (d, 1H, J = 8.0 Hz), 8.38 (d, 1H, J = 8.0 Hz), 8.04 (dd, 1H, J = 8.0, 1.8 Hz), 7.83 (t, 1H, J = 8.0 Hz), 7.65 (t, 1H, J = 8.0 Hz), 7.53 (d, 1H, J = 8.0 Hz), 4.42 (t, 2H, J = 8.0 Hz), 1.84-1.77 (m, 2H), 1.59-1.50 (m, 2H), 1.03 (t, 3H, J = 7.3 Hz).

### (3) Synthesis of (E)-5-butyl-2-styrylphenanthridin-6(5H)-one (MN340)

Anhydrous tetrahydrofuran (2 ml), potassium tert-butoxide (86.8 mg, 606 µmol), and 5-butyl-2-hydroxymethylphenanthridin-6(5H)-one (MN330) (56.0 mg, 200 µmol) were added to dimethyl benzylphosphonate (126 µl, 607 µmol) under ice cooling, and the mixture was agitated at room temperature for 62 hours. Water was added to the reaction solution, and an organic phase was extracted with ethyl acetate. The organic phase was washed with water and an aqueous solution of saturated sodium chloride, and the resultant was dried over magnesium sulfate, followed by concentration under a reduced pressure. A crude product was purified via silica gel column chromatography using hexane:ethyl acetate (4:1) as an eluting solvent (yield: 62.7 mg, 89%).

FAB-MS m/z 354 (M+H)⁺; ¹H NMR (500 MHz, CDCl₃): δ 8.56 (dd, 1H, J = 8.0, 1.3 Hz), 8.3 (d, 1H, J = 1.3 Hz), 8.35 (d, 1H, J = 8.0 Hz), 7.79 (t, 1H, J = 8.0 Hz), 7.73 (dd, 1H, J = 8.0, 1.3 Hz), 7.61 (t, 1H, J = 8.0 Hz), 7.57 (d, 2H, J = 8.0 Hz), 7.41 (d, 1H, J = 1.3 Hz), 7.40 (d, 2H, J = 8.0 Hz), 7.29 (t, 1H, J = 8.0 Hz), 7.19 (t, 2H, J = 8.0 Hz), 4.41 (t, 2H, J = 7.6 Hz), 1.84-1.77 (m, 2H), 1.57-1.50 (m, 2H), 1.03 (t, 3H, J = 7.3 Hz).

### (4) Synthesis of 5-butyl-2-phenethylphenanthridin-6(5H)-one (MN343)

Methanol, dichloromethane (2 ml), and 10% palladium-activated carbon (15 mg) were added to (E)-5-butyl-2-styrylphenanthridin-6(5H)-one (MN340) (35.3 mg, 99.9 µmol), and, in a hydrogen atmosphere, the mixture was agitated at room temperature for 1 hour. A reaction solution was filtered through celite, washed with dichloromethane, and then concentrated under a reduced pressure. A crude product was purified via silica gel column chromatography using hexane:ethyl acetate (8:1) as an eluting solvent (yield: 26.9 mg, 76%).

FAB-MS m/z 356 (M+H)⁺; ¹H NMR (500 MHz, CDCl₃): δ 8.55 (dd, 1H, J = 8.0, 1.2 Hz), 8.22 (d, 1H, J = 8.0 Hz), 8.05 (s, 1H), 7.74 (ddd, 1H, J = 8.0, 6.5, 1.2 Hz), 7.58 (t, 1H, J = 8.0 Hz), 7.37-7.29 (m, 4H), 7.24-7.21 (m, 3H), 4.38 (t, 2H, J = 7.9 Hz), 3.09-2.99 (m, 4H), 1.82-1.76 (m, 2H), 1.55-1.49 (m, 2H), 1.02 (t, 3H, J = 7.3 Hz).

### (Synthesis Example B-23)

### Synthesis of 5-butyl-2-hydroxyphenanthridin-6(5H)-one (PN-OH)

A process of synthesis is as shown in scheme 2.

### (1) Synthesis of 4-tert-butyldimethylsilyloxy-1-nitrobenzene (c)

Anhydrous N,N-dimethylformamide (8 ml), 1H-imidazole (383 mg, 5.62 mmol), and tert-butyldimethylchlorosilane (668 mg, 4.43 mmol) were added to 4-nitrophenol (557 mg, 4.00 mmol), and the mixture was agitated at room temperature for 14 hours. N,N-dimethylformamide was evaporated under a reduced pressure, water was added thereto, and an organic phase was extracted with ethyl acetate. The organic phase was washed with an aqueous solution of saturated sodium chloride, and the resultant was dried over sodium sulfate, followed by concentration under a reduced pressure. A crude product was purified via silica gel column chromatography using hexane:ethyl acetate (20:1) as an eluting solvent (yield: 801 mg, 79%).

### (2) Synthesis of 4-tert-butyldimethylsilyloxyaniline (d)

Ethyl acetate (6 ml) and 7.5% palladium-activated carbon (60 mg) were added to 4-tert-butyldimethylsilyloxy-1-nitrobenzene (c) (633 mg, 2.50 mmol), and, in a hydrogen atmosphere, the mixture was agitated at room temperature for 19 hours. Dichloromethane was added to the reaction solution, the resultant was filtered through celite, and the product was washed with ethyl acetate. Thereafter, a solvent was evaporated under a reduced pressure (yield: 160 mg, 99%).

### (3) Synthesis of 4'-tert-butyldimethylsilyloxybutyrylanilide (e)

Dichloromethane (1.5 ml), triethylamine (150 µl, 1.15 mmol), and butyryl chloride (80.0 µl, 766 µmol) were added to 4-tert-butyldimethylsilyloxyaniline (d) (168 mg, 754 mmol), and the mixture was agitated at room temperature for 15 hours. Water was added to the reaction solution, and an organic phase was extracted with dichloromethane. The organic phase was washed with water and an aqueous solution of saturated sodium chloride, and the resultant was dried over sodium sulfate, followed by concentration under a reduced pressure. A crude product was purified via silica gel column chromatography using hexane:ethyl acetate (4:1) as an eluting solvent (yield: 221 mg, 100%).

### (4) Synthesis of N-butyl-4-tert-butyldimethylsilyloxyaniline (f)

Tetrahydrofuran (6 ml) and 4'-tert-butyldimethylsilyloxybutyrylanilide (e) (205 mg, 700 mmol) were added to aluminum lithium hydride (159 mg, 4.20 mmol) under ice cooling, and the mixture was agitated at room temperature for 20 hours. Under ice cooling, sodium sulfate decahydrate and ethyl acetate were added to the reaction solution, the mixture was dried over sodium sulfate, and the resultant was concentrated under a reduced pressure. A crude product was purified via silica gel column chromatography using hexane:ethyl acetate (9:1) as an eluting solvent (yield: 88.4 mg, 45%).

### (5) Synthesis of N-butyl-4'-tert-butyldimethylsilyloxy-2-iodobenzanilide (g)

Anhydrous dichloromethane (1.5 ml), triethylamine (55 µl, 395 µmol), and 2-iodobenzoyl chloride (89.0 mg, 334 µmol) were added to N-butyl-4-tert-butyldimethylsilyloxybutyrylaniline (f) (84.8 mg, 303 µmol), and the mixture was agitated at room temperature for 15 hours. Water was added to the reaction solution, the mixture was subjected to extraction with dichloromethane, and the resultant was dried over magnesium sulfate, followed by concentration under a reduced pressure. A crude product was purified via silica gel column chromatography using hexane:ethyl acetate (9:1) as an eluting solvent (yield: 157 mg, 100%).

### (6) Synthesis of 5-butyl-2-hydroxyphenanthridin-6(5H)-one (PN-OH)

A target compound was obtained in the same manner as in Synthesis Example B-1-(3) except for the use of N-butyl-4'-tert-butyldimethylsilyloxy-2-iodobenzanilide (g) instead of N-butyl-2-iodo-N-phenylbenzamide (b) (91%).

FAB-MS m/z 268 (M+H)⁺; ¹H NMR (500 MHz, CDCl₃): δ 8.55 (d, 1H, J = 7.9 Hz), 8.10 (d, 1H, J = 7.9 Hz), 7.78 (d, 1H, J = 2.4 Hz), 7.67 (t, 1H, J = 7.3 Hz), 7.55 (t, 1H, J = 7.3 Hz), 7.28 (d, 1H, J = 9.2 Hz), 7.14 (dd, 1H, J = 9.2, 2.4 Hz), 6.68 (br-s, 1H), 4.37 (t, 2H, J = 7.3 Hz), 1.77 (quintet, 2H, J = 7.3 Hz), 1.49 (sextet, 2H, J = 7.3 Hz), 0.97 (t, 3H, J = 7.3 Hz).

### (Synthesis Example B-24)

### Synthesis of 5-butyl-2-(1'-hydroxyethyl)phenanthridin-6(5H)-one (PN-EtOH)

A target compound was obtained in the same manner as in Synthesis Example B-23-(3) except for the use of 1-(4-aminophenyl)ethanol instead of 4-tert-butyldimethylsilyloxyaniline (d).

FAB-MS m/z 296 (M+H)⁺; ¹H NMR (500 MHz, CDCl₃): δ 8.51 (dd, 1H, J = 7.9, 1.2 Hz), 8.23 (d, 1H, J = 8.5 Hz), 8.22 (d, 1H, J = 1.8 Hz), 7.72 (td, 1H, J = 7.3, 1.2 Hz), 7.56 (dd, 1H, J = 7.9, 7.3 Hz), 7.49 (dd, 1H, J = 8.5, 1.8 Hz), 7.29 (d, 1H, J = 8.5 Hz), 5.03 (q, 1H, J = 6.7 Hz), 4.33 (t, 2H, J = 7.9 Hz), 1.74 (quintet, 2H, J = 7.9 Hz), 1.57 (d, 3H, J = 6.7 Hz), 1.49 (sextet, 2H, J = 7.3 Hz), 0.99 (t, 3H, J = 7.3 Hz).

### (Synthesis Example B-25)

### Synthesis of 5-butyl-2-acetylphenanthridin-6(5H)-one (PN-Ac)

A target compound was obtained in the same manner as in Synthesis Example B-23-(3) except for the use of 1-(4-aminophenyl)ethanol instead of 4-tert-butyldimethylsilyloxyaniline (d).

FAB-MS m/z 294 (M+H)⁺; ¹H NMR (500 MHz, CDCl₃): δ 8.92 (d, 1H, J = 1.8 Hz), 8.53 (d, 1H, J = 7.3 Hz), 8.37 (d, 1H, J = 7.9 Hz), 8.10 (dd, 1H, J = 9.2, 1.8 Hz), 7.79 (t, 1H, J = 7.3 Hz), 7.61 (dd, 1H, J = 7.9, 7.3 Hz), 7.44 (d, 1H, J = 9.2 Hz), 4.39 (t, 2H, J = 7.9 Hz), 2.69 (s, 3H), 1.77 (quintet, 2H, J = 7.9 Hz), 1.52 (sextet, 2H, J = 7.3 Hz), 1.01 (t, 3H, J = 7.3 Hz).

### (Synthesis Example B-26)

### Synthesis of 6-butylbenzo[a]phenanthridin-5(6H)-one (MN313-A)

A process of synthesis is as shown in scheme 3.

### (1) Synthesis of 2-butylaminonaphthalene (h)

Anhydrous toluene (10 ml), tris(dibenzylideneacetone)dipalladium (98.9 mg, 109 µmol), 2,2'-bis(diphenylphosphino)-1,1'-binaphthyl (181 mg, 292 µmol), sodium tert-butoxide (1.00 g, 10.4 mmol), and butylamine (2.19 g, 29.9 mmol) were added to 2-bromonaphthalene (207 mg, 1.00 mmol), and the mixture was agitated at 85°C for 4 hours. The reaction solution was filtered through celite and washed with ethyl acetate. The organic phase was washed with water and an aqueous solution of saturated sodium chloride, and the resultant was dried over magnesium sulfate, followed by concentration under a reduced pressure. A crude product was purified via silica gel column chromatography using hexane:ethyl acetate (12:1) as an eluting solvent (yield: 202 mg, quant.).

### (2) Synthesis of 6-butylbenzo[a]phenanthridin-5(6H)-one (MN313-A)

A target compound was obtained in the same manner as in Synthesis Example B-23-(5), except for the use of 2-butylaminonaphthalene (h) instead of N-butyl-4-tert-butyldimethylsilyloxybutyrylaniline (f) and 2-bromobenzoyl chloride instead of 2-iodobenzoyl chloride.

FAB-MS m/z 302 (M+H)⁺; ¹H NMR (500 MHz, CDCl₃): δ 8.80 (d, 1H, J = 8.5 Hz), 8.68 (d, 1H, J = 8.5 Hz), 8.64 (dd, 1H, J = 8.5, 1.5 Hz), 7.95 (t, 2H, J = 8.5 Hz), 7.79 (td, 1H, J = 8.5, 1.5 Hz), 7.65-7.60 (m, 3H), 7.52 (t, 1H, J = 8.5 Hz), 4.50 (t, 2H, J = 7.5 Hz), 1.88-1.80 (m, 2H), 1.58-1.53 (m, 2H), 1.03 (t, 3H, J = 7.3 Hz).

### (Synthesis Example B-27)

### Synthesis of 6-butylbenzo[b]phenanthridin-5(6H)-one (MN313-B)

A target compound was obtained in the same manner as in Synthesis Example B-23-(5), except for the use of 2-butylaminonaphthalene (h) instead of N-butyl-4-tert-butyldimethylsilyloxybutyrylaniline (f) and 2-bromobenzoyl chloride instead of 2-iodobenzoyl chloride.

FAB-MS m/z 302 (M+H)⁺; ¹H NMR (500 MHz, CDCl₃): δ 8.90 (s, 1H), 8.57 (dd, 1H, J = 8.0, 1.8 Hz), 8.46 (d, 1H, J = 8.0 Hz), 7.98 (d, 1H, J = 8.0 Hz), 7.92 (d, 1H, J = 8.0 Hz), 7.82-7.78 (m, 1H), 7.73 (s, 1H), 7.62 (t, 1H, J = 7.3 Hz), 7.55 (t, 1H, J = 7.3 Hz), 7.48 (t, 1H, J = 7.3 Hz), 4.50 (t, 3H, J = 7.9 Hz), 1.92-1.85 (m, 2H), 1.63-1.52 (m, 2H), 1.06 (t, 3H, J = 7.3 Hz).

### (Synthesis Example B-28)

### Synthesis of 5-butylbenzo[c]phenanthridin-6(5H)-one (MN305)

A target compound was obtained in the same manner as in Synthesis Example B-1, except for the use of 1-aminonaphthalene instead of aniline and 2-bromobenzoyl chloride instead of 2-iodobenzoyl chloride.

FAB-MS m/z 302 (M+H)⁺; ¹H NMR (500 MHz, CDCl₃): δ 8.55 (dd, 1H, J = 8.0, 1.2 Hz), 8.29-8.22 (m, 3H), 7.92-7.89 (m, 1H), 7.81-7.77 (m, 1H), 7.73 (d, 1H, J = 8.0 Hz), 7.60 (t, 3H, J = 8.0 Hz), 7.56-7.50 (m, 2H), 4.59 (t, 2H, J = 7.3 Hz), 1.93-1.86 (m, 2H), 1.26-1.18 (m, 2H), 0.83 (t, 3H, J = 7.3 Hz).

### (Synthesis Example B-29)

### Synthesis of 6-butylbenzo[i]phenanthridin-5(6H)-one (MN306)

A target compound was obtained in the same manner as in Synthesis Example B-1, except for the use of 2-bromoaniline instead of aniline and 1-naphthoyl chloride instead of 2-iodobenzoyl chloride.

FAB-MS m/z 302 (M+H)⁺; ¹H NMR (500 MHz, CDCl₃): δ 10.30 (d, 1H, J = 9.0 Hz), 8.44 (dd, 1H, J = 8.0, 1.2 Hz), 8.39 (d, 1H, J = 9.0 Hz), 8n.17 (d, 1H, J = 9.0 Hz), 7.94 (dd, 1H, J = 8.0, 1.2 Hz), 7.77-7.73 (m, 1H), 7.65-7.60 (m, 2H), 7.49 (d, 1H, J = 8.0 Hz), 7.36 (t, 1H, J = 8.0 Hz), 4.50 (t, 2H, J = 7.6 Hz), 1.90-1.83 (m, 2H), 1.63-1.53 (m, 2H), 1.06 (t, 3H, J = 7.3 Hz).

### (Synthesis Example B-30)

### Synthesis of 5-butylbenzo[j]phenanthridin-6(5H)-one (MN314-B)

A target compound was obtained in the same manner as in Synthesis Example B-1, except for the use of 2-bromoaniline instead of aniline and 2-naphthoyl chloride instead of 2-iodobenzoyl chloride.

FAB-MS m/z 302 (M+H)⁺; ¹H NMR (500 MHz, CDCl₃): δ 9.14 (s, 1H), 8.74 (s, 1H), 8.48 (dd, 1H, J = 8.0, 1.5 Hz), 8.09 (d, 1H, J = 8.0 Hz), 8.04 (d, 1H, J = 8.0 Hz), 7.63 (t, 1H, J = 8.0 Hz), 7.57 (d, 1H, J = 8.0 Hz), 7.54 (dd, 1H, J = 8.0, 1.5 Hz), 7.42 (d, 1H, J = 8.0 Hz), 7.35 (t, 1H, J = 8.0 Hz), 4.43 (t, 2H, J = 7.6 Hz), 1.87-1.80 (m, 2H), 1.58-1.52 (m, 2H).

### (Synthesis Example B-31)

### Synthesis of 5-butylbenzo[k]phenanthridin-6(5H)-one (MN314-A)

A target compound was obtained in the same manner as in Synthesis Example B-1, except for the use of 2-bromoaniline instead of aniline and 2-naphthoyl chloride instead of 2-iodobenzoyl chloride.

FAB-MS m/z 302 (M+H)⁺; ¹H NMR (500 MHz, CDCl₃): δ 8.88 (d, 1H, J = 7.3 Hz), 8.66 (d, 1H, J = 8.5 Hz), 8.51 (d, 1H, J = 8.5 Hz), 8.01 (dd, 2H, J = 7.3, 3.0 Hz), 7.95 (d, 1H, J = 8.5 Hz), 7.70-7.63 (m, 3H), 7.59 (t, 1H, J = 7.3 Hz), 7.53 (d, 2H, J = 8.5 Hz), 7.36 (t, 2H, J = 8.5 Hz), 4.45 (t, 2H, J = 7.9 Hz), 1.90-1.83 (m, 3H), 1.57-1.51 (m, 2H), 1.03 (t, 3H, J = 7.5 Hz).

### (Synthesis Example B-32)

### Synthesis of 6-cyclohexylmethyl-8,9,10,11-tetrahydro-8,8,11,11-tetramethylbenzo[2,3-b] phenanthridin-5(6H)-one (MN299)

A target compound was obtained in the same manner as in Synthesis Example B-1, except for the use of 2-amino-5,6,7,8-tetrahydro-5,5,8,8-tetramethylnaphthalene instead of aniline and cyclohexylmethyl bromide instead of butyl iodide.

FAB-MS m/z 402 (M+H)⁺; ¹H NMR (500 MHz, CDCl₃): δ 8.52 (dd, 2H, J = 8.0, 1.2 Hz), 8.27 (d, 1H, J = 8.0 Hz), 8.21 (s, 1H), 7.73 (td, 1H, J = 8.0, 1.2 Hz), 7.54 (td, 1H, J = 8.0, 1.2 Hz), 7.29 (s, 1H), 4.30 (br s, 2H), 1.94-1.89 (m, 1H), 1.78 (s, 4H), 1.76 (t, 4H, J = 13.00 Hz), 1.41 (s, 6H), 1.38 (s, 6H), 1.29-1.15 (m, 6H).

### (Synthesis Example B-33)

### Synthesis of 6-benzyl-8,9,10,11-tetrahydro-8,8,11,11-tetramethylbenzo[2,3-b]phenanthridin-5(6H)-one (MN300)

A target compound was obtained in the same manner as in Synthesis Example B-1, except for the use of 2-amino-5,6,7,8-tetrahydro-5,5,8,8-tetramethylnaphthalene instead of aniline and benzyl bromide instead of butyl iodide.

FAB-MS m/z 396 (M+H)⁺; ¹H NMR (500 MHz, CDCl₃): δ 8.60 (dd, 2H, J = 8.0, 1.5 Hz), 8.29 (d, 1H, J = 8.0 Hz), 8.18 (s, 1H), 7.79-7.75 (m, 1H), 7.58 (t, 1H, J = 8.0 Hz), 7.37-7.28 (m, 4H), 7.23 (s, 1H), 7.22 (t, 1H, J = 8.0 Hz), 5.65 (s, 2H), 1.69 (d, 4H, J = 1.2 Hz), 1.36 (s, 6H), 1.14 (s, 6H).

### (Synthesis Example B-34)

### Synthesis of 4-butyl-11-(1',1',1',3',3',3'-hexafluoro-2'-hydroxypropan-2'-yl)-7-methoxy-[1,3] dioxolor[4,5-c]phenanthridin-5(4H)-one (HA-719)

The process of synthesis is as shown in scheme 4B below.

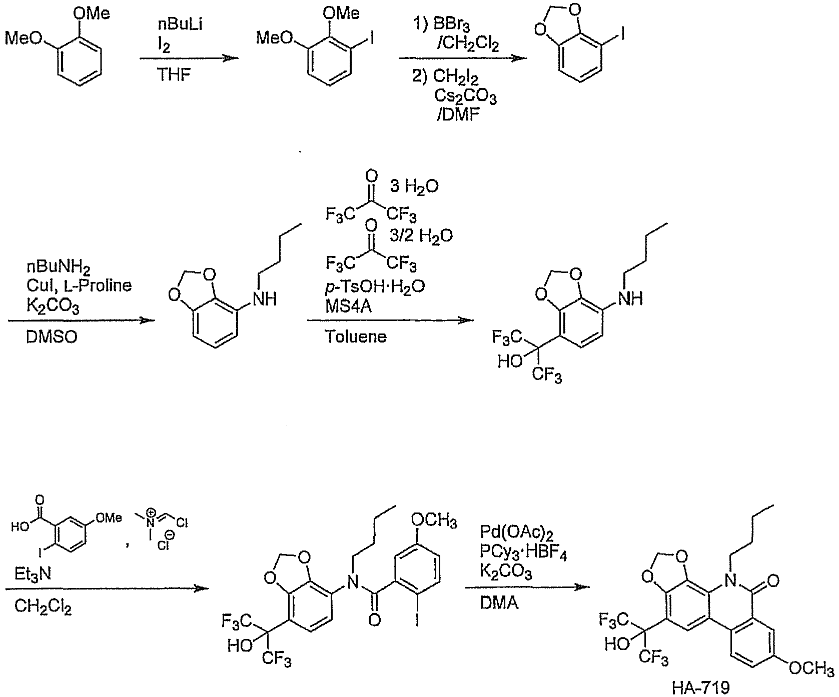

### (1) Synthesis of 3-iodo-1,2-dimethoxybenzene (i)

Anhydrous tetrahydrofuran (10 ml) and n-butyllithium (1.65 M hexane solution, 8.70 ml, 14.4 mmol) were added to 1,2-dimethoxybenzene (1.80 g, 13.0 mmol) at -10°C, and the mixture was agitated at room temperature for 2 hours. The reaction solution was cooled to -45°C, a solution of iodine (3.63 g, 14.3 mmol) dissolved in 10 ml of anhydrous tetrahydrofuran was added thereto, and the mixture was agitated at room temperature for 1.5 hours. An aqueous solution of saturated ammonium chloride was added to the reaction solution, and an organic phase was extracted with ethyl acetate. The organic phase was washed with an aqueous solution of 10% sodium thiosulfate and an aqueous solution of saturated sodium chloride, and the resultant was dried over sodium sulfate, followed by concentration under a reduced pressure. A crude product was purified via silica gel column chromatography using hexane:ethyl acetate (29:1) as an eluting solvent (yield: 2.44 g, 71%).

### (2) Synthesis of 3-iodobenzo[d][1,3]dioxole (j)

Anhydrous dichloromethane (10 ml) and boron tribromide (1.0 M dichloromethane solution, 14.0 ml, 14.0 mmol) were added to 3-iodo-1,2-dimethoxybenzene (i) (792 mg, 3.00 mmol) at -78°C, and the mixture was agitated at room temperature for 18 hours. The reaction solution was poured into iced water, dichloromethane was evaporated under a reduced pressure, and an organic phase was extracted with ethyl acetate. The organic phase was washed with an aqueous solution of saturated sodium chloride, and the resultant was dried over sodium sulfate, followed by concentration under a reduced pressure. Anhydrous N,N-dimethylformamide (30 ml), cesium carbonate (1.00 g, 3.07 mmol), and diiodomethane (0.250 ml, 3.10 mmol) were added to the residue, and the mixture was agitated at 120°C for 1 hour. N,N-dimethylformamide was evaporated under a reduced pressure, water was added thereto, and an organic phase was extracted with ethyl acetate. The organic phase was washed with water and an aqueous solution of saturated sodium chloride, and the resultant was dried over sodium sulfate, followed by concentration under a reduced pressure. A crude product was purified via silica gel column chromatography using hexane:ethyl acetate (50:1) as an eluting solvent (yield: 563 mg, 76%).

### (3) Synthesis of 3-(butylamino)benzo[d][1,3]dioxole (k)

Anhydrous dimethyl sulfoxide (2.0 ml), copper iodide (85.7 mg, 0.450 mmol), L-proline (104 mg, 0.900 mmol), potassium carbonate (415 mg, 3.00 mmol), and butylamine (0.60 ml, 6.07 mmol) were added to 3-iodobenzo[d][1,3]dioxole (j) (372 mg, 1.50 mmol), and the mixture was agitated at 90°C for 11 hours. Ethyl acetate was added to the reaction solution. The resultant was washed with water and an aqueous solution of saturated sodium chloride, dried over sodium sulfate, followed by concentration under a reduced pressure. A crude product was purified via silica gel column chromatography using hexane:ethyl acetate (50:1) as an eluting solvent (yield: 233 mg, 81%).

### (4) Synthesis of 2-[(4-butylamino)benzo[d][1,3]dioxole)]-1,1,1,3,3,3-hexafluoropropan-2-ol (1)

Anhydrous toluene (2.0 ml), hexafluoroacetone sesquihydrate (386 mg, 2.00 mmol), hexafluoroacetone trihydrate (440 mg, 2.00 mmol), p-toluenesulfonate monohydrate (19.0 mg, 0.100 mmol), and Molecular Sieve 4A (700 mg) were added to 3-(butylamino)benzo[d][1,3]dioxole (k) (193 mg, 1.00 mmol), and the mixture was agitated at 120°C for 9 hours. Ethyl acetate was added to the reaction solution, and Molecular Sieve 4A was separated by filtration. The organic phase was washed with water and an aqueous solution of saturated sodium chloride, and the resultant was dried over sodium sulfate, followed by concentration under a reduced pressure. A crude product was purified via silica gel column chromatography using hexane:ethyl acetate (19:1) as an eluting solvent (yield: 292 mg, 81%).

### (5) Synthesis of N-butyl-N-[4-(1,1,1,3,3,3-hexafluoro-2-hydroxypropan-2-yl)benzo[d] [1,3]dioxole]-2-iodo-5-methoxybenzamide (m)

Anhydrous dichloromethane (1.0 ml) and chloromethylenedimethyliminium chloride (76.8 mg, 0.600 mmol) were added to 2-iodo-5-methoxybenzoic acid (167 mg, 0.600 mmol) under ice cooling, and the resultant was agitated at room temperature for 30 minutes. A solution of 2-[(4-butylamino)benzo[d][1,3]dioxole]]-1,1,1,3,3,3-hexafluoropropan-2-ol (1) (72.0 mg, 0.200 mmol) dissolved in triethylamine (0.120 ml, 0.861 mmol) and dichloromethane (0.5 ml) was added to the reaction solution, and the mixture was agitated at room temperature for 20 hours. Water was added to the reaction solution, and an organic phase was extracted with ethyl acetate. The organic phase was washed with an aqueous solution of saturated sodium chloride, and the resultant was dried over sodium sulfate, followed by concentration under a reduced pressure. A crude product was purified via silica gel column chromatography using hexane:ethyl acetate (4:1) as an eluting solvent (yield: 123 mg, 99%).

### (6) Synthesis of 4-butyl-11-(1',1',1',3',3',3'-hexafluoro-2'-hydroxypropan-2'-yl)-7-methoxy-[1,3]dioxolo[4,5-c]phenanthridin-5(4H)-one (HA-719)

A target compound was obtained in the same manner as in Synthesis Example B-1-(3), except for the use of N-butyl-N-[4-(1,1,1,3,3,3-hexafluoro-2-hydroxypropan-2-yl)benzo[d][1,3]dioxole]-2-iodo-5-methoxybenzamide (m) instead of N-butyl-2-iodo-N-phenylbenzamide (b) (51 %).

HRMS (FAB) calculated: C₂₂H₂₀F₆NO₅ 492.1246; measured: 492.1257 (M+H)⁺; ¹H NMR (DMSO-d₆, 500 MHz) δ 8.94 (s, 1H), 8.20 (d, J = 9.2 Hz, 1H), 8.16 (s, 1H), 7.74 (d, J = 3.1 Hz, 1H), 7.46 (dd, J = 9.2, 3.1 Hz, 1H), 6.16 (s, 2H), 4.44 (t, J = 7.9 Hz, 2H), 3.89 (s, 3H), 1.68 (tt, J = 7.3, 7.9 Hz, 2H), 1.36 (qt, J = 7.3, 7.9 Hz, 2H), 0.92 (t, J = 7.9 Hz, 3H).

### (Synthesis Example B-35)

### Synthesis of 5-butyl-2-(1',1',1',3',3',3'-hexafluoro-2'-hydroxypropan-2'-yl)-4,8-dimethoxy-phenanthridin-6(5H)-one (KZ15)

A target compound was obtained in the same manner as in Synthesis Example B-34, except for the use of N-butyl-2-methoxyaniline instead of 3-(butylamino)benzo[d] [1,3]dioxole (k) and 2-bromo-5-methoxybenzoic acid instead of 2-iodo-5-methoxybenzoic acid.

¹H NMR (500 MHz, CDCl₃): δ 8.18 (s, 1H), 8.14 (d, 1H, J = 9.1 Hz), 7.92 (d, 1H, J = 3.1 Hz), 7.38 (dd, 1H, J = 8.5, 3.1 Hz), 7.23 (brs, 1H), 6.98 (s, 1H), 4.39 (t, 2H, J = 7.3 Hz), 4.11 (s, 3H), 3.96 (s, 3H), 1.82 (quin, 2H, J = 7.3 Hz), 1.55 (sext, 2H, J = 7.3 Hz), 1.05 (t, 3H, J = 7.3 Hz).

### (Synthesis Example B-36)

### Synthesis of 5-butyl-2-(1',1',1',3',3',3'-hexafluoro-2'-hydroxypropan-2'-yl)-3,8-dimethoxy-phenanthridin-6(5H)-one (KZ16)

A target compound was obtained in the same manner as in Synthesis Example B-34, except for the use of N-butyl-3-methoxyaniline instead of 3-(butylamino)benzo[d] [1,3]dioxole (k) and 2-bromo-5-methoxybenzoic acid instead of 2-iodo-5-methoxybenzoic acid.

^{I}H NMR (500 MHz, CDCl₃): δ 8.38 (s, 1H), 8.04 (d, 1H, J = 8.5 Hz), 7.93 (d, 1H, J = 3.1 Hz), 7.33 (dd, 1H, J = 8.5, 3.1 Hz), 7.32 (s, 1H), 4.56 (t, 2H, J = 7.3 Hz), 4.34 (s, 1H), 3.97 (s, 3H), 3.95 (s, 3H), 1.85 (quin, 2H, J = 7.3 Hz), 1.45 (sext, 2H, J = 7.3 Hz), 0.99 (t, 3H, J = 7.3 Hz).

### (Synthesis Example B-37)

### Synthesis of 5-butyl-2-(1',1',1',3',3',3'-hexafluoro-2'-hydroxypropan-2'-yl)-8,9-dimethoxy-phenanthridin-6(5H)-one (KZ25)

A target compound was obtained in the same manner as in Synthesis Example B-34, except for the use of N-butylaniline instead of 3-(butylamino)benzo[d][1,3]dioxole (k) and 2-bromo-4,5-dimethoxybenzoic acid instead of 2-iodo-5-methoxybenzoic acid.

¹H NMR (500 MHz, CDCl₃): δ 8.49 (s, 1H), 7.91 (s, 1H), 7.79 (d, 1H, J = 9.1 Hz), 7.52 (s, 1H), 7.44 (d, 1H, J = 9.1 Hz), 4.41 (brs, 1H), 4.36 (t, 2H, J = 7.3 Hz), 4.07 (s, 3H), 4.03 (s, 3H), 1.77 (quin, 2H, J = 7.3 Hz), 1.51 (sext, 2H, J = 7.3 Hz), 1.01 (t, 3H, J = 7.3 Hz).

### (Synthesis Example B-38)

### Synthesis of 5-butyl-2-(1',1',1',3',3',3'-hexafluoro-2'-hydroxypropan-2'-yl)-[1,3]dioxolo[4,5-j]

### phenanthridin-6(5H)-one (KZ26)

A target compound was obtained in the same manner as in Synthesis Example B-34, except for the use of N-butylaniline instead of 3-(butylamino)benzo[d][1,3]dioxole (k) and 2-bromo-4,5-methylenedioxybenzoic acid instead of 2-iodo-5-methoxybenzoic acid.

¹H NMR (500 MHz, CDCl₃): δ 8.43 (s, 1H), 7.84 (s, 1H), 7.79 (d, 1H, J = 9.1 Hz), 7.55 (s, 1H), 7.41 (d, 1H, J = 9.1 Hz), 6.13 (s, 2H), 4.45 (s, 1H), 4.31 (t, 2H, J = 7.3 Hz), 1.75 (quin, 2H, J = 7.3 Hz), 1.50 (sext, 2H, J = 7.3 Hz), 1.01 (t, 3H, J = 7.3 Hz).

### (Synthesis Example B-39)

### Synthesis of 5-butyl-2-(1',1',1',3',3',3'-hexafluoro-2'-hydroxypropan-2'-yl)-7,8-dimethoxy-phenanthridin-6(5H)-one (KZ27)

A target compound was obtained in the same manner as in Synthesis Example B-34, except for the use of N-butylaniline instead of 3-(butylamino)benzo[d][1,3]dioxole (k) and 2-bromo-5,6-dimethoxybenzoic acid instead of 2-iodo-5-methoxybenzoic acid.

¹H NMR (500 MHz, CDCl₃): δ 8.43 (s, 1H), 7.84 (s, 1H), 7.79 (d, 1H, J = 9.1 Hz), 7.55 (s, 1H), 7.41 (d, 1H, J = 9.1 Hz), 6.13 (s, 2H), 4.45 (s, 1H), 4.31 (t, 2H, J = 7.3 Hz), 1.75 (quin, 2H, J = 7.3 Hz), 1.50 (sext, 2H, J = 7.3 Hz), 1.01 (t, 3H, J = 7.3 Hz).

### (Synthesis Example B-40)

### Synthesis of 5-butyl-2-(1',1',1',3',3',3'-hexafluoro-2'-hydroxypropan-2'-yl)-8,9,10-trimethoxy-phenanthridin-6(5H)-one (KZ28)

A target compound was obtained in the same manner as in Synthesis Example B-34, except for the use of N-butylaniline instead of 3-(butylamino)benzo[d][1,3]dioxole (k) and 2-bromo-3,4,5-trimethoxybenzoic acid instead of 2-iodo-5-methoxybenzoic acid.

¹H NMR (500 MHz, CDCl₃): δ 9.70 (s, 1H), 7.94(s, 1H), 7.84 (d, 1H, J = 9.1 Hz), 7.46 (d, 1H, J = 9.1 Hz), 4.39 (t, 2H, J = 7.3 Hz), 4.04 (s, 3H), 4.02 (s, 3H), 3.65 (s, 3H), 3.89 (brs, 1H), 1.79 (quin, 2H, J = 7.3 Hz), 1.53 (sext, 2H, J = 7.3 Hz), 1.03 (t, 3H, J = 7.3 Hz).

### (Synthesis Example B-41)

### Synthesis of 5-butyl-2-(1',1',1',3',3',3'-hexafluoro-2'-hydroxypropan-2'-yl)-3,4-dimethoxy-phenanthridin-6(5H)-one (HA-726)

The process of synthesis is as shown in scheme 5B below.

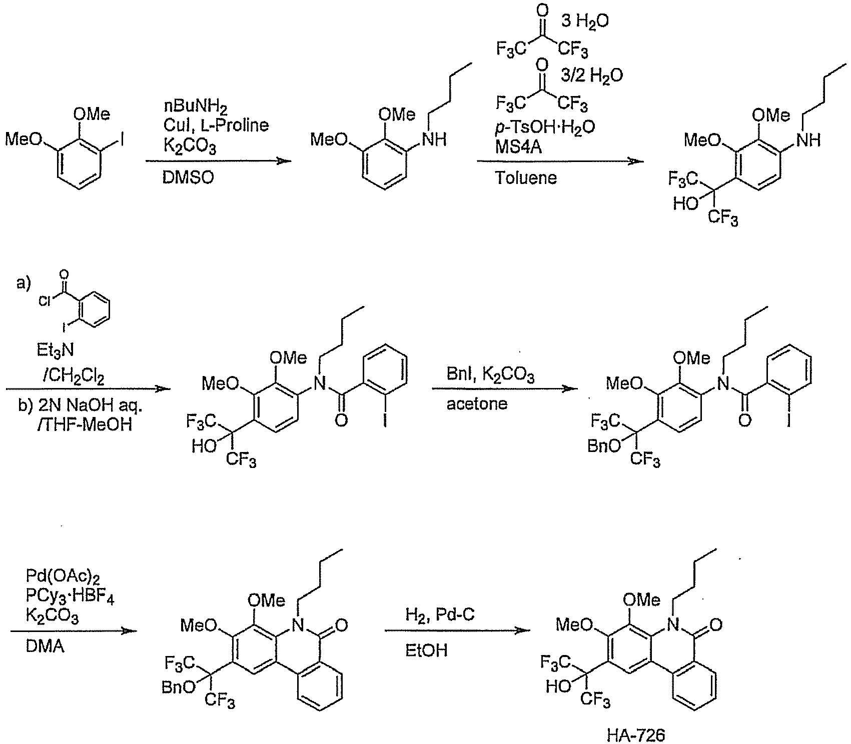

### (1) Synthesis of N-butyl-2,3-dimethoxyaniline (n)

A target compound was obtained in the same manner as in Synthesis Example B-34-(3), except for the use of 3-iodo-1,2-dimethoxybenzene (i) instead of 3-iodobenzo[d][1,3]dioxole (j).

### (2) Synthesis of 2-(4-butylamino-2,3-dimethoxyphenyl)-1,1,1,3,3,3-hexafluoropropan-2-ol (o)

A target compound was obtained in the same manner as in Synthesis Example B-34-(4), except for the use of N-butyl-2,3-dimethoxyaniline (n) instead of 3-(butylamino)benzo[d][1,3]dioxole (k).

### (3) Synthesis of N-butyl-N-[4-(1,1,1,3,3,3-hexafluoro-2-hydroxypropan-2-yl)-2,3-dimethoxyphenyl]-2-iodobenzamide (p)

Anhydrous dichloromethane (1.0 ml), triethylamine (35.0 µl, 0.251 mmol), and 2-iodobenzoyl chloride (52.1 mg, 0.196 mmol) were added to 2-(4-butylamino-2,3-dimethoxyphenyl)-1,1,1,3,3,3-hexafluoropropan-2-ol (o) (37.2 mg, 0.099 mmol), and the mixture was agitated at room temperature for 12 hours. Dichloromethane was evaporated under a reduced pressure, water was added thereto, and an organic phase was extracted with ethyl acetate. The organic phase was dried over sodium sulfate and then concentrated under a reduced pressure. Tetrahydrofuran (0.3 ml), methanol (0.3 ml), and an aqueous solution of 2 N sodium hydroxide were added to the residue, and the mixture was agitated at room temperature for 2 hours. 2 N hydrochloric acid was added to the reaction solution. The mixture was subjected to extraction with ethyl acetate, drying over sodium sulfate, and then concentration under a reduced pressure. A crude product was purified via silica gel column chromatography using hexane:dichloromethane (1:3) as an eluting solvent (yield: 58.7 mg, 98%).

### (4) Synthesis of N-butyl-N-[4-(2-benzyloxy-1,1,1,3,3,3-hexafluoropropan-2-yl)-2,3-dimethoxyphenyl]-2-iodobenzamide (q)

Acetone (0.8 ml), potassium carbonate (33.2 mg, 0.240 mmol), and benzyl iodide (87.2 mg, 0.400 mmol) were added to N-butyl-N-[4-(1,1,1,3,3,3-hexafluoro-2-hydroxypropan-2-yl)-2,3-dimethoxyphenyl]-2-iodobenzamide (p) (48.2 mg, 0.080 mmol), and the mixture was agitated at 60°C for 5 hours. Water was added to the reaction solution, and an organic phase was extracted with ethyl acetate. The organic phase was washed with an aqueous solution of saturated sodium chloride, and the resultant was dried over sodium sulfate, followed by concentration under a reduced pressure. A crude product was purified via silica gel column chromatography using hexane:ethyl acetate (6:1) as an eluting solvent (yield: 51.5 mg, 93%).

### (5) Synthesis of 5-butyl-2-(2'-benzyloxy-1',1',1',3',3',3'-hexafluoropropan-2'-yl)-3,4-dimethoxyphenanthridin-6(5H)-one (r)

A target compound was obtained in the same manner as in Synthesis Example B-1-(3), except for the use of N-butyl-N-[4-(2-benzyloxy-1,1,1,3,3,3-hexafluoro-propan-2-yl)-2,3-dimethoxyphenyl]-2-iodobenzamide (q) instead of N-butyl-2-iodo-N-phenylbenzamide (c) (77%).

### (6) Synthesis of 5-butyl-2-(1',1',1',3',3',3'-hexafluoro-2'-hydroxypropan-2'-yl)-3,4-dimethoxyphenanthridin-6(5H)-one (HA-726)

Ethanol (0.5 ml) and 7.5% palladium-activated carbon (4.0 mg) were added to 5-butyl-2-(2'-benzyloxy-1,1',1',,3',3',3'-hexafluoropropan-2'-yl)-3,4-dimethoxyphenanthridin-6(5H)-one (r) (29.0 mg, 0.051 mmol), and, in a hydrogen atmosphere, the mixture was agitated at room temperature for 6 hours. Dichloromethane was added to the reaction solution. The resultant was filtered through celite, washed with dichloromethane and ethyl acetate, and then concentrated under a reduced pressure. A crude product was purified via silica gel column chromatography using hexane:ethyl acetate (9:1) as an eluting solvent (yield: 24.2 mg, 99%).

HRMS (FAB) calculated: C₂₂H₂₂F₆NO₄ 478.1453; measured: 478.1445 (M+H)⁺; ¹H NMR (DMSO-d₆, 500 MHz) δ 8.76 (s, 1H), 8.50 (s, 1H), 8.33 (d, J = 8.6 Hz, 1H), 8.28 (d, J = 7.3 Hz, 1H), 7.87 (t, J = 8.6 Hz, 1H), 7.64 (t, J = 7.3 Hz, 2H), 4.49 (t, J = 7.3 Hz, 2H), 3.91 (s, 3H), 3.73 (s, 3H), 1.67 (tt, J = 7.3, 7.3 Hz, 2H), 1.28 (qt, J = 7.3, 7.3 Hz, 2H), 0.89 (t, J = 7.3 Hz, 3H).

### (Synthesis Example B-42)

### Synthesis of 5-butyl-2-(1',1',1',3',3',3'-hexafluoro-2'-methoxypropan-2'-yl)-1,4-dimethoxy-phenanthridin-6(5H)-one (KZ29)

A target compound was obtained in the same manner as in Synthesis Example B-41, except for the use of N-butyl-2,5-dimethoxyaniline instead of N-butyl-2,3-dimethoxyaniline (n) and iodomethane instead of benzyl iodide.

¹H NMR (500 MHz, CDCl₃): δ 8.88 (d, 1H, J = 8.6 Hz), 8.50 (d, 1H, J = 6.7 Hz), 7.72 (dd, 1H, J = 8.6, 6.7 Hz), 7.58 (dd, 1H, J = 8.6, 6.7 Hz), 7.17 (s, 1H), 4.54-4.30 (m, 2H), 3.93 (s, 3H), 3.59 (s, 3H), 3.51 (s, 3H), 1.95-1.80 (m, 2H), 1.45 (sext, 2H, J = 7.3 Hz), 1.00 (t, 3H, J = 7.3 Hz).

### (Synthesis Example B-43)

### Synthesis of 5-butyl-2-(1',1',1',3',3',3'-hexafluoro-2'-methoxypropan-2'-yl)-1,4,8-trimethoxyphenanthridin-6(5H)-one (KZ30)

A target compound was obtained in the same manner as in Synthesis Example B-42, except for the use of 2-iodo-5-methoxybenzoic acid and chloromethylenedimethyliminium chloride instead of 2-iodobenzoyl chloride and iodomethane instead of benzyl iodide.

¹H NMR (500 MHz, CDCl₃): δ 8.82 (d, 1H, J = 9.2 Hz), 7.93 (d, 1H, J = 3.1 Hz), 7.30 (dd, 1H, J = 9.2, 3.1 Hz), 7.12 (s, 1H), 4.54-4.30 (m, 2H), 3.96 (s, 3H), 3.92 (s, 3H), 3.57 (s, 3H), 3.50 (s, 3H), 1.95-1.80 (m, 2H), 1.46 (sext, 2H, J = 7.3 Hz), 1.01 (t, 3H, J = 7.3 Hz).

### (Synthesis Example B-44)

### Synthesis of 5-butyl-2-(1',1',1',3',3',3'-hexafluoro-2'-hydroxypropan-2'-yl)-7-methoxy-phenanthridin-6(5H)-one (KZ31)

A target compound was obtained in the same manner as in Synthesis Example B-34, except for the use of N-butylaniline instead of 3-(butylamino)benzo[d][1,3]dioxole (k) and 2-bromo-6-methoxybenzoic acid instead of 2-iodo-5-methoxybenzoic acid.

¹H NMR (500 MHz, CDCl₃): δ 8.64 (s, 1H), 8.37 (d, 1H, J = 9.1 Hz), 7.82 (d, 1H, J = 8.5 Hz), 7.48 (d, 1H, J = 2.4 Hz), 7.37 (d, 1H, J = 8.5 Hz), 7.10 (dd, 1H, J = 9.1, 2.4 Hz), 5.09 (brs, 1H), 4.26 (t, 2H, J = 7.3 Hz), 3.94 (s, 3H), 1.72 (quin, 2H, J = 7:3 Hz), 1.44 (sext, 2H, J = 7.3 Hz), 0.95 (t, 3H, J = 7.3 Hz).

### (Synthesis Example B-45)

### Synthesis of 5-butyl-2-(1',1',1',3',3',3'-hexafluoro-2'-hydroxypropan-2'-yl)-9-methoxy-phenanthridin-6(5H)-one (KZ32)

A target compound was obtained in the same manner as in Synthesis Example B-34, except for the use of N-butylaniline instead of 3-(butylamino)benzo[d][1,3]dioxole (k) and 2-bromo-4-methoxybenzoic acid instead of 2-iodo-5-methoxybenzoic acid.

¹H NMR (500 MHz, CDCl₃): δ 8.51 (s, 1H), 7.83 (d, 1H, J = 9.1 Hz), 7.81 (d, 1H, J = 7.9 Hz), 7.60 (dd, 1H, J = 7.9, 7.9 Hz), 7.33 (d, 1H, J = 9.1 Hz), 7.06 (d, 1H, J = 7.9 Hz), 5.22 (brs, 1H), 4.20 (t, 2H, J = 7.3 Hz), 4.04 (s, 3H), 1.68 (quin, 2H, J = 7.3 Hz), 1.48 (sext, 2H, J = 7.3 Hz), 0.99 (t, 3H, J = 7.3 Hz).

### (Synthesis Example B-46)

### Synthesis of 5-butyl-2-(1',1',1',3'3'3'-hexafluoro-2'-hydroxypropan-2'-yl)-10-methoxy-phenanthridin-6(5H)-one (KZ33)

A target compound was obtained in the same manner as in Synthesis Example B-34, except for the use of N-butylaniline instead of 3-(butylamino)benzo[d][1,3]dioxole (k) and 2-bromo-3-methoxybenzoic acid instead of 2-iodo-5-methoxybenzoic acid.

¹H NMR (500 MHz, CDCl₃): δ 9.75 (s, 1H), 8.16 (d, 1H, J = 7.9 Hz), 7.84 (d, 1H, J = 9.2 Hz), 7.50 (dd, 1H, J = 7.9, 7.9 Hz), 7.38 (d, 1H, J = 9.2 Hz), 7.22 (d, 1H, J = 7.9 Hz), 4.68 (brs, 1H), 4.30 (t, 2H, J = 7.3 Hz), 4.01 (s, 3H), 1.74 (quin, 2H, J = 7.3 Hz), 1.49 (sext, 2H, J = 7.3 Hz), 1.00 (t, 3H, J = 7.3 Hz).

### (Synthesis Example B-47)

### Synthesis of 5-butyl-2-(1',1',1',3',3',3'-hexafluoro-2'-hydroxypropan-2'-yl)-1-methoxy-phenanthridin-6(5H)-one (KZ34)

A target compound was obtained in the same manner as in Synthesis Example B-41, except for the use of N-butyl-3-methoxyaniline instead ofN-butyl-2,3-dimethoxyaniline (n).

¹H NMR (500 MHz, CDCl₃): δ 8.88 (d, 1H, J = 8.5 Hz), 8.59 (d, 1H, J = 6.7 Hz), 8.27 (brs, 1H), 7.79 (dd, 1H, J = 8.5, 6.7 Hz), 7.73 (d, 1H, J = 9.8 Hz), 7.65 (dd, 1H, J = 8.5, 6.7 Hz), 7.29 (d, 1H, J = 9.8 Hz), 4.48-4.40 (m, 1H), 4.35-4.4.22 (m, 1H), 3.86 (s, 3H), 1.90-1.73 (m, 2H), 1.60-0.97 (m, 2H), 1.03 (t, 3H, J = 7.3 Hz).

### (Synthesis Example B-48)

### Synthesis of 5-butyl-2-(1',1'1',3',3',3'-hexafluoro-2'-hydroxypropan-2'-yl)-1-hydroxy-phenanthridin-6(5H)-one (KZ35) and 5-butyl-2-(1',1',1',3',3',3'-hexafluoro-2'-hydroxypropan-2'-yl)-3-methoxyphenanthridin-6(5H)-one (KZ36)

Target compounds were obtained via separation using column chromatography in the same manner as in Synthesis Example B-34, except for the use of N-butyl-3-methoxyaniline instead of 3-(butylamino)benzo[d][1,3]dioxole (k) and 2-iodobenzoyl chloride instead of 2-iodo-5-methoxybenzoic acid.

KZ35: ¹H NMR (500 MHz, CDCl₃): δ 9.52 (d, 1H, J = 8.5 Hz), 8.54 (dd, 1H, J = 7.9, 1.2 Hz), 7.71 (ddd, 1H, J = 8.5, 8.5, 1.2 Hz), 7.53 (dd, 1H, J = 8.5, 7.9 Hz), 7.65 (d, 1H, J = 9.2 Hz), 6.91 (d, 1H, J = 9.2 Hz), 4.36 (t, 2H, J = 7.3 Hz), 3.50-3.20 (m, 2H), 1.80 (quin, 2H, J = 7.3 Hz), 1.52 (sext, 2H, J = 7.3 Hz), 1.03 (t, 3H, J = 7.3 Hz).

KZ36: ¹H NMR (500 MHz, CDCl₃): δ 9.52 (d, 1H, J = 8.5 Hz), 8.54 (dd, 1H, J = 7.9, 1.2 Hz), 7.71 (ddd, 1H, J = 8.5, 8.5, 1.2 Hz), 7.53 (dd, 1H, J = 8.5, 7.9 Hz), 7.65 (d, 1H, J = 9.2 Hz), 6.91 (d, 1H, J = 9.2 Hz), 4.36 (t, 2H, J = 7.3 Hz), 3.50-3.20 (m, 2H), 1.80 (quin, 2H, J = 7.3 Hz), 1.52 (sext, 2H, J = 7.3 Hz), 1.03 (t, 3H, J = 7.3 Hz).

### (Synthesis Example B-49)

### Synthesis of 2-(2'-benzyloxy-1',1',1',3',3',3'-hexafluoropropan-2'-yl)-5-butyl-3-methoxy-phenanthridin-6(5H)-one (KZ37)

A target compound was obtained in the same manner as in Synthesis Example B-41, except for the use of N-butyl-3-methoxyaniline instead of N-butyl-2,3-dimethoxyaniline (n).

¹H NMR (500 MHz, CDCl₃): δ 8.46 (d, 1H, J = 7.3 Hz), 8.38 (s, 1H), 7.55-7.37 (m, 8H), 6.92 (s, 1H), 4.73 (s, 2H), 4.38 (t, 2H, J = 7.3 Hz), 3.96 (s, 3H), 1.83 (quin, 2H, J = 7.3 Hz), 1.55 (sext, 2H, J = 7.3 Hz), 1.05 (t, 3H, J = 7.3 Hz).

### (Synthesis Example B-50)

### Synthesis of 5-butyl-2-(1',1',1',3',3',3'-hexafluoro-2'-hydroxypropan-2'-yl)-7,9-dimethoxy-phenanthridin-6(5H)-one (KZ38)

A target compound was obtained in the same manner as in Synthesis Example B-34, except for the use of N-butylaniline instead of 3-(butylamino)benzo[d][1,3]dioxole (k) and 2-bromo-2,4-dimethoxybenzoic acid instead of 2-iodo-5-methoxybenzoic acid.

¹H NMR (500 MHz, CDCl₃): δ 8.47 (s, 1H), 7.78 (d, 1H, J = 8.5 Hz), 7.27 (d, 1H, J = 8.5 Hz), 7.09 (d, 1H, J = 2.4 Hz), 6.59 (d, 1H, J = 2.4 Hz), 5.52 (brs, 1H), 4.20-4.05 (m, 2H), 4.02 (s, 3H), 3.92 (s, 3H), 1.63 (quin, 2H, J = 7.3 Hz), 1.42 (sext, 2H, J = 7.3 Hz), 0.94 (t, 3H, J = 7.3 Hz).

### (Synthesis Example B-51)

### Synthesis of 5-butyl-2-(1',1',1',3',3',3'-hexafluoro-2'-hydroxypropan-2'-yl)-7,8,9-trimethoxy-phenanthridin-6(5H)-one (KZ39)

A target compound was obtained in the same manner as in Synthesis Example B-34, except for the use of N-butylaniline instead of 3-(butylamino)benzo[d][1,3]dioxole (k) and 2-bromo-4,5,6-trimethoxybenzoic acid instead of 2-iodo-5-methoxybenzoic acid.

¹H NMR (500 MHz, CDCl₃): δ 8.49 (s, 1H), 7.78 (d, 1H, J = 9.2 Hz), 7.46 (s, 1H), 7.39 (d, 1H, J = 9.2 Hz), 4.31 (t, 2H, J = 7.3 Hz), 4.08 (s, 3H), 4.02 (s, 3H), 3.97 (s, 3H), 3.71 (brs, 1H), 1.77 (quin, 2H, J = 7.3 Hz), 1.53 (sext, 2H, J = 7.3 Hz), 1.02 (t, 3H, J = 7.3 Hz).

### (Synthesis Example B-52)

### Synthesis of 4-butyl-11-(1',1',1',3',3'3'-hexafluoro-2'-hydroxypropan-2'-yl)-[1,3]dioxolo [4,5-c]phenanthridin-5(4H)-one (HA-718)

A target compound was obtained in the same manner as in Synthesis Example B-34, except for the use of 2-iodobenzoyl chloride instead of 2-iodo-5-methoxybenzoic acid and chloromethylenedimethyliminium chloride.

HRMS (FAB) calculated: C₂₁H₁₈F₆NO₄ 462.1140; measured: 462.1169 (M+H)⁺; ¹H NMR (DMSO-d₆, 500 MHz) δ 8.98 (s, 1H), 8.32 (dd, J = 7.9, 1.2 Hz, 1H), 8.27 (s, 1H), 8.26 (d, J = 7.9 Hz, 1H), 7. 85 (td, J = 7.3, 1.2 Hz, 1H), 7.62 (t, J = 7.3 Hz, 1H), 6.19 (s, 2H), 4.43 (t, J = 7.9 Hz, 2H), 1.68 (tt, J = 7.3, 7.9 Hz, 2H), 1.37 (qt, J = 7.3, 7.3 Hz, 2H), 0.93 (t, J = 7.3 Hz, 3H).

### (Synthesis Example B-53)

### Synthesis of 5-butyl-2-(1',1',1',3',3',3'-hexafluoro-2'-hydroxypropan-2'-yl)-3,4,8-trimethoxy-phenanthridin-6(5H)-one (HA-727)

A target compound was obtained in the same manner as in Synthesis Example B-41, except for the use of 2-iodo-5-methoxybenzoic acid and chloromethylenedimethyliminium chloride instead of 2-iodobenzoyl chloride.

HRMS (FAB) calculated: C₂₃H₂₄F₆NO₅ 508.1559; measured: 508.1549 (M+H)⁺; ¹H NMR (DMSO-d₆, 500 MHz) δ 8.72 (s, 1H), 8.40 (s, 1H), 8.21 (d, J = 9.2 Hz, 1H), 7.75 (d, J = 3.1 Hz, 1H), 7.48 (dd, J = 9.2, 3.1 Hz, 1H), 4.50 (t, J = 7.3 Hz, 2H), 3.90 (s, 3H), 3.89 (s, 3H), 3.73 (s, 3H), 1.67 (tt, J = 7.3, 7.3 Hz, 2H), 1.28 (qt, J = 7.3, 7.3 Hz, 2H), 0.89 (t, J = 7.3 Hz, 3H).

### (Synthesis Example B-54)

### Synthesis of 11-(2'-benzyloxy-1',1',1',3',3',3'-hexafluoropropan-2'-yl)-4-butyl-7-methoxy-[1,3]dioxolo[4,5-c]phenanthridin-5(4H)-one (HA-758)

A target compound was obtained in the same manner as in Synthesis Example B-41-(4), except for the use of 4-butyl-11-(1',1',1',3',3',3'-hexafluoro-2'-hydroxypropan-2'-yl)-7-methoxy-[1,3]dioxolo[4,5-c]phenanthridin-5(4H)-one (HA-719) instead of N-butyl-N-[4-(2-benzyloxy-1,1,1,3,3,3-hexafluoropropan-2-yl)-2,3-dimethoxyphenyl]-2-iodo-benzamide (q).

HRMS (FAB) calculated: C₂₉H₂₆F₆NO₅ 582.1715; measured: 582.1704 (M+H)⁺; ¹H NMR (DMSO-d₆, 500 MHz) δ 7.80 (s, 1H), 7.73 (d, J = 3.1 Hz, 1H), 7.70 (d, J = 9.2 Hz, 1H), 7.39-7.47 (m, 5H), 7.31 (dd, J = 9.2, 3.1 Hz, 1H), 6.22 (s, 2H), 4.75 (s, 2H), 4.43 (t, J = 7.9 Hz, 2H), 1.69 (tt, J = 7.3, 7.9 Hz, 2H), 1.37 (qt, J = 7.3, 7.3 Hz, 2H), 0.93 (t, J = 7.3 Hz, 3H).

### [Test for anti-HCV activity using replicon cells]

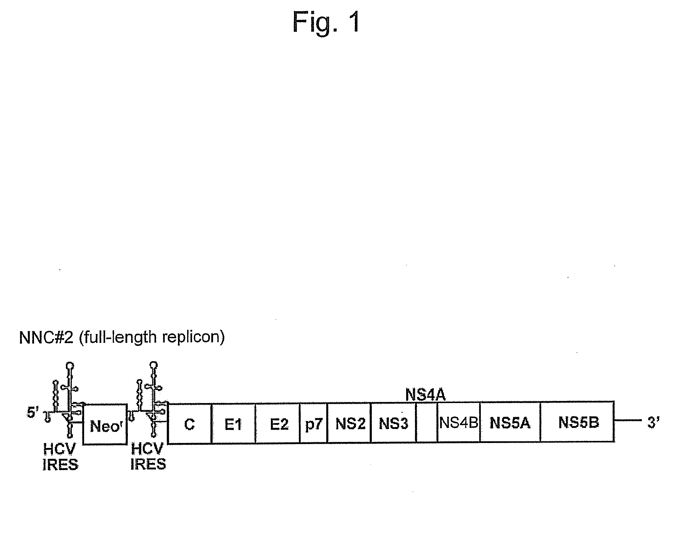

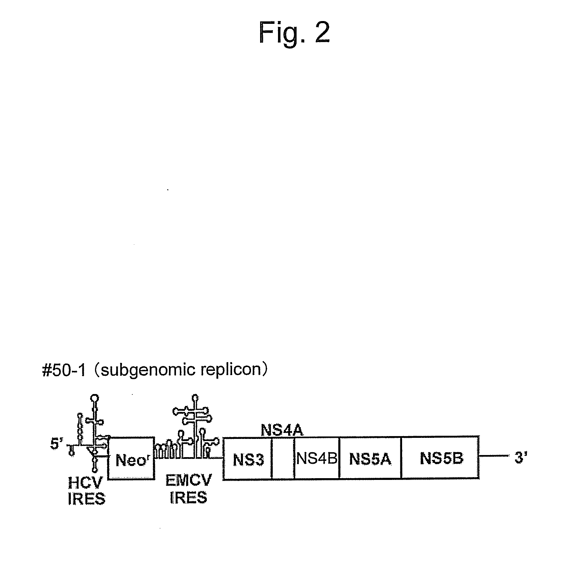

The 70% confluent #50-1 (subgenomic replion) cells or NNC #2 (full-length replicon) cells (Ishii et al., J. Virol., 2006, vol. 80, pp. 4510-4520) were dispersed in a culture bottle with the aid of trypsin, and the resulting dispersion was resuspended in a high-glucose Dulbecco's modified Eagle medium (DMEM) containing 10% FCS and 1 mg/ml G418 (Sigma) (see Figs. 1 and 2). The cell suspension adjusted at 5 x 10⁴ cells/ml with the use thereof was added to wells of a 96-well culture plate at 100 µl/well (the final cell density in each well: 5 x 10³ cells/ml), and culture was conducted at 37°C. Supernatants of culture media were removed from wells of the plate on the following day, 200 µl each of the solutions of test compounds appropriately diluted with a G418-free culture solution were added, and culture was further continued. Samples were prepared from cells in each well 3 days later with the use of the TaqMan^{®} Gene Expression Cell-to-CT^{®} (Applied Biosystems). Reverse transcription was carried out in accordance with the protocols for the ABI 7500 PCR systems (Applied Biosystems) at 37°C for 60 minutes, 95°C for 5 minutes, and retained at 4°C. Real-time PCR was carried out in accordance with the protocols for the ABI 7500 real-time PCR systems (Applied Biosystems) at 50°C for 2 minutes, 95°C for 10 minutes, 40 cycles at 95°C for 15 seconds, and 60°C for 1 minute.

In accordance with the method mentioned above, the level of the HCV genomic RNA and, as the internal standard, the level of mRNA of glyceraldehyde-3-phosphate dehydrogenase (GAPDH) (a house keeping gene of the host cells) were assayed, and a percentage (%) relative to the level of RNA of a control group to which the test compound was not administered was determined. The dose-response relationship of the test compound was analyzed based on the measured level of the HCV genomic RNA to determine EC₅₀, and the dose-response relationship of the test compound was analyzed based on the measured level of GAPDH RNA to determine CC₅₀. The term "EC₅₀" and "CC₅₀" used herein refers to 50% effective concentration (µM) and 50% cytotoxic concentration (µM), respectively.

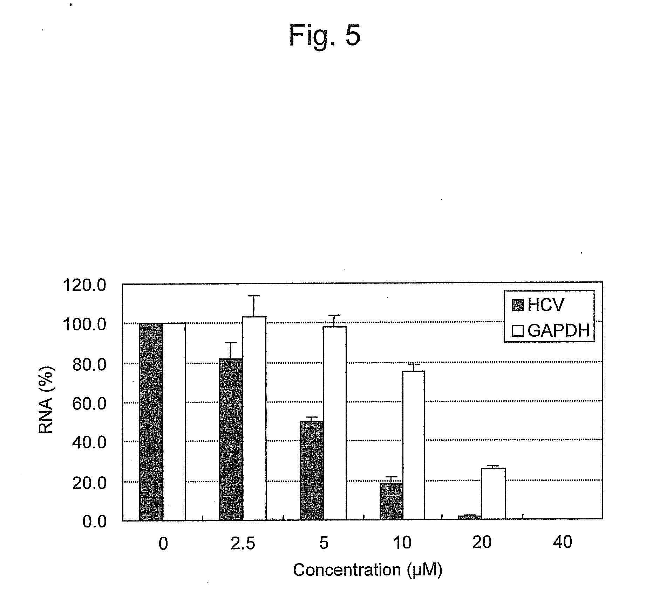

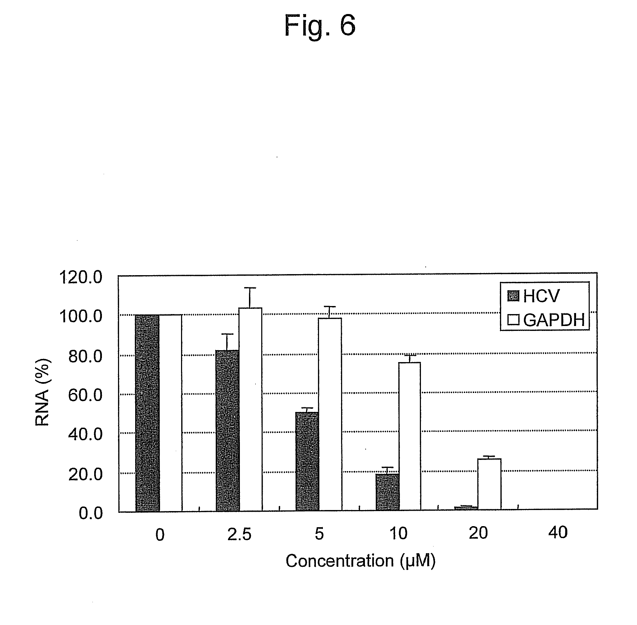

As shown in Figs. 3 to 6, compounds of Synthesis Example A-1 (AA-04), Synthesis Example A-2 (AA-24), Synthesis Example A-3 (AA-25), and Synthesis Example A-4 (AA-26) were found to have a high degree of anti-HCV activity in assays involving the use of full-length replion cells.

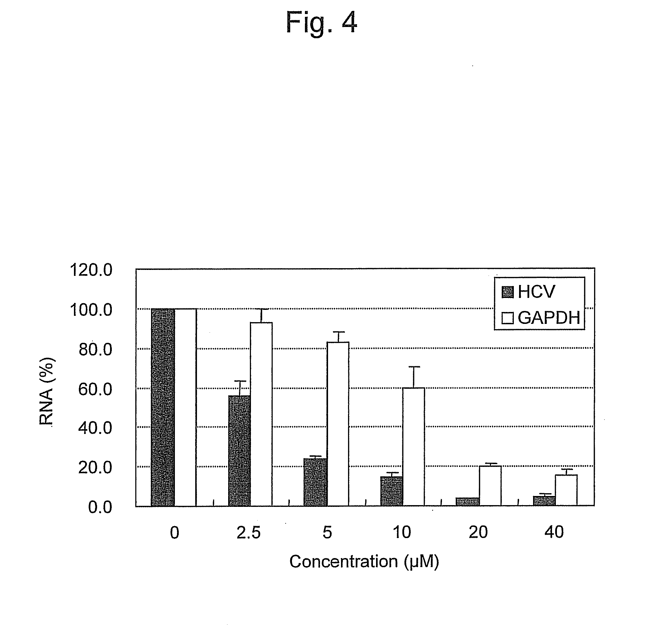

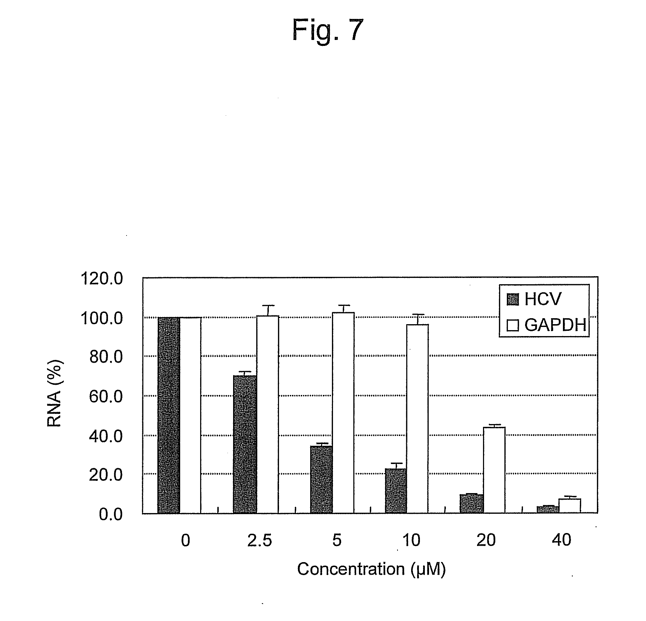

In particular, Compound AA-24 having a 5-butyl group exhibited a very high degree of anti-HCV activity; that is, EC₅₀ of 2.8 µM and CC₅₀ of 11.9 µM in assays involving the use of full-length replion cells (Fig. 4) and EC₅₀ of 4.0 µM and CC₅₀ of 20.5 µM in assays involving the use of subgenomic replion cells (Fig. 7) at non-cytotoxic concentrations.

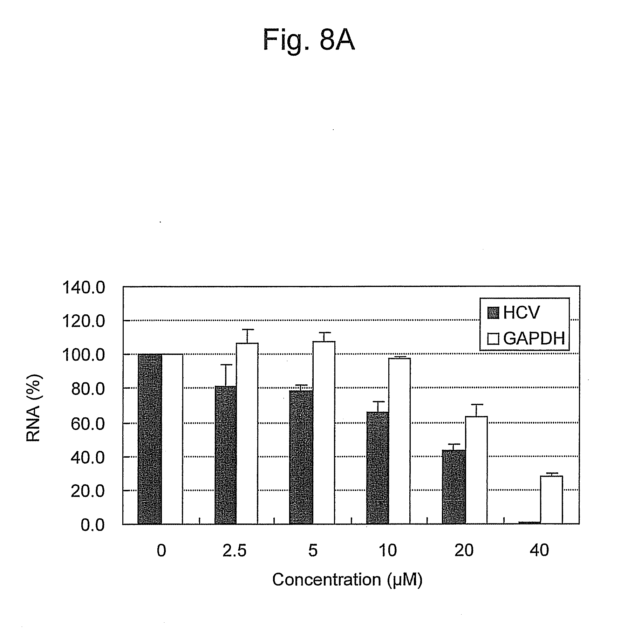

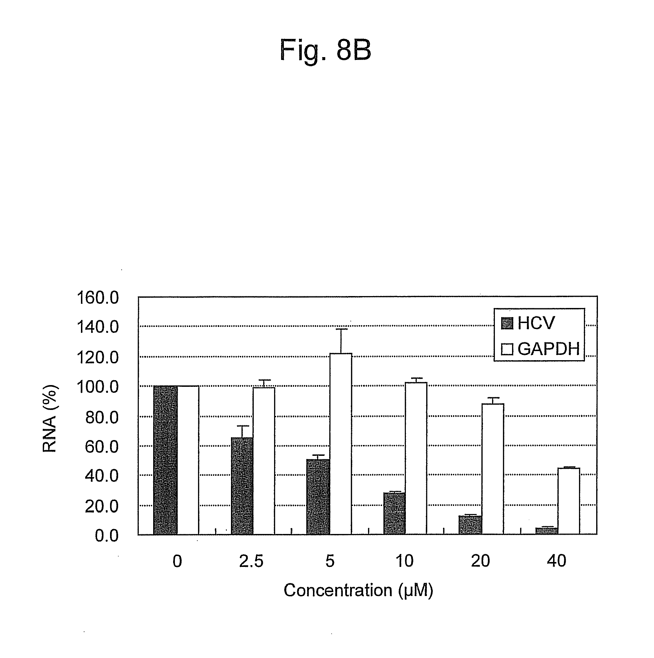

Subsequently, the compound of Synthesis Example B-14 (PN-iPr) also having a 5-butyl group as with Compound AA-24 was evaluated regarding anti-HCV activity observed in assays involving the use of full-length replion cells or subgenomic replion cells. The results are shown in Figs. 8A and 8B.

As shown in Figs. 8A and 8B, Compound PN-iPr exhibited a very high degree of anti-HCV activity; that is, EC₅₀ of 16.4 µM and CC₅₀ of 27.7 µM in assays involving the use of full-length replion cells and EC₅₀ of 4.0 µM and CC₅₀ of 20.5 µM in assays involving the use of subgenomic replion cells at non-cytotoxic concentrations.

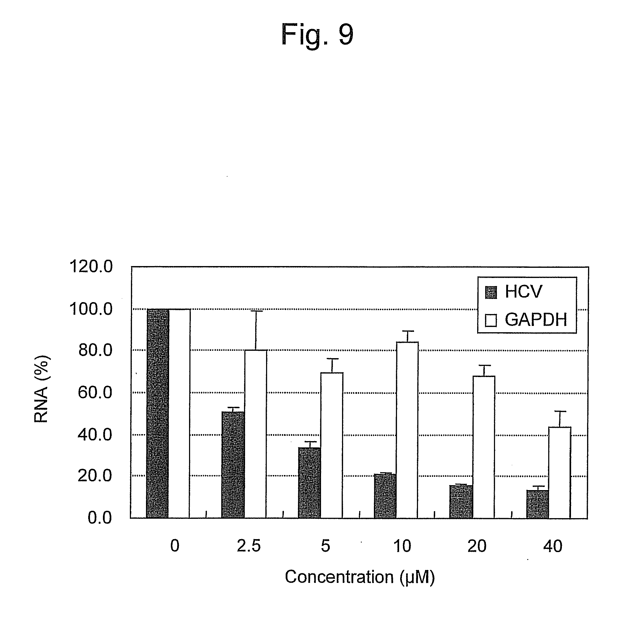

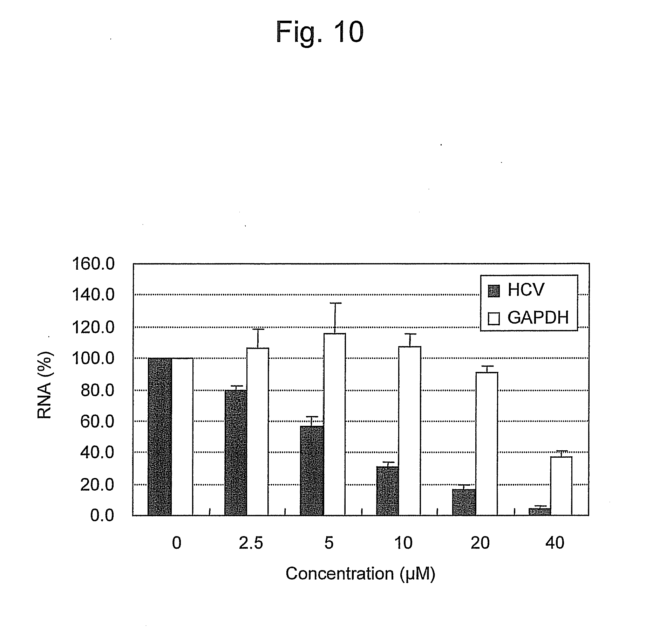

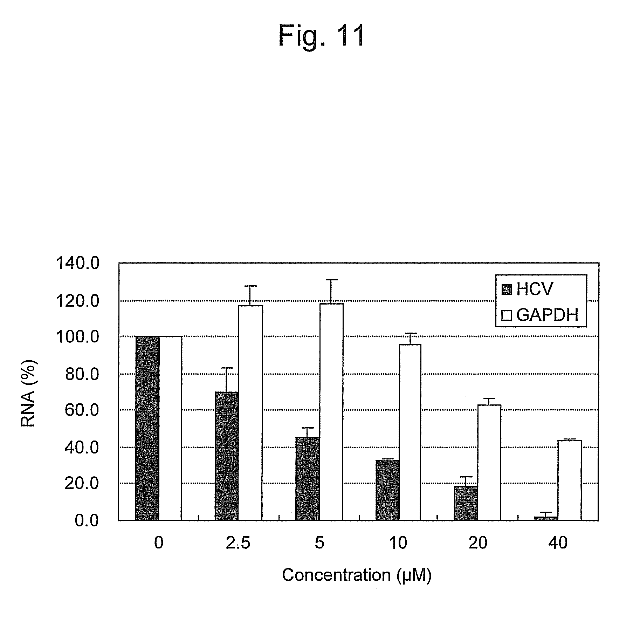

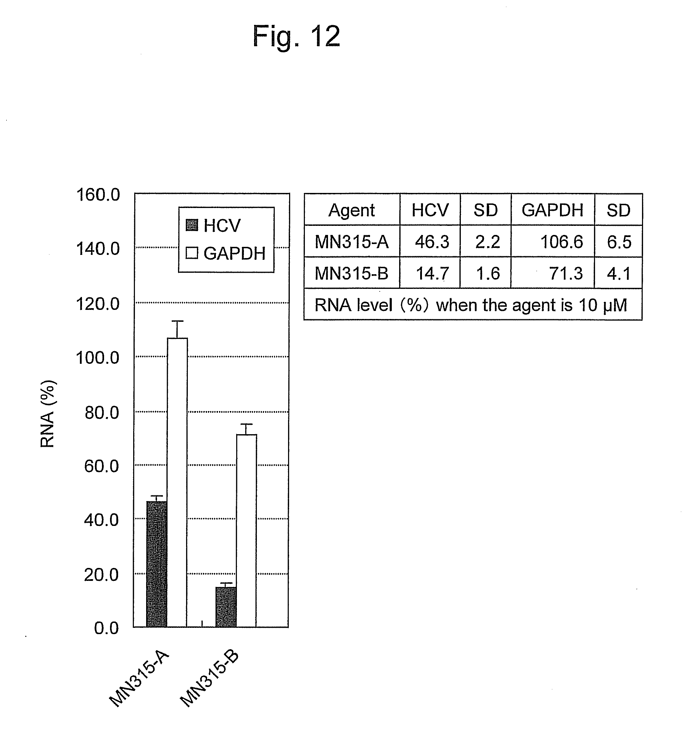

Based on the results demonstrated above, it was deduced that a certain correlation would exist between the presence of a 5-butyl group and anti-HCV activity. Accordingly, the compounds of Synthesis Example B-13(MN315-A), Synthesis Example B-15 (MN315-B), Synthesis Example B-16 (MN325), Synthesis Example B-20 (MN256), and Synthesis Example B-30 (MN314-B) also having 5-butyl groups were evaluated regarding anti-HCV activity via assays using subgenomic replion cells. Fig. 9 to Fig. 11 each show the results regarding Compounds MN314-B, MN325, and MN256, and Fig. 12 shows the results regarding Compounds MN315-A and MN315-B.

As shown in Figs. 9 to 11, Compounds MN314-B, MN325, and MN256 exhibited a low degree of cytotoxicity and a very high degree of anti-HCV activity; that is, MN314-B exhibited EC₅₀ of 2.0 µM and CC₅₀ of 33.5 µM; MN325 exhibited EC₅₀ of 6.1 µM and CC₅₀ of 34.4 µM; and MN256 exhibited EC₅₀ of 5.0 µM and CC₅₀ of 31.7 µM.

As shown in Fig. 12, Compound MN315-A exhibited the HCV genomic RNA level of 46.3% when it was administered at a concentration of 10 µM; that is, anti-HCV activity thereof was somewhat lower than that of other compounds. In contrast, Compound MN315-B exhibited the HCV genomic RNA level of 14.7% under the same conditions; that is, anti-HCV activity thereof was substantially the same as that of other compounds.

Table 1 shows the results of assays of anti-HCV activity conducted with the use of subgenomic replion cells regarding the compounds of Synthesis Example B-26 (MN313-A), Synthesis Example B-27 (MN313-B), Synthesis Example B-28 (MN305), Synthesis Example B-29 (MN306), Synthesis Example B-30 (MN314-B), and Synthesis Example B-31 (MN314-A), in each of which a 5-butyl group exists and a fused ring is formed.

As shown in Table 1, Compounds MN313-A, MN313-B, MN306, and MN314-B exhibited a very high degree of anti-HCV activity at non-cytotoxic concentrations.

**Table 1**

| Compound | EC₅₀ (µM) | CC₅₀ (µM) |
|---|---|---|
| MN-313-A (Synthesis Examples B-26) | 29.1 | 44.5 |
| MN-313-B (Synthesis Example B-27) | 3.2 | 6.3 |
| MN-305 (Synthesis Example B-28) | 19.5 | 24.3 |
| MN-306 (Synthesis Example B-29) | 29.9 | >40 |
| MN-314-B (Synthesis Example B-30) | 11.0 | >40 |
| MN-314-A (Synthesis Example B-31) | >31.4 | 31.4 |

### [Test for anti-HCV activity using replicon cells (luciferase assay)]

Subgenomic HCV RNA replicon cells (LucNeo#2, Goto et al., Biochem. Biophys. Res. Commun., 2006, vol. 343, pp. 879-884) having a luciferase gene were suspended in a Dulbecco's modified Eagle medium (DMEM) containing 10% FBS and 1 mg/ml G418 (Sigma). The cells were incubated for 24 hours and then cultured in G418-free fresh media each containing test compounds at various concentrations for 3 days. In order to perform luciferase assays, the cells were washed with PBS and treated with a lysate for 10 minutes with intermittent agitation. A cell lysate (25 µl) was transferred to a white microtiter plate. Luciferase assay reagents (100 µl, Promega, Tokyo) were added to each well and the luciferase activity was measured with a luminometer.

A tetrazolium staining solution (10 µl, Seikagaku Corporation, Tokyo) was added to each well in order to examine cytotoxicity (a cell survival rate). After the cells were incubated for 1 hour, the absorbance at the characteristic absorption wavelength (450 nm) was measured with the use of a microplate reader.

Based on the luciferase assay results, the HCV genomic RNA level was measured, and a percentage (%) relative to the level of RNA of a control group to which the test compound was not administered was determined. Based on the results of tetrazolium staining, cell survival rates (%) of the group to which the test compound had been administered and of a control group were determined. The dose-response relationship of the test compound was analyzed based on the results of quantitative luciferase assays to determine EC₅₀ (µM), and the dose-response relationship of the test compound was analyzed based on the results of tetrazolium staining to determine CC₅₀.

Table 2 shows the results of assays for anti-HCV activity and cytotoxicity of compounds of Synthesis Example B-36 to Synthesis Example B-55.

**Table 2**

| Compound | EC₅₀ (µM) | CC₅₀ (µM) |
|---|---|---|
| HA-719 (Synthesis Example B-34) | 0.050 | 6.4 |
| KZ25 (Synthesis Example B-37) | >2.8 | 2.8 |
| KZ26 (Synthesis Example B-38) | 2.3 | >100 |
| KZ27 (Synthesis Example B-39) | 5.5 | 14.2 |
| KZ28 (Synthesis Example B-40) | 8.3 | 9.4 |
| HA-726 (Synthesis Example B-41) | 2.6 | 10.4 |
| KZ29 (Synthesis Example B-42) | 30.4 | >100 |
| KZ30 (Synthesis Example B-43) | 9.2 | >100 |
| KZ31 (Synthesis Example B-44) | 10.0 | 12.6 |
| KZ32 (Synthesis Example B-45) | 16.6 | 28.9 |
| KZ33 (Synthesis Example B-46) | 5.0 | 6.9 |
| KZ34 (Synthesis Example B-47) | 7.2 | 25.3 |
| KZ35 (Synthesis Example B-48) | 44.0 | 52.3 |
| KZ36 (Synthesis Example B-48) | 2.2 | 5.7 |
| KZ37 (Synthesis Example B-49) | 2.6 | >100 |
| KZ38 (Synthesis Example B-50) | 2.6 | 8.2 |
| KZ39 (Synthesis Example B-51) | >2.0 | 2.0 |
| HA-718 (Synthesis Example B-52) | 0.29 | 24.2 |
| HA-727 (Synthesis Example B-53) | 1.8 | 6.8 |
| HA-758 (Synthesis Example B-54) | 1.71 | >100 |

As shown in Table 1, Compounds KZ26, KZ30, KZ37, and HA-758 exhibited a high degree of anti-HCV activity at non-cytotoxic concentrations. While Compound HA-719 exhibited a high degree of cytotoxicity, anti-HCV activity thereof was very high.

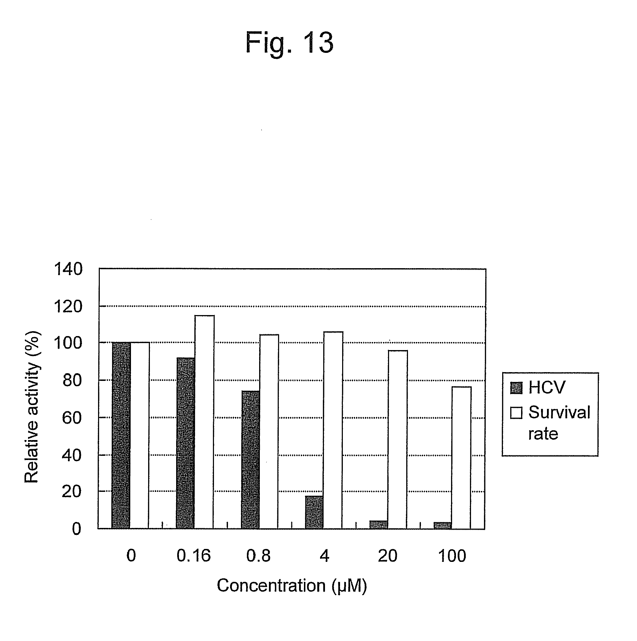

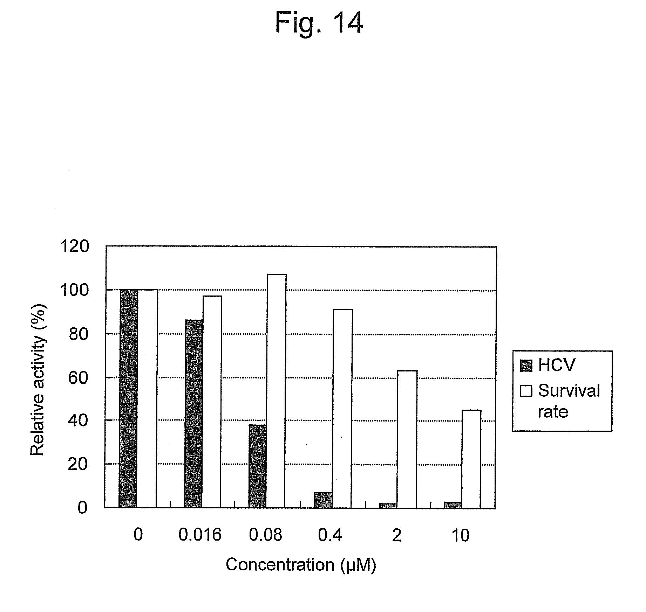

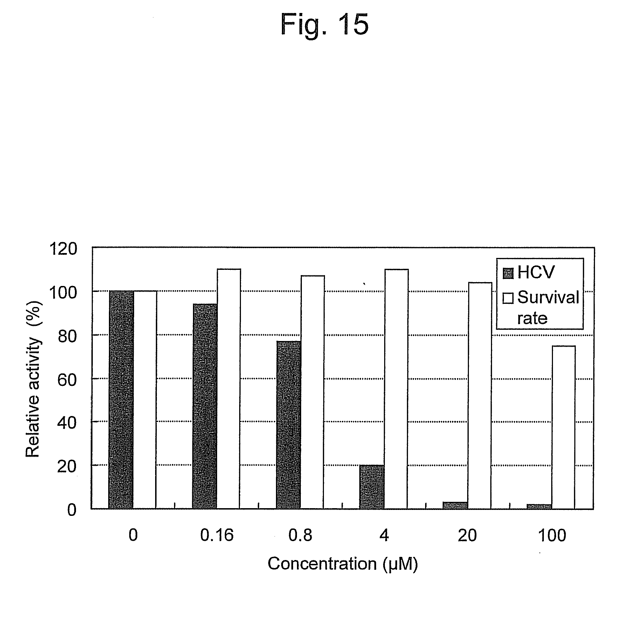

Figs. 13 to 15 show anti-HCV activity and cytotoxicity of Compounds KZ37, HA-719, and HA-758, respectively.

As shown in Figs. 13 to 15, the compounds of Synthesis Example B-34 (HA-719), Synthesis Example B-49 (KZ37), and Synthesis Example B-54 (HA-758) were found to exhibit a high degree of anti-HCV activity via luciferase assays.

All publications, patents, and patent applications cited herein are incorporated herein by reference in their entirety.

## Claims

1. A therapeutic agent for use in the prevention or treatment of hepatitis C which agent comprises, as an active ingredient, a compound represented by formula (I) or a pharmaceutically acceptable salt thereof: 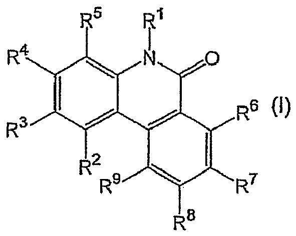 wherein,
R¹ is selected from among C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈g alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)); and
R² to R⁹ are each independently selected from Substituent group A consisting of hydrogen, halogen, hydroxyl, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl) and Q-(heteroarylalkyl) (wherein Q is O or S), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)),
provided that any two of R² to R⁵ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)), or
any two of R⁶ to R⁸ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)),
with the exception of 5-butyl-1-methylphenanthridin-6(5H)-one, 5-butyl-3-methylphenanthridin-6(5H)-one, 5-butyl-4-methylphenanthridin-6(5H)-one, 5-butyl-7-methylphenanthridin-6(5H)-one, 5-butyl-8-methylphenanthridin-6(5H)-one, 5-butyl-9-methylphenanthridin-6(5H)-one, 5-butyl-10-methylphenanthridin-6(5H)-one, 5-butylbenzo[c]phenanthridin-6(5H)-one, and 5-butylbenzo[k]phenanthridin-6(5H)-one.

2. The therapeutic agent for use in the prevention or treatment of hepatitis C according to claim 1, wherein R² to R⁹ satisfy any of the conditions [1] to [5] below:
[1] R⁵ to R⁹ are hydrogen atoms, any one of R² to R⁴ is a group selected from Substituent group A, and the other remaining groups are hydrogen atoms;
[2] R⁶ to R⁹ are hydrogen atoms, R² and R³ or R³ and R⁴ of R² to R⁵ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)), and the other remaining groups are hydrogen atoms;
[3] R² to R⁵ are hydrogen atoms, R⁶ and R⁷ or R⁷ and R⁸ of R⁶ to R⁹ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylakyl, Q-(C₁-C₈ alkyl), Q-(C₂-C₈ alkenyl), Q-(C₂-C₈ alkenyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)), and the other remaining groups are hydrogen atoms;
[4] R³ is 2'-hydroxy-1',1',1',3',3',3'-hexafluoropropyl or 2'-benzyloxy-1',1',1',3',3',3'-hexafluoropropyl, up to 3 members of R² and R⁴ to R⁹ are each independently a group selected from Substituent group A, and the other remaining groups are hydrogen atoms; and
[5] R³ is 2'-hydroxy-1',1',1',3',3',3'-hexafluoropropyl or 2'-benzyloxy-1',1',1',3',3,3'-hexafluoropropyl, R⁴ and R⁵ or R⁷ and R⁸ of R² and R⁴ to R⁹ together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more groups selected from among halogen, OH, NH₂, NO₂, C(O)Z (wherein Z is hydrogen, hydroxyl, C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, or NH₂), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, heteroarylalkyl, Q-(C₁-C₈ alkyl), Q-(C₂-C₈ alkenyl), Q-(C₂-C₈ alkynyl), Q-(C₃-C₆ cycloalkyl), Q-(C₃-C₆ cycloalkenyl), Q-(C₄-C₆ cycloalkynyl), Q-(heterocyclyl), Q-(aryl), Q-(arylalkyl), Q-(heteroaryl), and Q-(heteroarylalkyl) (wherein Q is O or S)), any one of the other groups is selected from Substituent group A, and the other remaining groups are hydrogen atoms.

3. The therapeutic agent for use in the prevention or treatment of hepatitis C according to claim 1 or 2, wherein R¹ is a group selected from the group consisting of butyl, benzyl, hexyl, and cyclohexylmethyl.

4. The therapeutic agent for use in the prevention or treatment of hepatitis C according to claim 2 or 3, wherein R² to R⁹ satisfy condition [1] and the group selected from among the Substituent group A is hydrogen, fluorine, trifluoromethyl, 1',1',1',3',3',3'-hexafluoropropyl, isopropyl, 2'-hydroxy-1',1',1',3',3',3'-hexafluoropropyl, or tert-butyl.

5. The therapeutic agent for use in the prevention or treatment of hepatitis C according to claim 4, wherein the group selected from among the Substituent group A is hydrogen.

6. The therapeutic agent for use in the prevention or treatment of hepatitis C according to claim 2 or 3, wherein R² to R⁹ satisfy condition [2] or [3] and cycloalkyl, heterocyclyl, or aryl that is fused to the phenanthridine ring is unsubstituted or substituted with one or more C₁-C₈ alkyl groups.

7. The therapeutic agent for use in the prevention or treatment of hepatitis C according to claim 2 or 3, wherein:
(a) R² to R⁹ satisfy condition [4] and the group selected from Substituent group A is hydroxyl or methoxyl; or
(b) R² to R⁹ satisfy condition [5] and cycloalkyl, heterocyclyl, or aryl that is fused to the phenanthridine ring is unsubstituted 1,3-dioxole.

8. The therapeutic agent for use in the prevention or treatment of hepatitis C according to claim 1, wherein the compound represented by formula (I) is selected from the group consisting of the compounds below:
5-butyl-2-(2'-hydroxy-1',1',1',3',3',3'-hexafluoropropan-2'-yl)-phenanthridin-6(5H)-one;
5-benzyl-2-(2'-hydroxy-1',1',,1',3',3',3'-hexafluoropropan-2'-yl)-phenanthridin-6(5H)-one;
5-hexyl-2-(2'-hydroxy-1',1',1',3',3',3'-hexafluoropropan-2'-yl)-phenanthridin-6(5H)-one;
5-cyclohexylmethyl-2-(2'-hydroxy-1',1',',1',3',3',3'-hexafluoropropan-2'-yl)-phenanthridin-6(5H)-one;
5-butyl-1-isopropylphenanthridin-6(5H)-one;
5-butyl-2-isopropylphenanthridin-6(5H)-one;
5-butyl-3-isopropylphenanthridin-6(5H)-one;
5-butyl-2-tert-butylphenanthridin-6(5H)-one;
6-butylbenzo[a]phenanthridin-5(6H)-one;
6-butylbenzo[b]phenanthridin-5(6H)-one;
6-butylbenzo[i]phenanthridin-5(6H)-one;
5-butylbenzo[j]phenanthridin-6(5H)-one;
6-butyl-8,9,10,11-tetrahydro-8,8,11,11-tetramethylbenzo[2,3-b] phenanthridin-5(6H)-one;
2-((2'-benzyloxy-1',1',1',3',3',3'-hexafluoropropan-2'-yl)-5-butyl-3-methoxyphenanthridin-6(5H)-one;
4-butyl-11-(1',1',1',3',3',3'-hexafluoro-2'-hydroxypropan-2'-yl)-7-methoxy-[1,3]dioxolo[4,5-c]phenanthridin-5(4H)-one; and
11-((2'-benzyloxy-1',1',1',3',3',3'-hexafluoropropan-2'-yl)-4-butyl-7-methoxy-[1,3]dioxolo[4,5-c]phenanthridin-5(4H)-one.

9. A compound represented by formula (IA) or a salt thereof: 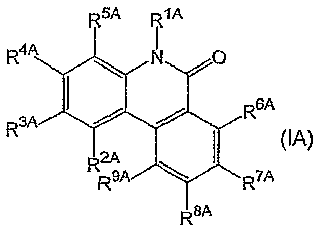 wherein
R^{1A} is either butyl or cyclohexylmethyl (each of these groups being independently unsubstituted or substituted with one or more groups selected from among halogen, OH, O-(C₂-C₈ alkynyl), O-(C₃-C₆ cycloalkyl), O-(C₃-C₆ cycloalkenyl), or O-(C₄-C₆ cycloalkynyl)); and
R^{2A} to R^{9A} are each independently selected from Substituent group B consisting of hydrogen, halogen, hydroxyl, C(O)Z (wherein Z is hydrogen, hydroxyl, C₁-C₈ alkyl, or NH₂), Q-(C₁-C₈ alkyl), Q-(C₂-C₈ alkenyl), Q-(C₂-C₈ alkynyl) and Q-(C₃-C₆ cycloalkyl) (wherein Q is O or S), C₁-C₈ alkyl, C₂-C₈ alkenyl, C₂-C₈ alkynyl, C₃-C₆ cycloalkyl, C₃-C₆ cycloalkenyl, C₄-C₆ cycloalkynyl, heterocyclyl, aryl, arylalkyl, arylalkenyl, heteroaryl, and heteroarylalkyl (each of these groups being independently unsubstituted or substituted with one or more groups selected from among halogen, OH, and O-(arylalkyl)),
provided that any two of R^{2A} to R^{5A} of R^{2A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), or
any two of R^{6A} to R^{9A} of R^{2A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups),
with the exception of 5-butyl-1-methylphenanthridin-6(5H)-one, 5-butyl-3-methylphenanthridin-6(5H)-one, 5-butyl-4-methylphenantbridin-6(5H)-one, 5-butyl-7-methylphenanthridin-6(5H)-one, 5-butyl-8-methylphenanthridin-6(5H)-one, 5-butyl-9-methylphenanthridin-6(5H)-one, 5-butyl-10-methylphenanthridin-6(5H)-one, 5-butylbenzo[c]phenanthridin-6(5H)-one, 5-butylbenzo[k]phenanthridin-6(5H)-one, 5-butylphenanthridin-6(5H)-one, and 5-(4-bromobutyl)phenanthridin-6(5H)-one.

10. The compound according to claim 9 or a salt thereof, wherein R^{2A} to R^{9A} satisfy any of the conditions [1A] to [5A] below:
[1A] R^{5A} to R^{9A} are hydrogen atoms, any one of R^{2A} to R^{4A} is a group selected from Substituent group B, and the other remaining groups are hydrogen atoms;
[2A] R^{6A} to R^{9A} are hydrogen atoms, R^{2A} and R^{3A} or R^{3A} and R^{4A} of R^{2A} to R^{5A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), and the other remaining groups are hydrogen atoms;
[3A] R^{2A} to R^{5A} are hydrogen atoms, R^{6A} and R^{7A} or R^{7A} and R^{8A} of R^{6A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), and the other remaining groups are hydrogen atoms;
[4A] R^{3A} is 2'-hydroxy-1',1',1',3',3',3'-hexafluoropropyl or 2'-benzyloxy-1',1',1',3',3',3'-hexafluoropropyl, up to 3 members of R^{2A} and R^{4A} to R^{9A} are each independently a group selected from Substituent group B, and the other remaining groups are hydrogen atoms; and
[5A] R^{3A} is 2'-hydroxy-1',1',1',3',3',3'-hexafluoropropyl or 2'-benzyloxy-1',1',1',3',3',3'-hexafluoropropyl, R^{4A} and R^{5A} or R^{7A} and R^{8A} of R^{2A} and R^{4A} to R^{9A} together with carbon atoms on the phenanthridine ring to which they are bound may form cycloalkyl, heterocyclyl, or aryl fused to the phenanthridine ring (the cycloalkyl, heterocyclyl, or aryl fused to the phenanthridine ring being independently unsubstituted or substituted with one or more C₁-C₈ alkyl groups), any one of the other groups is selected from Substituent group B, and the other remaining groups are hydrogen atoms.

11. The compound according to claim 9 or 10 or a salt thereof, wherein R^{1A} is butyl.

12. The compound according to claim 9 or a salt thereof, wherein the compound represented by formula (IA) is selected from the group consisting of the compounds below:
5-butyl-2-(2'-hydroxy-1',1',1',3',3',3'-hexafluoropropan-2'-yl)-phenanthridin-6(5H)-one;
5-cyclohexylmethyl-2-(2'-hydroxy-1',1',1',3',3',3'-hexafluoropropan-2'-yl)-phenanthridin-6(5H)-one;
5-butyl-1-isopropylphenanthridin-6(5H)-one;
5-butyl-2-isopropylphenanthridin-6(5H)-one;
5-butyl-3-isopropylphenanthridin-6(5H)-one;
5-butyl-2-tert-butylphenanthridin-6(5H)-one;
6-butylbenzo[a]phenanthridin-5(6H)-one;
6-butylbenzo[b]phenanthridin-5(6H)-one;
6-butylbenzo[i]phenanthridin-5(6H)-one;
5-butylbenzo[j]phenanthridin-6(5H)-one;
6-butyl-8,9,10,11-tetrahydro-8,8,11,11-tetramethylbenzo[2,3-b]phenanthridin-5(6H)-one;
2-((2'-benzyloxy-1',1',1',3',3',3'-hexafluoropropan-2'-yl)-5-butyl-3-methoxyphenanthridin-6(5H)-one;
4-butyl-11-(1',1',1',3',3',3'-hexafluoro-2'-hydroxypropan-2'-yl)-7-methoxy-[1,3] dioxolo[4,5-c]phenanthridin-5(4H)-one; and
11-((2'-benzyloxy-1',1',1', 3',3',3'-hexafluoropropan-2'-yl)-4-butyl-7-methoxy-[1,3]dioxolo[4,5-c]phenanthridin-5(4H)-one.

13. A method of preparing the compound represented by formula (IA) according to claim 9 or a salt thereof comprising the steps described below:
step (1A) of allowing an aniline derivative represented by formula (II): 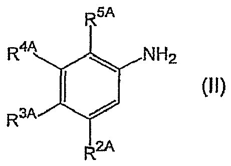 wherein
R^{2A} to R^{5A} are as defined in claim 9,
to be bound to a benzoyl halide represented by formula (III): 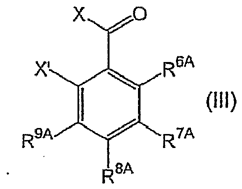 wherein
R^{6A} to R^{9A} are as defined in claim 9; and
X and X' are each independently selected from among halogen atoms, to generate a benzanilide represented by formula (IV): 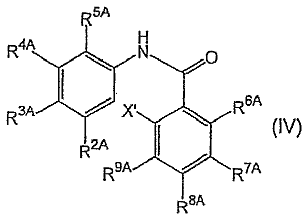 wherein
R^{2A} to R^{9A} and X' are as defined in claim 9;
step (1B) of allowing the benzanilide represented by formula (IV) to react with a halide represented by formula (V):
R^{1A}-X" (V)
wherein
R^{1A} is as defined in claim 9; and
X" is halogen,
to generate a N-substituted benzanilide represented by formula (VI): 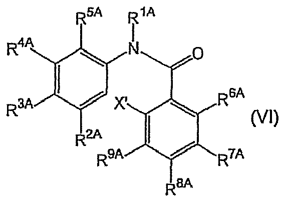 wherein R^{1A} to R^{9A} and X' are as defined above; and
step (1C) of subjecting the N-substituted benzanilide represented by formula (VI) to an intramolecular cyclization reaction in the presence of palladium acetate to generate the compound represented by formula (IA).

14. A method of preparing the compound represented by formula (IA) according to claim 9 or a salt thereof comprising the steps described below:
step (2C) of allowing N-substituted aniline represented by formula (IV"): 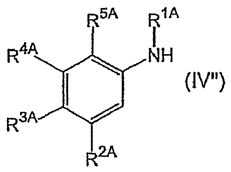 wherein
R^{1A} to R^{5A} are as defined in claim 9,
to be bound to a benzoyl halide represented by formula (III): 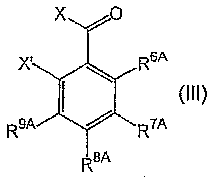 wherein
R^{6A} to R^{9A} are as defined in claim 9; and
X and X' are each independently selected from among halogen atoms, to generate a N-substituted benzanilide represented by formula (VI): 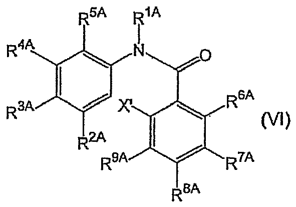 wherein
R^{1A} to R^{9A} and X' are as defined in claim 9; and
step (2D) of subjecting the N-substituted benzanilide represented by formula (VI) to an intramolecular cyclization reaction in the presence of palladium acetate to generate the compound represented by formula (IA).

15. A method of preparing the compound represented by formula (IA) according to claim 9 or a salt thereof comprising the steps described below:
step (4C) of allowing a N-substituted aniline represented by formula (IV"): 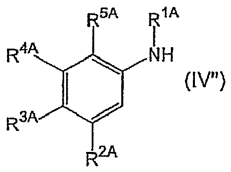 wherein
R^{1A} to R^{5A} are as defined in claim 9,
to be bound to a halobenzoic acid represented by formula (III'): 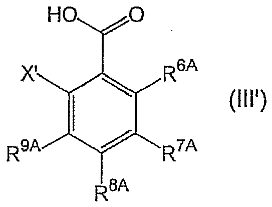 wherein
R^{6A} to R^{9A} are as defined in claim 9; and
X' is a halogen atom,
to generate a N-substituted benzanilide represented by formula (VI): 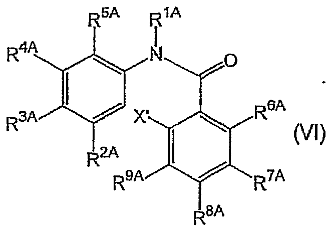 wherein
R^{1A} to R^{9A} and X' are as defined above; and
step (4D) of subjecting the N-substituted benzanilide represented by formula (VI) to an intramolecular cyclization reaction in the presence of palladium acetate to generate the compound represented by formula (IA).

## Patentansprüche

1. Therapeutisches Mittel zur Verwendung bei der Prävention oder Behandlung von Hepatitis C, wobei das Mittel als Wirkstoff eine Verbindung, die durch Formel (I) dargestellt wird, oder ein pharmazeutisch annehmbares Salz davon umfasst: 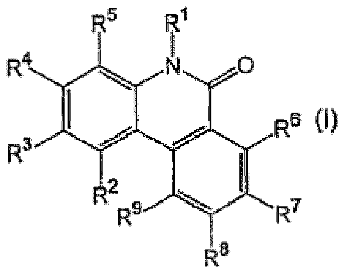 wobei
R¹ aus C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl und Heteroarylalkyl ausgewählt ist (wobei diese Gruppen jeweils unabhängig voneinander unsubstituiert sind oder mit einer oder mehreren Gruppen substituiert sind, die aus Halogen, OH, NH₂, NO₂, C(O)Z (wobei Z Wasserstoff, Hydroxy, C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl oder NH₂ ist), C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl, Heteroarylalkyl, Q-(C₁-C₈-Alkyl), Q-(C₂-C₈-Alkenyl), Q-(C₂-C₈-Alkinyl), Q-(C₃-C₆-Cycloalkyl), Q-(C₃-C₆-Cycloalkenyl), Q-(C₄-C₆-Cycloalkinyl), Q-(Heterocyclyl), Q-(Aryl), Q-(Arylalkyl), Q-(Heteroaryl) und Q-(Heteroarylalkyl) (wobei Q = O oder S ist) ausgewählt sind); und
R² bis R⁹ jeweils unabhängig aus Substituentengruppe A ausgewählt sind, die aus Wasserstoff, Halogen, Hydroxy, C(O)Z (wobei Z Wasserstoff, Hydroxy, C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl oder NH₂ ist), Q-(C₁-C₈-Alkyl), Q-(C₂-C₈-Alkenyl), Q-(C₂-C₈-Alkinyl), Q-(C₃-C₆-Cycloalkyl), Q-(C₃-C₆-Cycloalkenyl), Q-(C₄-C₆-Cycloalkinyl), Q-(Heterocyclyl), Q-(Aryl), Q-(Arylalkyl), Q-(Heteroaryl) und Q-(Heteroarylalkyl) (wobei Q = O oder S ist), C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl und Heteroarylalkyl besteht (wobei diese Gruppen jeweils unabhängig voneinander unsubstituiert sind oder mit einer oder mehreren Gruppen substituiert sind, die aus Halogen, OH, NH₂, NO₂, C(O)Z (wobei Z Wasserstoff, Hydroxy, C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl oder NH₂ ist), C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl, Heteroarylalkyl, Q-(C₁-C₈-Alkyl), Q-(C₂-C₈-Alkenyl), Q-(C₂-C₈-Alkinyl), Q-(C₃-C₆-Cycloalkyl), Q-(C₃-C₆-Cycloalkenyl), Q-(C₄-C₆-Cycloalkinyl), Q-(Heterocyclyl), Q-(Aryl), Q-(Arylalkyl), Q-(Heteroaryl) und Q-(Heteroarylalkyl) (wobei Q = O oder S ist) ausgewählt sind);
mit der Maßgabe, dass je zwei von R² bis R⁵ zusammen mit Kohlenstoffatomen am Phenanthridinring, an die sie gebunden sind, Cycloalkyl, Heterocyclyl oder Aryl, das mit dem Phenanthridinring kondensiert ist, bilden können (wobei das Cycloalkyl, Heterocyclyl oder Aryl, das mit dem Phenanthridinring kondensiert ist, jeweils unabhängig unsubstituiert ist oder mit einer oder mehreren Gruppen substituiert ist, die aus Halogen, OH, NH₂, NO₂, C(O)Z (wobei Z Wasserstoff, Hydroxy, C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl oder NH₂ ist), C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl, Heteroarylalkyl, Q-(C₁-C₈-Alkyl), Q-(C₂-C₈-Alkenyl), Q-(C₂-C₈-Alkinyl), Q-(C₃-C₆-Cycloalkyl), Q-(C₃-C₆-Cycloalkenyl), Q-(C₄-C₆-Cycloalkinyl), Q-(Heterocyclyl), Q-(Aryl), Q-(Arylalkyl), Q-(Heteroaryl) und Q-(Heteroarylalkyl) (wobei Q = O oder S ist) ausgewählt sind); oder
je zwei von R⁶ bis R⁸ zusammen mit Kohlenstoffatomen am Phenanthridinring, an die sie gebunden sind, Cycloalkyl, Heterocyclyl oder Aryl, das mit dem Phenanthridinring kondensiert ist, bilden können (wobei das Cycloalkyl, Heterocyclyl oder Aryl, das mit dem Phenanthridinring kondensiert ist, jeweils unabhängig unsubstituiert ist oder mit einer oder mehreren Gruppen substituiert ist, die aus Halogen, OH, NH₂, NO₂, C(O)Z (wobei Z Wasserstoff, Hydroxy, C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl oder NH₂ ist), C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl, Heteroarylalkyl, Q-(C₁-C₈-Alkyl), Q-(C₂-C₈-Alkenyl), Q-(C₂-C₈-Alkinyl), Q-(C₃-C₆-Cycloalkyl), Q-(C₃-C₆-Cycloalkenyl), Q-(C₄-C₆-Cycloalkinyl), Q-(Heterocyclyl), Q-(Aryl), Q-(Arylalkyl), Q-(Heteroaryl) und Q-(Heteroarylalkyl) (wobei Q = O oder S ist) ausgewählt sind);
mit Ausnahme von 5-Butyl-1-methylphenanthridin-6(5H)-on, 5-Butyl-3-methylphenanthridin-6(5H)-on, 5-Butyl-4-methylphenanthridin-6(5H)-on, 5-Butyl-7-methylphenanthridin-6(5H)-on, 5-Butyl-8-methylphenanthridin-6(5H)-on, 5-Butyl-9-methylphenanthridin-6(5H)-on, 5-Butyl-10-methylphenanthridin-6(5H)-on, 5-Butylbenzo[c]phenanthridin-6(5H)-on und 5-Butylbenzo[k]phenanthridin-6(5H)-on.

2. Therapeutisches Mittel zur Verwendung bei der Prävention oder Behandlung von Hepatitis C gemäß Anspruch 1, wobei R² bis R⁹ irgendwelchen der folgenden Bedingungen [1] bis [5] genügen:
[1] R⁵ bis R⁹ sind Wasserstoffatome, irgendeines von R² bis R⁴ ist eine Gruppe, die aus Substituentengruppe A ausgewählt ist, und die übrigen Gruppen sind Wasserstoffatome;
[2] R⁶ bis R⁹ sind Wasserstoffatome, R² und R³ oder R³ und R⁴ von R² bis R⁵ können zusammen mit Kohlenstoffatomen am Phenanthridinring, an die sie gebunden sind, Cycloalkyl, Heterocyclyl oder Aryl, das mit dem Phenanthridinring kondensiert ist, bilden (wobei das Cycloalkyl, Heterocyclyl oder Aryl, das mit dem Phenanthridinring kondensiert ist, jeweils unabhängig unsubstituiert ist oder mit einer oder mehreren Gruppen substituiert ist, die aus Halogen, OH, NH₂, NO₂, C(O)Z (wobei Z Wasserstoff, Hydroxy, C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl oder NH₂ ist), C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl, Heteroarylalkyl, Q-(C₁-C₈-Alkyl), Q-(C₂-C₈-Alkenyl), Q-(C₂-C₈-Alkinyl), Q-(C₃-C₆-Cycloalkyl), Q-(C₃-C₆-Cycloalkenyl), Q-(C₄-C₆-Cycloalkinyl), Q-(Heterocyclyl), Q-(Aryl), Q-(Arylalkyl), Q-(Heteroaryl) und Q-(Heteroarylalkyl) (wobei Q = O oder S ist) ausgewählt sind), und die übrigen Gruppen sind Wasserstoffatome;
[3] R² bis R⁵ sind Wasserstoffatome, R⁶ und R⁷ oder R⁷ und R⁸ von R⁶ bis R⁹ können zusammen mit Kohlenstoffatomen am Phenanthridinring, an die sie gebunden sind, Cycloalkyl, Heterocyclyl oder Aryl, das mit dem Phenanthridinring kondensiert ist, bilden (wobei das Cycloalkyl, Heterocyclyl oder Aryl, das mit dem Phenanthridinring kondensiert ist, jeweils unabhängig unsubstituiert ist oder mit einer oder mehreren Gruppen substituiert ist, die aus Halogen, OH, NH₂, NO₂, C(O)Z (wobei Z Wasserstoff, Hydroxy, C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl oder NH₂ ist), C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl, Heteroarylalkyl, Q-(C₁-C₈-Alkyl), Q-(C₂-C₈-Alkenyl), Q-(C₂-C₈-Alkinyl), Q-(C₃-C₆-Cycloalkyl), Q-(C₃-C₆-Cycloalkenyl), Q-(C₄-C₆-Cycloalkinyl), Q-(Heterocyclyl), Q-(Aryl), Q-(Arylalkyl), Q-(Heteroaryl) und Q-(Heteroarylalkyl) (wobei Q = O oder S ist) ausgewählt sind), und die übrigen Gruppen sind Wasserstoffatome;
[4] R³ ist 2'-Hydroxy-1',1',1',3',3',3'-hexafluorpropyl oder 2'-Benzyloxy-1',1',1',3',3',3'-hexafluorpropyl, bis zu 3 Vertreter von R² und R⁴ bis R⁹ sind jeweils unabhängig eine Gruppe, die aus Substituentengruppe A ausgewählt ist, und die übrigen Gruppen sind Wasserstoffatome; und
[5] R³ ist 2'-Hydroxy-1',1',1',3',3',3'-hexafluorpropyl oder 2'-Benzyloxy-1',1',1',3',3',3'-hexafluorpropyl, R⁴ und R⁵ oder R⁷ und R⁸ von R² bis R⁹ können zusammen mit Kohlenstoffatomen am Phenanthridinring, an die sie gebunden sind, Cycloalkyl, Heterocyclyl oder Aryl, das mit dem Phenanthridinring kondensiert ist, bilden (wobei das Cycloalkyl, Heterocyclyl oder Aryl, das mit dem Phenanthridinring kondensiert ist, jeweils unabhängig unsubstituiert ist oder mit einer oder mehreren Gruppen substituiert ist, die aus Halogen, OH, NH₂, NO₂, C(O)Z (wobei Z Wasserstoff, Hydroxy, C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl oder NH₂ ist), C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl, Heteroarylalkyl, Q-(C₁-C₈-Alkyl), Q-(C₂-C₈-Alkenyl), Q-(C₂-C₈-Alkinyl), Q-(C₃-C₆-Cycloalkyl), Q-(C₃-C₆-Cycloalkenyl), Q-(C₄-C₆-Cycloalkinyl), Q-(Heterocyclyl), Q-(Aryl), Q-(Arylalkyl), Q-(Heteroaryl) und Q-(Heteroarylalkyl) (wobei Q = O oder S ist) ausgewählt sind), irgendeine der anderen Gruppen ist aus Substituentengruppe A ausgewählt, und die übrigen Gruppen sind Wasserstoffatome.

3. Therapeutisches Mittel zur Verwendung bei der Prävention oder Behandlung von Hepatitis C gemäß Anspruch 1 oder 2, wobei R¹ eine Gruppe ist, die aus der Gruppe ausgewählt ist, welche aus Butyl, Benzyl, Hexyl und Cyclohexylmethyl besteht.

4. Therapeutisches Mittel zur Verwendung bei der Prävention oder Behandlung von Hepatitis C gemäß Anspruch 2 oder 3, wobei R² bis R⁹ Bedingung [1] genügen und es sich bei der Gruppe, die aus Substituentengruppe A ausgewählt ist, um Wasserstoff, Fluor, Trifluormethyl, 1',1',1',3',3',3'-Hexafluorpropyl, Isopropyl, 2'-Hydroxy-1',1',1',3',3',3'-hexafluorpropyl oder tert-Butyl handelt.

5. Therapeutisches Mittel zur Verwendung bei der Prävention oder Behandlung von Hepatitis C gemäß Anspruch 4, wobei es sich bei der Gruppe, die aus Substituentengruppe A ausgewählt ist, um Wasserstoff handelt.

6. Therapeutisches Mittel zur Verwendung bei der Prävention oder Behandlung von Hepatitis C gemäß Anspruch 2 oder 3, wobei R² bis R⁹ Bedingung [2] oder [3] genügen und das Cycloalkyl, Heterocyclyl oder Aryl, das mit dem Phenanthridinring kondensiert ist, unsubstituiert ist oder mit einer oder mehreren C₁-C₈-Alkylgruppen substituiert ist.

7. Therapeutisches Mittel zur Verwendung bei der Prävention oder Behandlung von Hepatitis C gemäß Anspruch 2 oder 3, wobei:
(a) R² bis R⁹ Bedingung [4] genügen und es sich bei der Gruppe, die aus Substituentengruppe A ausgewählt ist, um Hydroxy oder Methoxy handelt; oder
(b) R² bis R⁹ Bedingung [5] genügen und es sich bei dem Cycloalkyl, Heterocyclyl oder Aryl, das mit dem Phenanthridinring kondensiert ist, um unsubstituiertes 1,3-Dioxol handelt.

8. Therapeutisches Mittel zur Verwendung bei der Prävention oder Behandlung von Hepatitis C gemäß Anspruch 1, wobei die durch Formel (I) dargestellte Verbindung aus der Gruppe ausgewählt ist, die aus den folgenden Verbindungen besteht:
5-Butyl-2-(2'-hydroxy-1',1',1',3',3',3'-hexafluoropropan-2'-yl)-phenanthridin-6(5H)-on;
5-Benzyl-2-(2'-hydroxy-1',1',1',3',3',3'-hexafluoropropan-2'-yl)-phenanthridin-6(5H)-on;
5-Hexyl-2-(2'-hydroxy-1',1',1',3',3',3'-hexafluoropropan-2'-yl)-phenanthridin-6(5H)-on;
5-Cyclohexylmethyl-2-(2'-hydroxy-1',1',1',3',3',3'-hexafluoropropan-2'-yl)-phenanthridin-6(5H)-on;
5-Butyl-1-isopropylphenanthridin-6(5H)-on;
5-Butyl-2-isopropylphenanthridin-6(5H)-on;
5-Butyl-3-isopropylphenanthridin-6(5H)-on;
5-Butyl-2-tert-butylphenanthridin-6(5H)-on;
6-Butylbenzo[a]phenanthridin-5(6H)-on;
6-Butylbenzo[b]phenanthridin-5(6H)-on;
6-Butylbenzo[i]phenanthridin-5(6H)-on;
5-Butylbenzo[j]phenanthridin-6(5H)-on;
6-Butyl-8,9,10,11-tetrahydro-8,8,11,11-tetramethylbenzo[2,3-b]phenanthridin-5(6H)-on;
2-((2'-Benzyloxy-1',1',1',3',3',3'-hexafluorpropan-2'-yl)-5-butyl-3-methoxyphenanthridin-6(5H)-on;
4-Butyl-11-(1',1',1',3',3',3'-hexafluor-2'-hydroxypropan-2'-yl)-7-methoxy-[1,3]dioxolo[4,5-c]phenanthridin-5(4H)-on und
11-((2'-benzyloxy-1',1',1',3',3',3'-hexafluoropropan-2'-yl)-4-butyl-7-methoxy-[1,3]dioxolo[4,5-c]phenanthridin-5(4H)-on.

9. Verbindung, die durch Formel (IA) dargestellt wird, oder ein Salz davon: 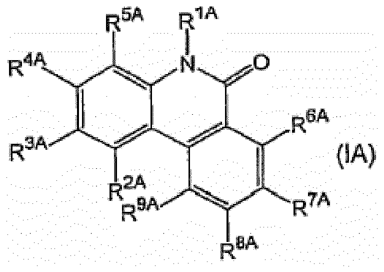 wobei
R^{1A} entweder Butyl oder Cyclohexylmethyl ist (wobei diese Gruppen jeweils unabhängig voneinander unsubstituiert sind oder mit einer oder mehreren Gruppen substituiert sind, die aus Halogen, OH, O-(C₂-C₈-Alkinyl), O-(C₃-C₆-Cycloalkyl), O-(C₃-C₆-Cycloalkenyl) oder O(C₄-C₆-Cycloalkinyl) ausgewählt sind); und
R^{2A} bis R^{9A} jeweils unabhängig aus Substituentengruppe B ausgewählt sind, die aus Wasserstoff, Halogen, Hydroxy, C(O)Z (wobei Z Wasserstoff, Hydroxy, C₁-C₈-Alkyl oder NH₂ ist), Q-(C₁-C₈-Alkyl), Q-(C₂-C₈-Alkenyl), Q-(C₂-C₈-Alkinyl) und Q-(C₃-C₆-Cycloalkyl) (wobei Q = O oder S ist), C₁-C₈-Alkyl, C₂-C₈-Alkenyl, C₂-C₈-Alkinyl, C₃-C₆-Cycloalkyl, C₃-C₆-Cycloalkenyl, C₄-C₆-Cycloalkinyl, Heterocyclyl, Aryl, Arylalkyl, Arylalkenyl, Heteroaryl und Heteroarylalkyl besteht (wobei diese Gruppen jeweils unabhängig voneinander unsubstituiert sind oder mit einer oder mehreren Gruppen substituiert sind, die aus Halogen, OH und O-(Arylalkyl) ausgewählt sind);
mit der Maßgabe, dass je zwei von R^{2A} bis R^{5A} von R^{2A} bis R^{9A} zusammen mit Kohlenstoffatomen am Phenanthridinring, an die sie gebunden sind, Cycloalkyl, Heterocyclyl oder Aryl, das mit dem Phenanthridinring kondensiert ist, bilden können (wobei das Cycloalkyl, Heterocyclyl oder Aryl, das mit dem Phenanthridinring kondensiert ist, jeweils unabhängig unsubstituiert ist oder mit einer oder mehreren C₁-C₈-Alkylgruppen substituiert ist); oder
je zwei von R^{6A} bis R^{9A} von R^{2A} bis R^{9A} zusammen mit Kohlenstoffatomen am Phenanthridinring, an die sie gebunden sind, Cycloalkyl, Heterocyclyl oder Aryl, das mit dem Phenanthridinring kondensiert ist, bilden können (wobei das Cycloalkyl, Heterocyclyl oder Aryl, das mit dem Phenanthridinring kondensiert ist, jeweils unabhängig unsubstituiert ist oder mit einer oder mehreren C₁-C₈-Alkylgruppen substituiert ist);
mit Ausnahme von 5-Butyl-1-methylphenanthridin-6(5H)-on, 5-Butyl-3-methylphenanthridin-6(5H)-on, 5-Butyl-4-methylphenanthridin-6(5H)-on, 5-Butyl-7-methylphenanthridin-6(5H)-on, 5-Butyl-8-methylphenanthridin-6(5H)-on, 5-Butyl-9-methylphenanthridin-6(5H)-on, 5-Butyl-10-methyl-phenanthridin-6(5H)-on, 5-Butylbenzo[c]phenanthridin-6(5H)-on, 5-Butyl-benzo[k]phenanthridin-6(5H)-on, 5-Butylphenanthridin-6(5H)-on und 5-(4-Brombutyl)phenanthridin-6(5H)-on.

10. Verbindung gemäß Anspruch 9 oder Salz davon, wobei R^{2A} bis R^{9A} irgendwelchen der folgenden Bedingungen [1A] bis [5A] genügen:
[1A] R^{5A} bis R^{9A} sind Wasserstoffatome, irgendeines von R^{2A} bis R^{4A} ist eine Gruppe, die aus Substituentengruppe B ausgewählt ist, und die übrigen Gruppen sind Wasserstoffatome;
[2A] R^{6A} bis R^{9A} sind Wasserstoffatome, R^{2A} und R^{3A} oder R^{3A} und R^{4A} von R^{2A} bis R^{5A} können zusammen mit Kohlenstoffatomen am Phenanthridinring, an die sie gebunden sind, Cycloalkyl, Heterocyclyl oder Aryl, das mit dem Phenanthridinring kondensiert ist, bilden (wobei das Cycloalkyl, Heterocyclyl oder Aryl, das mit dem Phenanthridinring kondensiert ist, jeweils unabhängig unsubstituiert ist oder mit einer oder mehreren C₁-C₈-Alkylgruppen substituiert ist), und die übrigen Gruppen sind Wasserstoffatome;
[3A] R^{2A} bis R^{5A} sind Wasserstoffatome, R^{6A} und R^{7A} oder R^{7A} und R^{8A} von R^{6A} bis R^{9A} können zusammen mit Kohlenstoffatomen am Phenanthridinring, an die sie gebunden sind, Cycloalkyl, Heterocyclyl oder Aryl, das mit dem Phenanthridinring kondensiert ist, bilden (wobei das Cycloalkyl, Heterocyclyl oder Aryl, das mit dem Phenanthridinring kondensiert ist, jeweils unabhängig unsubstituiert ist oder mit einer oder mehreren C₁-C₈-Alkylgruppen substituiert ist), und die übrigen Gruppen sind Wasserstoffatome;
[4A] R^{3A} ist 2'-Hydroxy-1',1',1',3',3',3'-hexafluorpropyl oder 2'-Benzyloxy-1',1',1',3',3',3'-hexafluorpropyl, bis zu 3 Vertreter von R^{2A} und R^{4A} bis R^{9A} sind jeweils unabhängig eine Gruppe, die aus Substituentengruppe B ausgewählt ist, und die übrigen Gruppen sind Wasserstoffatome; und
[5A] R^{3A} ist 2'-Hydroxy-1',1',1',3',3',3'-hexafluorpropyl oder 2'-Benzyloxy-1',1',1',3',3',3'-hexafluorpropyl, R^{4A} und R^{5A} oder R^{7A} und R^{8A} von R^{2A} und R^{4A} bis R^{9A} können zusammen mit Kohlenstoffatomen am Phenanthridinring, an die sie gebunden sind, Cycloalkyl, Heterocyclyl oder Aryl, das mit dem Phenanthridinring kondensiert ist, bilden (wobei das Cycloalkyl, Heterocyclyl oder Aryl, das mit dem Phenanthridinring kondensiert ist, jeweils unabhängig unsubstituiert ist oder mit einer oder mehreren C₁-C₈-Alkylgruppen substituiert ist), irgendeine der anderen Gruppen ist aus Substituentengruppe B ausgewählt, und die übrigen Gruppen sind Wasserstoffatome.

11. Verbindung gemäß Anspruch 9 oder 10 oder Salz davon, wobei R^{1A} Butyl ist.

12. Verbindung gemäß Anspruch 9 oder Salz davon, wobei die durch Formel (IA) dargestellte Verbindung aus der Gruppe ausgewählt ist, die aus den folgenden Verbindungen besteht:
5-Butyl-2-(2'-hydroxy-1',1',1',3',3',3'-hexafluoropropan-2'-yl)-phenanthridin-6(5H)-on;
5-Benzyl-2-(2'-hyd roxy-1',1',1',3',3',3'-hexafluoropropan-2'-yl)-phenanthridin-6(5H)-on;
5-Hexyl-2-(2'-hyd roxy-1',1',1',3',3',3'-hexafluoropropan-2'-yl)-phenanthridin-6(5H)-on;
5-Cyclohexylmethyl-2-(2'-hydroxy-1',1',1',3',3',3'-hexafluoropropan-2'-yl)-phenanthridin-6(5H)-on;
5-Butyl-1-isopropylphenanthridin-6(5H)-on;
5-Butyl-2-isopropylphenanthridin-6(5H)-on;
5-Butyl-3-isopropylphenanthridin-6(5H)-on;
5-Butyl-2-tert-butylphenanthridin-6(5H)-on;
6-Butylbenzo[a]phenanthridin-5(6H)-on;
6-Butylbenzo[b]phenanthridin-5(6H)-on;
6-Butylbenzo[i]phenanthridin-5(6H)-on;
5-Butylbenzo[j]phenanthridin-6(5H)-on;
6-Butyl-8,9,10,11-tetrahyd ro-8,8,11,11-tetramethylbenzo[2,3-b]phenanthridin-5(6H)-on;
2-((2'-Benzyloxy-1',1',1',3',3',3'-hexafluorpropan-2'-yl)-5-butyl-3-methoxyphenanthridin-6(5H)-on;
4-Butyl-11-(1',1',1',3',3',3'-hexafluor-2'-hydroxypropan-2'-yl)-7-methoxy-[1,3]dioxolo[4,5-c]phenanthridin-5(4H)-on und
11-((2'-benzyloxy-1',1',1',3',3',3'-hexafluoropropan-2'-yl)-4-butyl-7-methoxy-[1,3]dioxolo[4,5-c]phenanthridin-5(4H)-on.

13. Verfahren zur Herstellung der durch Formel (IA) dargestellten Verbindung gemäß Anspruch 9 oder eines Salzes davon, umfassend die im Folgenden beschriebenen Schritte:
Schritt (1A) des Verbindens eines durch Formel (II) dargestellten Anilinderivats 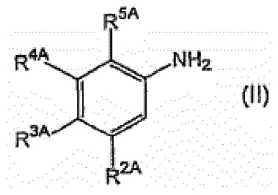 wobei R^{2A} bis R^{5A} wie in Anspruch 9 definiert sind,
mit einem durch Formel (III) dargestellten Benzoylhalogenid: 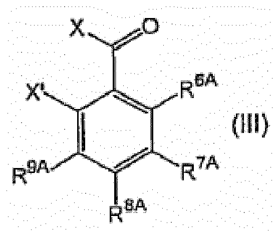 wobei R^{6A} bis R^{9A} wie in Anspruch 9 definiert sind und
X und X' jeweils unabhängig aus Halogenatomen ausgewählt sind,
unter Bildung eines durch Formel (IV) dargestellten Benzanilids: 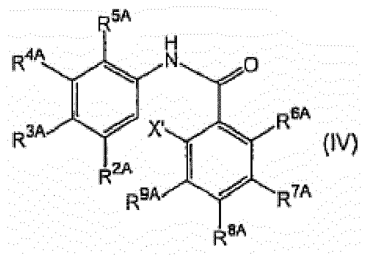 wobei R^{2A} bis R^{9A} und X' wie in Anspruch 9 definiert sind;
Schritt (1B) des Reagierenlassens des durch Formel (IV) dargestellten Benzanilids mit einem durch Formel (V) dargestellten Halogenid:
R^{1A}-X" (V),
wobei R^{1A} wie in Anspruch 9 definiert ist und
X" ein Halogen ist;
unter Bildung eines durch Formel (VI) dargestellten N-substituierten Benzanilids: 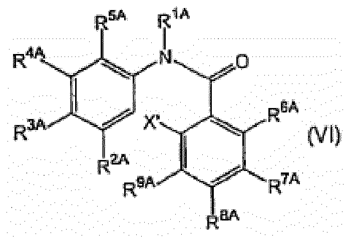 wobei R^{1A} bis R^{9A} und X' wie oben definiert sind; und
Schritt (1C) des Durchführens einer intramolekularen Cyclisierungsreaktion mit dem durch Formel (VI) dargestellten N-substituierten Benzanilid in Gegenwart von Palladiumacetat unter Bildung der durch Formel (IA) dargestellten Verbindung.

14. Verfahren zur Herstellung der durch Formel (IA) dargestellten Verbindung gemäß Anspruch 9 oder eines Salzes davon, umfassend die im Folgenden beschriebenen Schritte:
Schritt (2C) des Verbindens eines durch Formel (IV") dargestellten N-substituierten Anilins 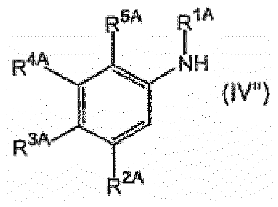 wobei R^{1A} bis R^{5A} wie in Anspruch 9 definiert sind,
mit einem durch Formel (III) dargestellten Benzoylhalogenid: 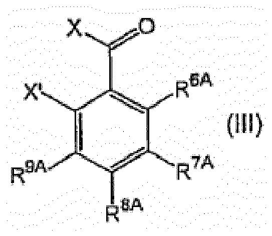 wobei R^{6A} bis R^{9A} wie in Anspruch 9 definiert sind und
X und X' jeweils unabhängig aus Halogenatomen ausgewählt sind,
unter Bildung eines durch Formel (VI) dargestellten N-substituierten Benzanilids: 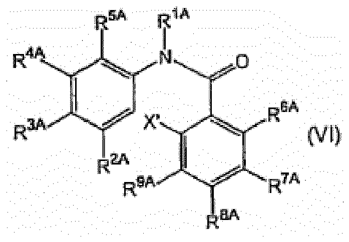 wobei R^{1A} bis R^{9A} und X' wie oben definiert sind; und
Schritt (2D) des Durchführens einer intramolekularen Cyclisierungsreaktion mit dem durch Formel (VI) dargestellten N-substituierten Benzanilid in Gegenwart von Palladiumacetat unter Bildung der durch Formel (IA) dargestellten Verbindung.

15. Verfahren zur Herstellung der durch Formel (IA) dargestellten Verbindung gemäß Anspruch 9 oder eines Salzes davon, umfassend die im Folgenden beschriebenen Schritte:
Schritt (4C) des Verbindens eines durch Formel (IV") dargestellten N-substituierten Anilins 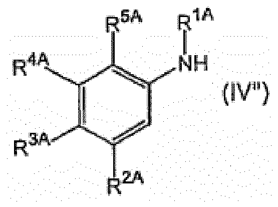 wobei R^{1A} bis R^{5A} wie in Anspruch 9 definiert sind,
mit einer durch Formel (III') dargestellten Halogenbenzoesäure: 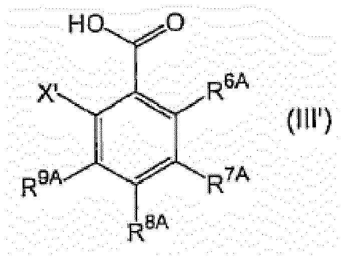 wobei R^{6A} bis R^{9A} wie in Anspruch 9 definiert sind und
X' ein Halogenatom ist;
unter Bildung eines durch Formel (VI) dargestellten N-substituierten Benzanilids: 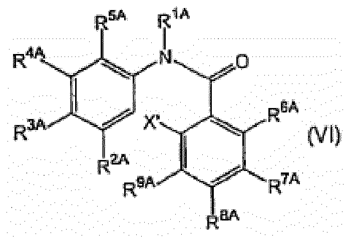 wobei R^{1A} bis R^{9A} und X' wie oben definiert sind; und
Schritt (4D) des Durchführens einer intramolekularen Cyclisierungsreaktion mit dem durch Formel (VI) dargestellten N-substituierten Benzanilid in Gegenwart von Palladiumacetat unter Bildung der durch Formel (IA) dargestellten Verbindung.

## Revendications

1. Agent thérapeutique destiné à être utilisé dans la prévention ou le traitement de l'hépatite C, lequel agent comprend, comme ingrédient actif, un composé représenté par la formule (I) ou un sel pharmaceutiquement acceptable de celui-ci : 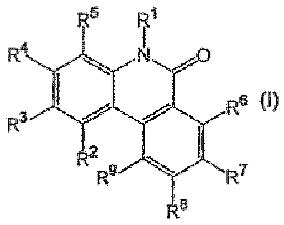 où,
R¹ est choisi parmi C₁-C₈ alkyle, C₂-C₈ alcényle, C₂-C₈ alcynyle, C₃-C₆ cycloalkyle, C₃-C₆ cycloalcényle, C₄-C₆ cycloalcynyle, hétérocyclyle, aryle, arylalkyle, arylalcényle, hétéroaryle, et hétéroarylalkyle (chacun de ces groupes étant indépendamment non substitué ou substitué avec un ou plusieurs groupes choisis parmi halogène, OH, NH₂, NO₂, C(O)Z (où Z est hydrogène, hydroxyle, C₁-C₈ alkyle, C₂-C₈ alcényle, C₂-C₈ alcynyle, C₃-C₆ cycloalkyle, C₃-C₆ cycloalcényle, C₄-C₆ cycloalcynyle, ou NH₂), C₁-C₈ alkyle, C₂-C₈ alcényle, C₂-C₈ alcynyle, C₃-C₆ cycloalkyle, C₃-C₆ cycloalcényle, C₄-C₆ cycloalcynyle, hétérocyclyle, aryle, arylalkyle, arylalcényle, hétéroaryle, hétéroarylalkyle, Q-(C₁-C₈ alkyle), Q-(C₂-C₈ alcényle), Q-(C₂-C₈ alcynyle), Q-(C₃-C₆ cycloalkyle), Q-(C₃-C₆ cycloalcényle), Q-(C₄-C₆ cycloalcynyle), Q-(hétérocyclyle), Q-(aryle), Q-(arylalkyle), Q-(hétéroaryle), et Q-(hétéroarylalkyle) (où Q est O ou S)) ; et
R² à R⁹ sont choisis chacun indépendamment dans le groupe de substituants A consistant en hydrogène, halogène, hydroxyle, C(O)Z (où Z est hydrogène, hydroxyle, C₁-C₈ alkyle, C₂-C₈ alcényle, C₂-C₈ alcynyle, C₃-C₆ cycloalkyle, C₃-C₆ cycloalcényle, C₄-C₆ cycloalcynyle, ou NH₂), Q-(C₁-C₈ alkyle), Q-(C₂-C₈ alcényle), Q-(C₂-C₈ alcynyle), Q-(C₃-C₆ cycloalkyle), Q-(C₃-C₆ cycloalcényle), Q-(C₄-C₆ cycloalcynyle), Q-(hétérocyclyle), Q-(aryle), Q-(arylalkyle), Q-(hétéroaryle) et Q-(hétéroarylalkyle) (où Q est O ou S), C₁-C₈ alkyle, C₂-C₈ alcényle, C₂-C₈ alcynyle, C₃-C₆ cycloalkyle, C₃-C₆ cycloalcényle, C₄-C₆ cycloalcynyle, hétérocyclyle, aryle, arylalkyle, arylalcényle, hétéroaryle, et hétéroarylalkyle (chacun de ces groupes étant indépendamment non substitué ou substitué avec un ou plusieurs groupes choisis parmi halogène, OH, NH₂, NO₂, C(O)Z (où Z est hydrogène, hydroxyle, C₁-C₈ alkyle, C₂-C₈ alcényle, Crocs alcynyle, C₃-C₆ cycloalkyle, C₃-C₆ cycloalcényle, C₄-C₆ cycloalcynyle, ou NH₂), C₁-C₈ alkyle, C₂-C₈ alcényle, C₂-C₈ alcynyle, C₃-C₆ cycloalkyle, C₃-C₆ cycloalcényle, C₄-C₆ cycloalcynyle, hétérocyclyle, aryle, arylalkyle, arylalcényle, hétéroaryle, hétéroarylalkyle, Q-(C₁-C₈ alkyle), Q-(C₂-C₈ alcényle), Q-(C₂-C₈ alcynyle), Q-(C₃-C₆ cycloalkyle), Q-(C₃-C₆ cycloalcényle), Q-(C₄-C₆ cycloalcynyle), Q-(hétéro-cyclyle), Q-(aryle), Q-(arylalkyle), Q-(hétéroaryle), et Q-(hétéroarylalkyle) (où Q est O ou S)),
à condition que deux quelconques de R² à R⁵ avec les atomes de carbone sur le cycle phénanthridine auxquels ils sont liés puissent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes choisis parmi halogène, OH, NH₂, NO₂, C(O)Z (où Z est hydrogène, hydroxyle, C₁-C₈ alkyle, C₂-C₈ alcényle, C₂-C₈ alcynyle, C₃-C₆ cycloalkyle, C₃-C₆ cycloalcényle, C₄-C₆ cycloalcynyle ou NH₂), C₁-C₈ alkyle, C₂-C₈ alcényle, C₂-C₈ alcynyle, C₃-C₆ cycloalkyle, C₃-C₆ cycloalcényle, C₄-C₆ cycloalcynyle, hétérocyclyle, aryle, arylalkyle, arylalcényle, hétéroaryle, hétéroarylalkyle, Q-(C₁-C₈ alkyle), Q-(C₂-C₈ alcényle), Q-(C₂-C₈ alcynyle), Q-(C₃-C₆ cycloalkyle), Q-(C₃-C₆ cycloalcényle), Q-(C₄-C₆ cycloalcynyle), Q-(hétérocyclyle), Q-(aryle), Q-(arylalkyle), Q-(hétéroaryle), et Q-(hétéro-arylalkyle) (où Q est O ou S)), ou
deux quelconques de R⁶ à R⁸ avec les atomes de carbone sur le cycle phénanthridine auxquels ils sont liés peuvent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes choisis parmi halogène, OH, NH₂, NO₂, C(O)Z (où Z est hydrogène, hydroxyle, C₁-C₈ alkyle, C₂-C₈ alcényle, C₂-C₈ alcynyle, C₃-C₆ cycloalkyle, C₃-C₆ cycloalcényle, C₄-C₆ cycloalcynyle, ou NH₂), C₁-C₈ alkyle, C₂-C₈ alcényle, C₂-C₈ alcynyle, C₃-C₆ cycloalkyle, C₃-C₆ cycloalcényle, C₄-C₆-cycloalcynyle, hétérocyclyle, aryle, arylalkyle, arylalcényle, hétéroaryle, hétéroarylalkyle, Q-(C₁-C₈ alkyle), Q-(C₂-C₈ alcényle), Q-(C₂-C₈ alcynyle), Q-(C₃-C₆ cycloalkyle), Q-(C₃-C₆ cycloalcényle), Q-(C₄-C₆ cycloalcynyle), Q-(hétérocyclyle), Q-(aryle), Q-(arylalkyle), Q-(hétéroaryle), et Q-(hétéro-arylalkyle) (où Q est O ou S)),
à l'exception de la 5-butyl-1-méthylphénanthridin-6(5H)-one, la 5-butyl-3-méthylphénanthridin-6(5H)-one, la 5-butyl-4-méthyl-phénanthridin-6(5H)-one, la 5-butyl-7-méthylphénanthridin-6(5H)-one, la 5-butyl-8-méthylphénanthridin-6(5H)-one, la 5-butyl-9-méthyl-phénanthridin-6(5H)-one, la 5-butyl-10-méthylphénanthridin-6(5H)-one, la 5-butylbenzo[c]phénanthridin-6(5H)-one, et la 5-butylbenzo[k]phénanthridin-6(5H)-one.

2. Agent thérapeutique destiné à être utilisé dans la prévention ou le traitement de l'hépatite C selon la revendication 1, où R² à R⁹ satisfont l'une quelconque des conditions [1] à [5] ci-dessous :
[1] R⁵ à R⁹ sont des atomes d'hydrogène, l'un quelconque de R² à R⁴ est un groupe choisi dans le groupe de constituants A, et les autres groupes restants sont des atomes d'hydrogène ;
[2] R⁶ à R⁹ sont des atomes d'hydrogène, R² et R³ ou R³ et R⁴ de R² à R⁵ avec les atomes de carbone sur le cycle phénanthridine auxquels ils sont liés peuvent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes choisis parmi halogène, OH, NH₂, NO₂ C(O)Z (où Z est hydrogène, hydroxyle, C₁-C₈ alkyle, C₂-C₈ alcényle, C₂-C₈ alcynyle, C₃-C₆ cycloalkyle, C₃-C₆ cycloalcényle, C₄-C₆ cycloalcynyle, ou NH₂), C₁-C₈ alkyle, C₂-C₈ alcényle, C₂-C₈ alcynyle, C₃-C₆ cycloalkyle, C₃-C₆ cycloalcényle, C₄-C₆ cycloalcynyle, hétérocyclyle, aryle, arylalkyle, arylalcényle, hétéroaryle, hétéroarylalkyle, Q-(C₁-C₈ alkyle), Q-(C₂-C₈ alcényle), Q-(C₂-C₈ alcynyle), Q-(C₃-C₆ cycloalkyle), Q-(C₃-C₆ cycloalcényle), Q-(C₄-C₆ cycloalcynyle), Q-(hétérocyclyle), Q-(aryle), Q-(arylalkyle), Q-(hétéroaryle), et Q-(hétéroarylalkyle) (où Q est O ou S)), et les autres groupes restants sont des atomes d'hydrogène ;
[3] R² à R⁵ sont des atomes d'hydrogène, R⁶ et R⁷ ou R⁷ et R⁸ de R⁶ à R⁹ avec les atomes de carbone sur le cycle phénanthridine auxquels ils sont liés peuvent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes choisis parmi halogène, OH, NH₂, NO₂, C(O)Z (où Z est hydrogène, hydroxyle, C₁-C₈ alkyle, C₂-C₈ alcényle, C₂-C₈ alcynyle, C₃-C₆ cycloalkyle, C₃-C₆ cycloalcényle, C₄-C₆ cycloalcynyle ou NH₂), C₁-C₈ alkyle, C₂-C₈ alcényle, C₂-C₈ alcynyle, C₃-C₆ cycloalkyle, C₃-C₆ cycloalcényle, C₄-C₆ cycloalcynyle, hétérocyclyle, aryle, arylalkyle, arylalcényle, hétéroaryle, hétéroarylalkyle, Q-(C₁-C₈ alkyle), Q-(C₂-C₈ alcényle), Q-(C₂-C₈ alcynyle), Q-(C₃-C₆ cycloalkyle), Q-(C₃-C₆ cyclo-alcényle), Q-(C₄-C₆ cycloalcynyle), Q-(hétérocyclyle), Q-(aryle), Q-(arylalkyle), Q-(hétéroaryle), et Q-(hétéroarylalkyle), (où Q est O ou S)), et les autres groupes restants sont des atomes d'hydrogène ;
[4] R³ est 2-hydroxy-1',1',1',3',3',3'-hexafluoropropyle ou 2'-benzyloxy-1',1',1',3',3',3'-hexafluoropropyle, jusqu'à 3 membres de R² et R⁴ à R⁹ sont chacun indépendamment un groupe choisi dans le groupe de substituants A, et les autres groupes restants sont des atomes d'hydrogène ; et
[5] R³ est 2'-hydroxy-1',1',1',3',3',3'-hexafluoropropyle ou 2'-benzyloxy-1',1',1',3',3',3'-hexafluoropropyle, R⁴ et R⁵ ou R⁷ et R⁸ de R² et R⁴ à R⁹ avec les atomes de carbone sur le cycle phénanthridine auxquels ils sont liés peuvent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes choisis parmi halogène, OH, NH₂, NO₂, C(O)Z (où Z est hydrogène, hydroxyle, C₁-C₈ alkyle, C₂-C₈ alcényle, C₂-C₈ alcynyle, C₃-C₆ cycloalkyle, C₃-C₆ cycloalcényle, C₄-C₆ cycloalcynyle ou NH₂), C₁-C₈ alkyle, C₂-C₈ alcényle, C₂-C₈ alcynyle, C₃-C₆ cycloalkyle, C₃-C₆ cycloalcényle, C₄-C₆ cycloalcynyle, hétérocyclyle, aryle, arylalkyle, arylalcényle, hétéroaryle, hétéroarylalkyle, Q-(C₁-C₈ alkyle), Q-(C₂-C₈ alcényle), Q-(C₂-C₈ alcynyle), Q-(C₃-C₆ cycloalkyle), Q-(C₃-C₆ cyclo-alcényle), Q-(C₄-C₆ cycloalcynyle), Q-(hétérocyclyle), Q-(aryle), Q-(aryl-alkyle), Q-(hétéroaryle), et Q-(hétéroarylalkyle) (où Q est O ou S)), l'un quelconque des autres groupes est choisi dans le groupe de substituants A, et les autres groupes restants sont des atomes d'hydrogène.

3. Agent thérapeutique destiné à être utilisé dans la prévention ou le traitement de l'hépatite C selon la revendication 1 ou 2, où R¹ est un groupe choisi dans le groupe consistant en butyle, benzyle, hexyle et cyclohexylméthyle.

4. Agent thérapeutique destiné à être utilisé dans la prévention ou le traitement de l'hépatite C selon la revendication 2 ou 3, où R² à R⁹ satisfont la condition [1] et le groupe choisi dans le groupe de substituants A est l'hydrogène, le fluor, trifluorométhyle, 1',1',1',3',3',3'-hexafluoropropyle, isopropyle, 2'-hydroxy-1',1',1',3',3',3'-hexafluoropropyle, ou tert-butyle.

5. Agent thérapeutique destiné à être utilisé dans la prévention ou le traitement de l'hépatite C selon la revendication 4, où le groupe choisi dans le groupe de substituants A est l'hydrogène.

6. Agent thérapeutique destiné à être utilisé dans la prévention ou le traitement de l'hépatite C selon la revendication 2 ou 3, où R² à R⁹ satisfont la condition [2] ou [3] et cycloalkyle, hétérocyclyle ou aryle qui est condensé au cycle phénanthridine est non substitué ou substitué avec un ou plusieurs groupes C₁-C₈ alkyle.

7. Agent thérapeutique destiné à être utilisé dans la prévention ou le traitement de l'hépatite C selon la revendication 2 ou 3, où :
(a) R² à R⁹ satisfont la condition [4] et le groupe choisi dans le groupe de substituants A est hydroxyle ou méthoxyle ; ou
(b) R² à R⁹ satisfont la condition [5] et cycloalkyle, hétérocyclyle ou aryle qui est condensé au cycle phénanthridine est 1,3-dioxole non substitué.

8. Agent thérapeutique destiné à être utilisé dans la prévention ou le traitement de l'hépatite C selon la revendication 1, où le composé représenté par la formule (I) est choisi dans le groupe consistant en les composés ci-dessous :
la 5-butyl-2-(2'-hydroxy-1',1',1',3',3',3'-hexafluoropropan-2'-yl)-phénanthridin-6(5H)-one ;
la 5-benzyl-2-(2'-hydroxy-1',1',1',3',3',3'-hexafluoropropan-2'-yl)-phénanthridin-6(5H)-one ;
la 5-hexyl-2-(2'-hydroxy-1',1',1',3',3',3'-hexafluoropropan-2'-yl)-phénanthridin-6(5H)-one ;
la 5-cyclohexylméthyl-2-(2'-hydroxy-1',1',1',3',3',3'-hexafluoropropan-2'-yl)-phénanthridin-6(5H)-one ;
la 5-butyl-1-isopropylphénanthridin-6(5H)-one ;
la 5-butyl-2-isopropylphénanthridin-6(5H)-one ;
la 5-butyl-3-isopropylphénanthridin-6(5H)-one ;
la 5-butyl-2-tert-butylphénanthridin-6(5H)-one ;
la 6-butylbenzo[a]phénanthridin-5(6H)-one ;
la 6-butylbenzo[b]phénanthridin-5(6H)-one ;
la 6-butylbenzo[i]phénanthridin-5(6H)-one ;
la 5-butylbenzo[j]phénanthridin-6(5H)-one ;
la 6-butyl-8,9,10,11-tétrahydro-8,8,11,11-tétraméthylbenzo[2,3-b]phénanthridin-5(6H)-one ;
la 2-(2'-benzyloxy-1',1',1',3',3',3'-hexafluoropropan-2'-yl)-5-butyl-3-méthoxyphénanthridin-6(5H)-one ;
la 4-butyl-11-(1',1',1',3',3',3'-hexafluoro-2'-hydroxypropan-2'-yl)-7-méthoxy-[1,3]dioxolo[4,5-c]phénanthridin-5(4H)-one ; et
la 11-(2'-benzyloxy-1',1',1',3',3',3'-hexafluoropropan-2'-yl)-4-butyl-7-méthoxy-[1,3]dioxolo[4,5-c]phénanthridin-5(4H)-one.

9. Composé représenté par la formule (IA) ou sel de celui-ci : 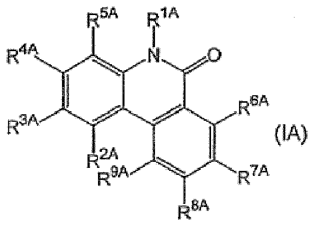 où
R^{1A} est butyle ou cyclohexylméthyle (chacun de ces groupes étant indépendamment non substitué ou substitué avec un ou plusieurs groupes choisis parmi halogène, OH, O-(C₂-C₈ alcynyle), O-(C₃-C₆ cyclo-alkyle), O-(C₃-C₆ cycloalcényle), ou O-(C₄-C₆ cycloalcynyle)) ; et
R^{2A} à R^{9A} sont choisis chacun indépendamment dans le groupe de substituants B consistant en hydrogène, halogène, hydroxyle, C(O)Z (où Z est hydrogène, hydroxyle, C₁-C₈ alkyle ou NH₂), Q-(C₁-C₈ alkyle), Q-(C₂-C₈ alcényle), Q-(C₂-C₈ alcynyle) et Q-(C₃-C₆ cycloalkyle) (où Q est O ou S), C₁-C₈ alkyle, C₂-C₈ alcényle, C₂-C₈ alcynyle, C₃-C₆ cycloalkyle, C₃-C₆ cycloalcényle, C₄-C₆ cycloalcynyle, hétérocyclyle, aryle, arylalkyle, arylalcényle, hétéroaryle et hétéroarylalkyle (chacun de ces groupes étant indépendamment non substitué ou substitué avec un ou plusieurs groupes choisis parmi halogène, OH et O-(arylalkyle)),
à condition que deux quelconques de R^{2A} à R^{5A} de R^{2A} à R^{9A} avec les atomes de carbone sur le cycle phénanthridine auxquels ils sont liés puissent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes C₁-C₈ alkyle), ou
deux quelconques de R^{6A} à R^{9A} de R^{2A} à R^{9A} avec les atomes de carbone sur le cycle phénanthridine auxquels ils sont liés peuvent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes C₁-C₈ alkyle),
à l'exception de la 5-butyl-1-méthylphénanthridin-6(5H)-one, la 5-butyl-3-méthylphénanthridin-6(5H)-one, la 5-butyl-4-méthylphénanthridin-6(5H)-one, la 5-butyl-7-méthylphénanthridin-6(5H)-one, la 5-butyl-8-méthylphénanthridin-6(5H)-one, la 5-butyl-9-méthylphénanthridin-6(5H)-one, la 5-butyl-10-méthylphénanthridin-6(5H)-one, la 5-butyl-benzo[c]phénanthridin-6(5H)-one, la 5-butylbenzo[k]phénanthridin-6(5H)-one, la 5-butylphénanthridin-6(5H)-one, et la 5-(4-bromobutyl)-phénanthridin-6(5H)-one.

10. Composé selon la revendication 9 ou sel de celui-ci, où R^{2A} à R^{9A} satisfont l'une quelconque des conditions [1A] à [5A] ci-dessous :
[1A] R^{5A} à R^{9A} sont des atomes d'hydrogène, l'un quelconque de R^{2A} à R^{4A} est un groupe choisi dans le groupe de substituants B et les autres groupes restants sont des atomes d'hydrogène ;
[2A] R^{6A} à R^{9A} sont des atomes d'hydrogène, R^{2A} et R^{3A} ou R^{3A} et R^{4A} de ^{2A} à R^{5A} avec les atomes de carbone sur le cycle phénanthridine auxquels ils sont liés peuvent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes C₁-C₈ alkyle), et les autres groupes restants sont des atomes d'hydrogène ;
[3A] R^{2A} à R^{5A} sont des atomes d'hydrogène, R^{6A} et R^{7A} ou R^{7A} et R^{8A} de R^{6A} à R^{9A} avec les atomes de carbone sur le cycle phénanthridine auxquels ils sont liés peuvent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes C₁-C₈ alkyle), et les autres groupes restants sont des atomes d'hydrogène ;
[4A] R^{3A} est 2'-hydroxy-1',1',1',3',3',3'-hexafluoropropyle ou 2'-benzyloxy-1',1',1',3',3',3'-hexafluoropropyle, jusqu'à 3 membres de R^{2A} et R^{4A} à R^{9A} sont chacun indépendamment un groupe choisi dans le groupe de substituants B, et les autres groupes restants sont des atomes d'hydrogène ; et
[5A] R^{3A} est 2'-hydroxy-1',1',1',3',3',3'-hexafluoropropyle ou 2'-benzyloxy-1',1',1',3',3',3'-hexafluoropropyle, R^{4A} et R^{5A} ou R^{7A} et R^{8A} de R^{2A} et R^{4A} à R^{9A} avec les atomes de carbone sur le cycle phénanthridine auxquels ils sont liés peuvent former cycloalkyle, hétérocyclyle ou aryle condensé au cycle phénanthridine (le cycloalkyle, hétérocyclyle ou aryle condensé au cycle phénanthridine étant indépendamment non substitué ou substitué avec un ou plusieurs groupes C₁-C₈ alkyle), l'un quelconque des autres groupes est choisi dans le groupe de substituants B, et les autres groupes restants sont des atomes d'hydrogène.

11. Composé selon la revendication 9 ou 10 ou sel de celui-ci, où R^{1A} est butyle.

12. Composé selon la revendication 9 ou sel de celui-ci, où le composé représenté par la formule (IA) est choisi dans le groupe consistant en les composés ci-dessous :
la 5-butyl-2-(2'-hydroxy-1',1',1',3',3',3'-hexafluoropropan-2'-yl)-phénanthridin-6(5H)-one ;
la 5-cyclohexylméthyl-2-(2'-hydroxy-1',1',1',3',3',3'-hexafluoro-propan-2'-yl)-phénanthridin-6(5H)-one ;
la 5-butyl-1-isopropylphénanthridin-6(5H)-one ;
la 5-butyl-2-isopropylphénanthridin-6(5H)-one ;
la 5-butyl-3-isopropylphénanthridin-6(5H)-one ;
la 5-butyl-2-tert-butylphénanthridin-6(5H)-one ;
la 6-butylbenzo[a]phénanthridin-5(6H)-one ;
la 6-butylbenzo[b]phénanthridin-5(6H)-one ;
la 6-butylbenzo[i]phénanthridin-5(6H)-one ;
la 5-butylbenzo[j]phénanthridin-6(5H)-one ;
la 6-butyl-8,9,10,11-tétrahydro-8,8,11,11-tétraméthylbenzo[2,3-b]phénanthridin-5(6H)-one ;
la 2-(2'-benzyloxy-1',1',1',3',3',3'-hexafluoropropan-2'-yl)-5-butyl-3-méthoxyphénanthridin-6(5H)-one ;
la 4-butyl-11-(1',1',1',3',3',3'-hexafluoro-2'-hydroxypropan-2'-yl)-7-méthoxy-[1,3]dioxolo[4,5-c]phénanthridin-5(4H)-one ; et
la 11-(2'-benzyloxy-1',1',1',3',3',3'-hexafluoropropan-2'-yl)-4-butyl-7-méthoxy-[1,3]dioxolo[4,5-c]phénanthridin-5(4H)-one.

13. Procédé de préparation du composé représenté par la formule (IA) selon la revendication 9 ou d'un sel de celui-ci comprenant les étapes décrites ci-dessous :
l'étape (1A) de liaison d'un dérivé de l'aniline représenté par la formule (II) : 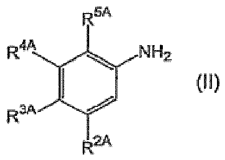 où
R^{2A} à R^{5A} sont définis comme dans la revendication 9,
à un halogénure de benzoyle représenté par la formule (III) : 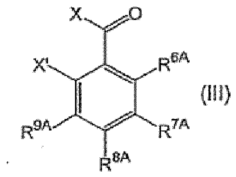 où
R^{6A} à R^{9A} sont définis comme dans la revendication 9 ; et
X et X' sont choisis chacun indépendamment parmi des atomes d'halogène,
pour former un benzanilide représenté par la formule (IV) : 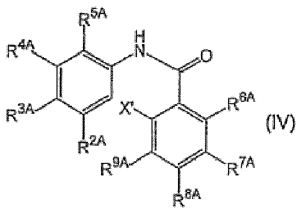 où
R^{2A} à R^{9A} et X' sont définis comme dans la revendication 9 ;
l'étape (1B) de réaction du benzanilide représenté par la formule (IV) avec un halogénure représenté par la formule (V) :
R^{1A}-X" (V)
où
R^{1A} est défini comme dans la revendication 9 ; et
X" est halogène,
pour former un benzanilide N-substitué représenté par la formule (VI) : 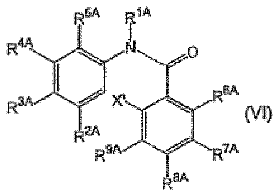 où R^{1A} à R^{9A} et X' sont définis comme ci-dessus ; et
l'étape (1C) d'exposition du benzanilide N-substitué représenté par la formule (VI) à une réaction de cyclisation intramoléculaire en présence d'acétate de palladium pour former le composé représenté par la formule (IA).

14. Procédé de préparation du composé représenté par la formule (IA) selon la revendication 9 ou d'un sel de celui-ci comprenant les étapes décrites ci-dessous :
l'étape (2C) de liaison d'une aniline N-substituée représentée par la formule (IV") : 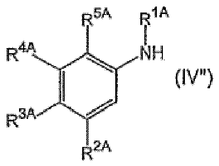 où
R^{1A} à R^{5A} sont définis comme dans la revendication 9,
à un halogénure de benzoyle représenté par la formule (III) : 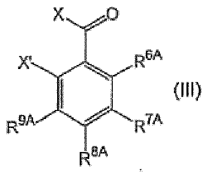 où
R^{6A} à R^{9A} sont définis comme dans la revendication 9 ; et
X et X' sont choisis chacun indépendamment parmi des atomes d'halogène,
pour former un benzanilide N-substitué représenté par la formule (VI) : 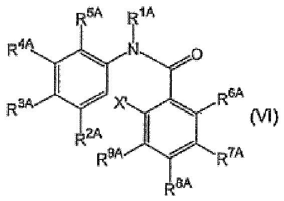 où
R^{1A} à R^{9A} et X' sont définis comme dans la revendication 9 ; et
l'étape (2D) d'exposition du benzanilide N-substitué représenté par la formule (VI) à une réaction de cyclisation intramoléculaire en présence d'acétate de palladium pour former le composé représenté par la formule (IA).

15. Procédé de préparation du composé représenté par la formule (IA) selon la revendication 9 ou d'un sel de celui-ci comprenant les étapes décrites ci-dessous :
l'étape (4C) de liaison d'une aniline N-substituée représentée par la formule (IV") : 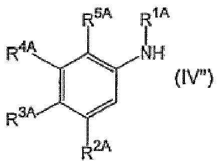 où
R^{1A} à R^{5A} sont définis comme dans la revendication 9,
à un acide halobenzoïque représenté par la formule (III') : 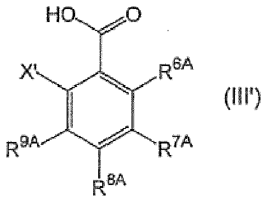 où
R^{6A} à R^{9A} sont définis comme dans la revendication 9 ; et
X' est un atome d'halogène,
pour former un benzanilide N-substitué par la formule (VI) : 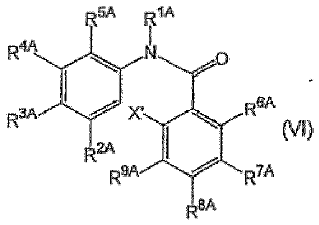 où
R^{1A} à R^{9A} et X' sont définis comme ci-dessus ; et
l'étape (4D) d'exposition du benzanilide N-substitué représenté par la formule (VI) à une réaction de cyclisation intramoléculaire en présence d'acétate de palladium pour former le composé représenté par la formule (IA).
